(19) Europäisches Patentamt
European Patent Office
Office européen des brevets

(11) Numéro de publication: **0 396 435 B1**

(12) **FASCICULE DE BREVET EUROPEEN**

(45) Date de publication de fascicule du brevet: **21.06.95**

(51) Int. Cl.⁶: **C07C 229/16**, C07D 213/38, C07D 241/12, C07D 233/64, C07D 257/02, B01D 53/04

(21) Numéro de dépôt: **90400488.4**

(22) Date de dépôt: **22.02.90**

(54) **Dérivés polyazotés et leurs complexes métalliques utilisables pour la fixation de l'oxygène.**

(30) Priorité: **22.02.89 FR 8902315**

(43) Date de publication de la demande:
**07.11.90 Bulletin 90/45**

(45) Mention de la délivrance du brevet:
**21.06.95 Bulletin 95/25**

(84) Etats contractants désignés:
**AT BE CH DE DK ES FR GB GR IT LI LU NL SE**

(56) Documents cités:
**EP-A- 0 099 685**
**EP-A- 0 287 436**
**DE-A- 1 768 849**
**US-A- 2 751 390**
**US-A- 2 781 389**

**JOURNAL OF THE CHEMICAL SOCIETY, no. 6, mars 1984, pages 335-336; C. RALEIGH et al.: "Autoxidation pathways of Co11-complexes of pyridyl-containing pentamines involving dioxygen complexes as intermediates"**

(73) Titulaire: **L'AIR LIOUIDE, SOCIETE ANONYME POUR L'ETUDE ET L'EXPLOITATION DES PROCEDES GEORGES CLAUDE**
**75, Ouai d'Orsay**
**F-75321 Paris Cédex 07 (FR)**

(72) Inventeur: **Boisselier-Cocolios, Brigitte**
**44, rue de Marcoussis**
**F-91470 Limours (FR)**
Inventeur: **Guilard, Roger**
**48, rue du Cottage**
**F-21120 Fontaine-Les-Dijon (FR)**
Inventeur: **Jean, Christophe**
**6, rue Rameau**
**F-21300 Chenove (FR)**
Inventeur: **Taurin, Laurent**
**2, ruelle de l'Epinette,**
**Bâtiment 2**
**F-91160 Longjumeau (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

INORGANIC CHEMISTRY, vol. 14, no. 10, octobre 1975, pages 2322-2326; G. McLENDON et al.: "Combination of dioxygen with N,N-bis(2-aminoethyl)glycinatocobalt(II) and with diethylenetriamine-N-acetatocobalt(II)"

(74) Mandataire: **Le Moenner, Gabriel et al**
**L'AIR LIOUIDE, Société Anonyme**
**pour l'étude et l'exploitation des procédés**
**Georges Claude**
**75, Ouai d'Orsay**
**F-75321 Paris Cédex 07 (FR)**

## Description

L'invention a pour objet des dérivés polyazotés dont les complexes métalliques constituent notamment des complexants de l'oxygène. Elle concerne également la préparation de ces dérivés et de leurs complexes métalliques ainsi que les applications des complexes en particulier pour séparer de l'oxygène d'un mélange gazeux.

Il est bien entendu que l'on entend par "oxygène" l'oxygène moléculaire, ou encore dioxygène ($O_2$) dans ce qui suit.

La demande de brevet EP-A-99.685 décrit des composés de formule générale :

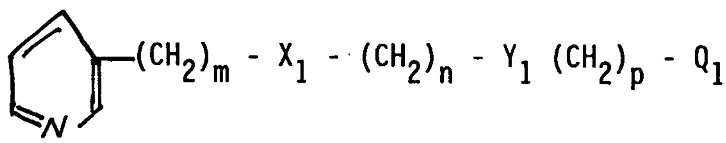

dans laquelle :

- $X_1$ et $Y_1$, représentent -O-, -S- ou $NR_2$, $R_2$ étant H, un radical alkyle en $C_1$-$C_5$ ou -CHO ;
- m est compris entre 0 et 6 ;
- n est compris entre 2 et 6 ;
- p est compris entre 0 et 6 ;
- et $Q_1$ représente $-CH_2OH$ ou $CO_2R_1$, $R_1$ étant H, un cation pharmaceutiquement acceptable, un radical alkyle, cycloalkyle, aralkyle ou phényle substitué ou non.

Ces composés sont décrits comme étant des inhibiteurs du thromboxane $A_2$.

Le brevet US-P-2.751.390 décrit des composés de formule générale :

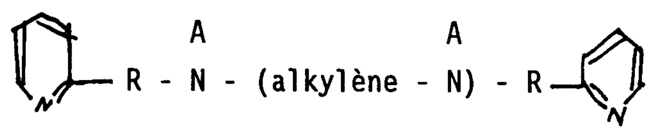

dans laquelle :

- A est un radical $-CH_2-COOH$ ;
- R est un radical aliphatique en $C_1$-$C_{12}$ ;
- "alkylène" représente :
  - . $CH_2-CH_2$,
  - . $CH-CH_2$
    $CH_3$
  - . $CH_2-CH_2-CH_2$
- n est compris entre 1 et 4.

Ces composés permettent la formation de complexes métalliques avec des ions métalliques en solution aqueuse.

La demande de brevet EP-A-287.436 décrit des composés de formule générale :

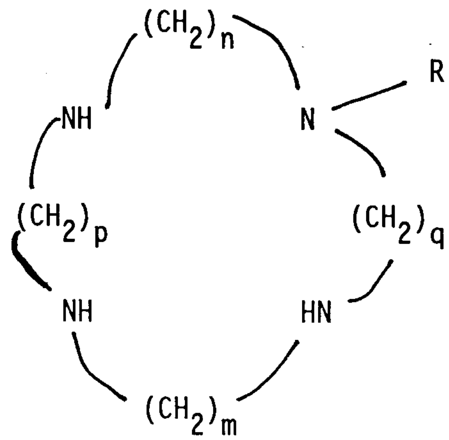

dans laquelle :

- n, m, p et q sont égaux à 2 ou 3, p et q pouvant être égaux à 4 quand n et m sont égaux à 3 ;
- R est un radical organique, notamment polymérisable.

Selon l'exemple 2 de ce document, R représente un radical $-(CH_2)_4-CN$.

Ces composés, sous forme polymère, peuvent être utilisés comme résine échangeuse d'ions, notamment pour le traitement des déchets radio-actifs, pour la purification du sang de malades atteints de la maladie de Wilson ou pour l'analyse des métaux dans l'eau de mer.

La demande de brevet australien AU-A-11685/88 décrit notamment des composés de formule $\beta$ tels que décrits ci-dessous. Ces composés sont utiles en tant qu'agents de diagnostic.

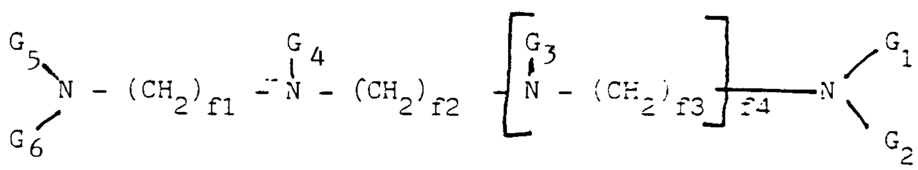 β

dans laquelle :

- $G_1$, $G_2$, $G_3$, $G_4$, et $G_5$ identiques ou différents, représentent H, ortho-$CH_2C_6H_4OH$, $-CH_2-COOH$ ou

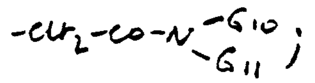

- $G_6$ représente H, ou ortho-$CH_2C_6H_4OH$,
  ou un reste

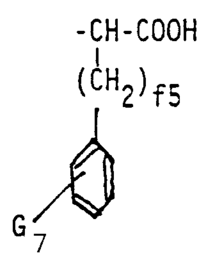

$G_7$ représentant $NO_2$, $NH_2$,

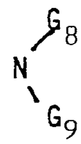

$G_8$ et $G_9$, identiques ou différents, étant H ou un radical alkyle en $C_1$-$C_4$, le maléimide ou une halotriazine substituée,

sous réserve que lorsque $G_6$ est H, l'un de $G_1$, $G_2$, $G_3$, $G_4$ ou $G_5$ est

$-CH_2-COOH$,

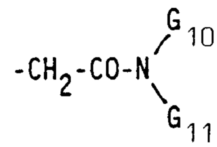

$G_{10}$ et $G_{11}$ identiques ou différents, représentant H ou un radical alkyle en $C_1$-$C_4$ ;

- $f_1$, $f_2$ et $f_3$ représentent 2 ou 3 ;
- $f_4$ représente 0 ou 1 ;
- $f_5$ représente un entier compris entre 0 et 10.

Depuis la découverte par CALVIN en 1946 de molécules du type base de Schiff coordinées au cobalt capables de fixer reversiblement l'oxygène moléculaire, de nombreux autres complexants ont été proposés pour séparer l'oxygène d'un mélange de gaz, et le récupérer sélectivement par désorption à partir du complexant.

Le complexant mis en contact avec le mélange gazeux renfermant l'oxygène peut se présenter sous forme solide ou en solution dans un milieu aqueux ou organique.

Les complexants les plus couramment utilisés en solution aqueuse sont constitués par les polyamines linéaires ou cycliques, les polyamines éthers, ou encore des acides aminés , ces composés étant complexés à un métal tel que le cobalt.

Les composés sus-mentionnés comportent au moins trois fonctions azotées ou fonctions coordinantes, c'est-à-dire capables d'établir une liaison de coordination avec un métal. Par fonction azotée, on désigne tout groupe renfermant au moins un atome d'azote à savoir un groupe amine primaire, secondaire ou tertiaire ou un hétérocycle azoté.

L'intérêt d'un complexant est lié à différentes caractéristiques physico-chimiques et en particulier à son affinité pour l'oxygène, à une concentration donnée, et à sa durée de vie.

La séparation de l'oxygène d'un mélange gazeux est réalisée, dans un premier temps, par absorption complexante de l'oxygène par un produit complexant approprié, suivie dans un second temps, par une étape de désorption de l'oxygène du complexant.

On conçoit donc aisément que l'affinité pour l'oxygène d'un complexant ne doit pas être trop élevée de manière à ce que l'étape de désorption de l'oxygène à partir du complexant s'effectue avec une dépense d'énergie la plus faible possible.

Parmi les modes de désorption les plus usuels, on distinguera les méthodes utilisant d'une part le vide, et d'autre part le transfert d'électrons.

Dans le cas d'une désorption par le vide (telle que décrite dans le brevet US 4393711 de 1982), il a été déterminé par les inventeurs de la présente demande que la pression totale du gaz lors de la dépression doit avantageusement être supérieure ou égale à 0,07 bar à 15 °C. Par conséquent, à une concentration de 0,1M, qui est la concentration de référence, l'affinité du complexant doit préférentiellement être comprise entre $10^4$ et $10^7$ $mole^{-1}.1$ pour permettre une désorption par le vide satisfaisante.

Dans le cas d'une désorption par transfert d'électrons (par exemple suivant la méthode décrite dans la demande européenne n°0283761 de 1988), le complexant doit être électro-actif (c'est-à-dire capable d'être oxydé ou réduit à une électrode) et peut présenter une affinité supérieure ou égale à $10^7$ $mole^{-1}.1$.

Par ailleurs, il est bien entendu que plus la durée de vie d'un complexant est longue, plus ce dernier sera d'intérêt commercial.

La durée de vie la plus longue observée à ce jour pour un complexant d'oxygène, en milieu aqueux, est de l'ordre d'un mois, à la concentration de $10^{-3}$M, pour le composé de formule

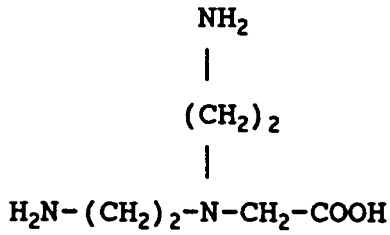

(Mc Lendon et al, Inorganic. Chemistry, vol. 14, n° 10, 2322-2326 (1975)).

A la concentration de O,1M, ce produit se dégrade beaucoup plus vite, et sa durée de vie est approximativement de l'ordre de 5 jours.

L'invention repose sur la mise en évidence par les inventeurs de l'intérêt de groupements fonctionnels donnés dans une structure de produit déterminée, au regard en particulier de l'abaissement de l'affinité et de l'augmentation de la durée de vie.

L'invention a donc pour but de fournir des dérivés polyazotés porteurs de groupements fonctionnels spécifiques capables après complexation à un métal, de fixer de l'oxygène.

Elle vise également à fournir des complexants de l'oxygène utilisables en solution aqueuse, possédant des durées de vie supérieures à un an, à la concentration de 0,1M.

L'invention a en outre pour but de fournir un procédé de préparation de ces dérivés polyazotés et de formation de complexes métalliques correspondants, exploitable industriellement.

L'invention vise encore à fournir un procédé de séparation de l'oxygène d'un mélange gazeux par absorption complexante permettant de récupérer aisément de l'oxygène dudit mélange gazeux, avec une dépense réduite en énergie pour l'étape de désorption.

Les derivés polyazotés de l'invention sont caractérisés en ce qu'ils correspondent à la formule générale (I) suivante :

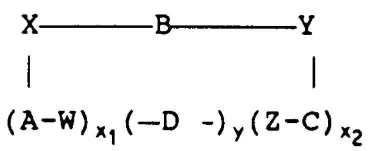

(I)

dans laquelle y vaut 0 ou 1,

a) lorsque y = 0 :

. $x_1$ et $x_2$ valent 0 ou 1, $x_1$ et $x_2$ ne pouvant pas valoir 0 simultanément ;

. les constituants A, B et C, identiques ou différents, représentent :

- une chaîne alcoylène $-(CH_2)_x-$, dans laquelle x représente un nombre entier de 1 à 4, le cas échéant substituée par un, ou plusieurs, groupe P, ou par un, ou plusieurs, groupe $P_1$, $P_1$ représentant un groupe alcoyle de 1 à 4 atomes de carbone, le cas échéant substitué par un, ou plusieurs, groupe P, P représentant :

  * un groupe -COR dans lequel R représente un hydroxyle (-OH), une amine primaire, ou substituée par un, ou deux, groupe alcoyle de 1 à 4 atomes de carbone, une fonction -OR′ dans laquelle R′ représente soit un groupe alcoyle de 1 à 4 atomes de carbone, soit un cycle aromatique de 6 à 14 atomes de carbone le cas échéant substitué en position ortho et/ou méta et/ou para par un atome d'halogène, un groupe alcoyle de 1 à 4 atomes de carbone, alcoxy de 1 à 4 atomes de carbone, hydroxy, un groupe aryle, un hétérocycle aromatique, un groupe nitro, un groupe $-(CH_2)_t-COR$ dans lequel R a la signification indiquée ci-dessus, et t représente un nombre entier de 0 à 4, ou une amine primaire ou substituée par un, ou deux, groupe alcoyle de 1 à 4 atomes de carbone ;

  * un cycle aromatique de 6 à 14 atomes de carbone, le cas échéant substitué en position ortho et/ou méta et/ou para par un atome d'halogène, un groupe alcoyle de 1 à 4 atomes de carbone, alcoxy de 1 à 4 atomes de carbone, hydroxy, un groupe aryle, un hétérocycle aromatique, un groupe nitro, un groupe $-(CH_2)_t-COR$ dans lequel R a la signification indiquée ci-dessus, et t représente un nombre entier de 0 à 4, ou une amine primaire ou substituée par un, ou deux, groupe alcoyle de 1 à 4 atomes de carbone ;

  * un hétérocycle aromatique, notamment azoté, de 4 à 12 atomes de carbone, le cas échéant substitué en position ortho, et/ou méta, et/ou para, par un atome d'halogène, un groupe

alcoyle de 1 à 4 atomes de carbone, alcoxy de 1 à 4 atomes de carbone, hydroxy, un hétérocycle aromatique, un groupe aryle, un groupe nitro, un groupe $-(CH_2)_t-COR$ dans lequel R a la signification indiquée ci-dessus et t représente un nombre entier de 0 à 4, ou une amine primaire ou substituée par un, ou deux, groupe alcoyle de 1 à 4 atomes de carbone,

* un groupe amine primaire,ou substitué par un, ou deux, groupe alcoyle de 1 à 4 atomes de carbone,
* un groupe CN,
* un groupe alcoyle de 1 à 4 atomes de carbone,
* un groupe alcoxy de 1 à 4 atomes de carbone,
* une fonction hydroxyle,
* un groupe nitro,
* un atome d'halogène,

- un cycle aromatique de 6 à 14 atomes de carbone dont 2 atomes de carbone sont engagés respectivement dans une liaison avec W et X dans le cas de A, ou avec X et Y dans le cas de B, ou avec Y et Z dans le cas de C, ce cycle aromatique étant le cas échéant substitué par un, ou plusieurs, groupe P et/ou $P_1$, P et $P_1$ ayant les significations indiquées ci-dessus,
- un groupe du type

$$-(CH_2)_{x_3}-V-(CH_2)_{x_4}-$$

dans lequel $x_3$ et $x_4$ sont des nombres entiers variant de 1 à 3, et V représente un cycle aromatique de 6 à 14 atomes de carbone, le cas échéant substitué par un, ou plusieurs, groupe P et/ou $P_1$, P et $P_1$ ayant les significations indiquées ci-dessus,

. les constituants X et Y, identiques ou différents, représentent respectivement :

- lorsque $x_1$ et $x_2$ valent respectivement 1 :
  * un groupe

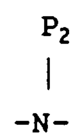

dans lequel $P_2$ représente un atome d'hydrogène ou le groupe P ou $P_1$, P et $P_1$ ayant les significations indiquées ci-dessus,

  * un hétérocycle aromatique azoté, de 4 à 12 atomes de carbone dont 2 atomes de carbone sont engagés respectivement dans une liaison avec A et B dans le cas de X, ou avec B et C dans le cas de Y, cet hétérocycle étant le cas échéant, substitué par un, ou plusieurs, groupe P et/ou $P_1$, P et $P_1$ ayant les significations indiquées ci-dessus,
- lorsque $x_1 = 0$ ou lorsque $x_2 = 0$, : le substituant Y ou X respectivement terminal représente
  * un groupe -COR dans lequel R a la signification indiquée ci-dessus.
  * un hétérocycle aromatique azoté, de 4 à 12 atomes de carbone, le cas échéant substitué en position ortho, et/ou méta, et/ou para, par un, ou plusieurs, groupe P et/ou $P_1$, P et $P_1$ ayant les significations indiquées ci-dessus,
  * un groupe CN,
  * un groupe

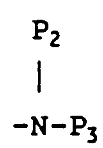

dans lequel $P_2$ et $P_3$, identiques ou différents, représentent un atome d'hydrogène ou le groupe P ou $P_1$, P et $P_1$ ayant les significations indiquées ci-dessus,

. W et Z, identiques ou différents, représentent respectivement les groupes indiqués ci-dessus pour X et Y lorsque $x_1$ et $x_2$ valent respectivement zéro, ou encore un cycle aromatique de 6 à 14 atomes de carbone, le cas échéant substitué par un, ou plusieurs, groupe P et/ou $P_1$, P et $P_1$ ayant les significations indiquées ci-dessus, à l'exclusion des dérivés de formule

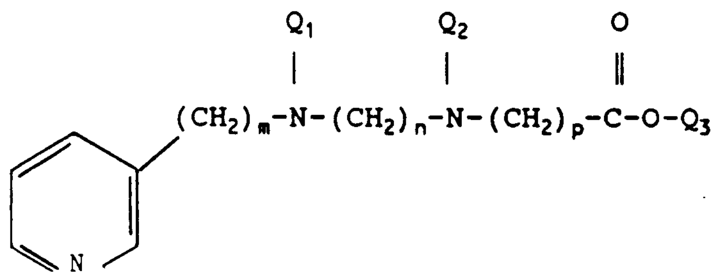

dans laquelle :

- m et p sont des nombres entiers variant de 1 à 4,
- n est un nombre entier variant de 2 à 4,
- $Q_1$ et $Q_2$, identiques ou différents, représentent un atome d'hydrogène ou un groupe alcoyle de 1 à 4 atomes de carbone,
- $Q_3$ représente un atome d'hydrogène, un groupe alcoyle de 1 à 4 atomes de carbone, ou un groupe phényle ou phényle substitué par un, deux, ou trois substituants choisis indépendamment entre un atome de chlore et un groupe alcoyle de 1 à 3 atomes de carbone ;

b) lorsque y = 1,

. $x_1$ et $x_2$ valent 1

. D a la même signification que A, B et C définis ci-dessus,

. W, X, Y et Z, identiques ou différents, représentent respectivement :

* les groupes indiqués ci-dessus pour X et Y lorsque $x_1$ et $x_2$ valent respectivement 1, ou
* un cycle aromatique de 6 à 14 atomes de carbone dont 2 atomes de carbone sont engagés respectivement dans une liaison avec A et D dans le cas de W, ou avec B et A dans le cas de X, ou avec C et B dans le cas de Y, ou avec D et C dans le cas de Z, ce cycle aromatique étant le cas échéant substitué par un, ou plusieurs groupe P et/ou $P_1$, P et $P_1$ ayant les significations indiquées ci- dessus, ou
* un groupe du type

$$-(CH_2)_{x_3}-V-(CH_2)_{x_4}-$$

dans lequel $x_3$ et $x_4$ sont des nombres entiers variant de 1 à 3, et V représente un cycle aromatique de 6 à 14 atomes de carbone, le cas échéant substitué par un, ou plusieurs, groupe P et/ou $P_1$, P et $P_1$ ayant les significations indiquées ci-dessus,

* un groupe

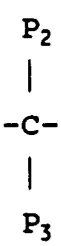

dans lequel $P_2$ et $P_3$
ont les significations indiquées ci-dessus,
étant entendu que, aussi bien lorsque y = 0 ou 1, les dérivés polyazotés correspondant comportent 4 ou 5 fonctions coordinantes dont une est représentée par le groupe -COR , R ayant la signification indiquée ci-dessus, les autres fonctions coordinantes étant des groupes

azotés choisis parmi:

- les groupes

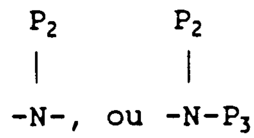

dans lesquels $P_2$ et $P_3$ ont les significations indiquées ci-dessus,

- les hétérocycles aromatiques azotés de 4 à 12 atomes de carbone, le cas échéant substitués par un, ou plusieurs, groupe P et/ou $P_1$ tels que définis ci-dessus, sous réserve que lorsque les dérivés polyazotés comportent 4 fonctions coordinantes,

* soit les trois groupes azotés sont des groupes

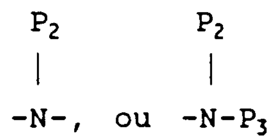

tels que définis ci-dessus, les dérivés polyazotés correspondant comportant alors un cycle aromatique de 6 à 14 atomes de carbone, le cas échéant substitué par un, ou plusieurs, groupe P et/ou $P_1$ tels que définis ci-dessus,

* soit un au moins parmi les trois groupes azotés représente un hétérocycle aromatique azoté, le cas échéant substitué par un, ou plusieurs, groupe P et/ou $P_1$ tels que définis ci-dessus,

les dérivés de formule $\alpha$ :

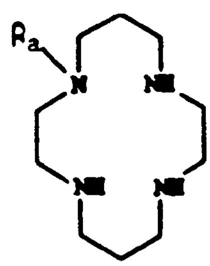

dans laquelle $R_a$ représente :

. un groupe $-(CH_2)_n-COOH$ dans lequel n est un nombre entier variant de 1 à 3.

. ou un groupe

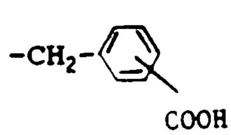

les composés de formule $\beta$ tels que définis et avant dans la description de l'art antérieur, étant exclus.

Les hétérocycles aromatiques constituant les groupes azotés définis ci-dessus, sont plus particulièrement choisis parmi les dérivés des groupes suivants : pyridine, imidazole, quinoléine, isoquinoléine, pyrrole, pyrimidine, pyrazine, pyridazine, indole, carbazole, purine, phénazine, thiazole, isothiazole, oxazole, isoxazole.

Parmi les cycles aromatiques définis ci-dessus, on distinguera notamment les dérivés des groupes suivants: benzène, biphényle, naphtalène, azulène, anthracène, phénanthracène.

L'invention vise également les sels de ces dérivés avec des acides minéraux ou organiques. Il s'agit en particulier d'halogénures, à savoir de chlorures ou de bromures, ou encore d'iodures ou de fluorures, de sulfates, de phosphates, de nitrates, ou d'acétates et de citrates.

Les dérivés polyazotés de l'invention ou leurs sels avec des acides minéraux ou organiques peuvent exister sous forme d'hydrates.

Par la présence d'une fonction coordinante du type -COR, notamment du type -COOH, et de 3 ou 4 autres fonctions coordinantes de type azoté, les dérivés de l'invention constituent des réactifs de choix pour la formation, en association avec un métal de transition de complexes métalliques très stables.

Des dérivés préférés de l'invention sont ceux correspondant à la formule suivante :

$$(W-A)_{x_1}-X-B-Y-(C-Z)_{x_2} \qquad (Ia)$$

dans laquelle W, A, X, B, Y, C, Z, $x_1$ et $x_2$ ont les significations indiquées ci-dessus lorsque y = 0.

Parmi les dérivés de formule Ia sus-mentionnés, on distinguera plus particulièrement ceux caractérisés en ce que $x_1 = x_2 = 1$ et répondant à la formule Ib,

W-A-X-B-Y-C-Z.

D'autres dérivés de formule Ia sont caractérisés en ce que $x_1$ ou $x_2$ est égal à O.

Les dérivés dans lesquels $x_1 = 0$ répondent à la formule Ic

X-B-Y-C-Z.

Lorsque $x_2 = 0$, les dérivés correspondants répondent à la formule Id

W-A-X-B-Y.

Des dérivés particulièrement avantageux de l'invention, répondant aux formules Ib, Ic ou Id sont caractérisés en ce qu'ils possèdent 4 fonctions coordinantes.

Parmi les dérivés de formule Ib, dans lesquels $x_1 = x_2 = 1$, on distinguera plus particulièrement ceux caractérisés en ce que l'un de W ou de Z représente le groupe -COR ,R ayant la signification indiquée ci-dessus, tandis que l'autre de W ou de Z, ainsi que X et Y, représentent respectivement un groupe azoté.

Avantageusement, les composés sus-mentionnés sont caractérisés en ce que :

- l'un de W ou de Z représente :
  . soit un groupe pyridyle, pyrimidyle ou imidazolyle
  . soit un groupe aminobenzyle
- X et Y représentent respectivement un groupe -NH-,
- A,B et C représentent respectivement un groupe $-(CH_2)_x-$ , x étant un nombre entier variant de 1 à 4.

Parmi les dérivés de formule Ic, on distinguera plus particulièrement ceux dans lesquels :

- l'un de X ou de Z représente le groupe -COR , R ayant la signification indiquée ci-dessus tandis que l'autre de X ou de Z, ainsi que Y, représentent un groupe azoté, et
- l'un de B, ou de C, ou de Y, est substitué par un groupe alcoyle de 1 à 4 atomes de carbone substitué par un groupe azoté.

Avantageusement, les composés de formule Ic sont caractérisés en ce que

* l'un de X ou de Z représente :
  . soit un groupe pyridyle, pyrimidyle ou imidazolyle,
  . soit un groupe aminobenzyle,
  . soit une amine primaire $-NH_2$,

* Y représente un groupe

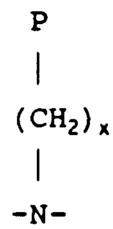

dans lequel x représente un nombre entier de 1 à 4, et P représente :

. soit un groupe pyridyle, pyrimidyle ou imidazolyle,

. soit un groupe aminobenzyle,

. soit une amine primaire $-NH_2$,

* B et C représentent respectivement un groupe $-(CH_2)_x-$, x étant défini ci-dessus,

Parmi les dérivés de formule Id, on distinguera plus particulièrement ceux dans lesquels

- l'un de W ou de Y représente le groupe -COR tel que défini ci-dessus, tandis que l'autre de W ou de Y, ainsi que X, représentent un groupe azoté, et
- l'un de A, ou de B, ou de X est substitué par un groupe alcoyle de 1 à 4 atomes de carbone substitué par un groupe azoté.

Des composés préférés de formule Id sont caractérisés en ce que

* l'un de W ou de Y représente

. soit un groupe pyridyle, pyrimidyle ou imidazolyle,

. soit un groupe aminobenzyle,

. soit une amine primaire $-NH_2$,

* X représente un groupe

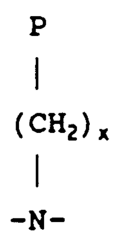

dans lequel x représente un nombre entier variant de 1 à 4 et P représente :

. soit un groupe pyridyle, pyrimidyle ou imidazolyle,

. soit un groupe aminobenzyle,

. soit une amine primaire $-NH_2$,

* A et B représentent respectivement un groupe $-(CH_2)-_x$, x étant défini ci-dessus.

Selon un aspect particulièrement avantageux de l'invention, les composés de formule Ia sus-mentionnés comportent 5 fonctions coordinantes.

Parmi ces derniers, des composés préférés répondent à la formule Ib dans laquelle

- W, X, Y, et Z représentent un groupe azoté,
- l'un de W ou de Z est substitué par un groupe alcoyle de 1 à 4 atomes de carbone substitué par un groupe -COR, R ayant la signification indiquée ci-dessus.

Avantageusement, les composés sus-mentionnés sont caractérisés en ce que :

- l'un de W ou de Z représente :

  . soit un groupe pyridyle, pyrimidyle ou imidazolyle,

  . soit un groupe aminobenzyle,

  . soit une amine primaire $-NH_2$,
- tandis que l'autre de W ou de Z représente un groupe $-NH-(CH_2)_x-COR$ dans lequel R et x ont les significations indiquées ci-dessus,
- X et Y représentent respectivement un groupe -NH-,
- A, B et C représentent respectivement un groupe $-(CH_2)-_x$, x étant défini ci-dessus.

Parmi les dérivés de formule Ib comportant 5 fonctions coordinantes, on distinguera plus particulièrement encore ceux caractérisés en ce que :

- l'un de W ou de Z représente le groupe -COR, R ayant la signification indiquée ci-dessus
- tandis que l'autre de W et de Z, ainsi que X et Y représentent un groupe azoté, et
- l'un de A, X, B, Y, ou C est substitué par un groupe alcoyle de 1 à 4 atomes de carbone substitué par un groupe azoté.

Avantageusement, les composés sus-mentionnés sont caractérisés en ce que :

- l'un de W ou de Z représente :
  . soit un groupe pyridyle, pyrimidyle ou imidazolyle,
  . soit un groupe aminobenzyle,
  . soit une amine primaire $-NH_2$,
- l'un de X ou de Y représente -NH- tandis que l'autre de X et de Y représente

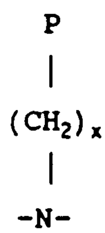

dans lequel x représente un nombre entier variant de 1 à 4 et P représente :
  . soit un groupe pyridyle, pyrimidyle ou imidazolyle,
  . soit un groupe aminobenzyle,
  . soit une amine primaire $-NH_2$,
- A, B et C représentent respectivement $-(CH_2)_x-$, x étant défini ci-dessus.

Une autre catégorie de dérivés préférés de l'invention est représentée par ceux correspondant à la formule générale IIa suivante :

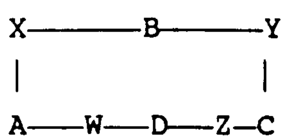

(IIa)

dans laquelle W, A, X, B, Y, C, Z et D ont les significations indiquées ci-dessus lorsque y = 1.

Des dérivés de formule IIa particulièrement avantageux sont ceux comportant 5 fonctions coordinantes et caractérisés en ce que:

- W, X, Y, et Z représentent respectivement un groupe azoté,
- l'un de W, X, Y ou Z étant substitué par un groupe alcoyle de 1 à 4 atomes de carbone lui-même substitué par le groupe -COR, R ayant la signification indiquée ci-dessus.

Avantageusement, les dérivés sus-mentionnés sont caractérisés en ce que :

- W, X, Y et Z représentent respectivement
  * soit un groupe pyridyle, pyrimidyle ou imidazolyle, le cas échéant substitué par un groupe alcoyle de 1 à 4 atomes de carbone lui-même substitué par le groupe -COR, R ayant la signification indiquée ci-dessus.
  * soit un groupe -NH-, ou

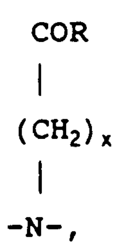

x et R ayant les significations indiquées ci-dessus.

D'une manière générale, l'ensemble des dérivés préférés de l'invention, décrits ci-dessus, comportent avantageusement, à titre de fonction coordinante du type -COR, un groupe carboxyle -COOH.

Des dérivés de formule Ia particulièrement avantageux de l'invention sont ceux comportant un cycle aromatique, notamment un groupe phényle, et un groupe - COR, notamment un carboxyle, placés respectivement aux extrémités de la chaîne principale de ces composés (c'est-à-dire la chaîne constituée par W et/ou X, Y, et/ou Z). Parmi de tels dérivés, on distinguera plus particulièrement ceux caractérisés en ce que :

- lorsque $x_1 = x_2$ (dérivés de formule Ib), W ou Z représente un groupe phényle ou un groupe aminobenzyle;
- lorsque $x_1 = 0$, (dérivés de formule Ic) ou lorsque $x_2 = 0$ (dérivés de formule Id), X ou Z, dans le premier cas, ou , W ou Y, dans le second cas, représente un groupe phényle ou un groupe aminobenzyle.

L'aspect préféré de tels dérivés provient du fait que la présence de ce cycle aromatique en addition des autres fonctions coordinantes, confère une très bonne stabilité aux complexes métalliques obtenus à partir de ces dérivés.

A titre d'exemple de tels dérivés, on citera notamment celui dont la formule est la suivante :

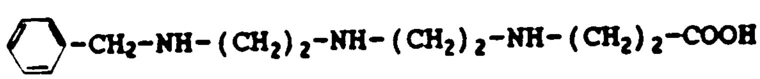

Des dérivés de formule IIa particulièrement avantageux sont ceux pour lesquels W, X, Y et Z représentent une amine secondaire ou tertiaire, l'un de ces 4 constituants étant substitués par un groupe $-(CH_2)_x$-COR, x et R étant définis ci-dessus, tandis qu'un autre de ces 4 constituants, est substitué par un groupe alcoyle de 1 à 4 atomes de carbone substitué par un cycle aromatique, notamment un groupe phényle.

Les raisons de l'aspect préféré de ces dérivés sont identiques à celles précisées ci-dessus pour ceux de formule Ia comportant un groupe -COR et un cycle aromatique.

Il faut préciser également que cette structure de type cyclame des dérivés de formule II précisée ci-dessus est particulièrement avantageuse pour la formation de dérivés métalliques ; une telle structure semble en effet, orienter de façon optimale les fonctions coordinantes vis-à-vis du métal auquel ces fonctions vont se lier pour former le complexe métallique (ou complexant).

L'aspect avantageux de la présence d'un cycle aromatique dans la structure des dérivés sus-mentionnés, réside dans le fait que le caractère hydrophobe de ce cycle aromatique éloigne les molécules d'eau environnantes, empêchant ainsi l'eau d'être activée au niveau du complexant.

A titre d'exemple, un dérivé de type cyclame particulièrement préféré est celui représenté par la formule suivante :

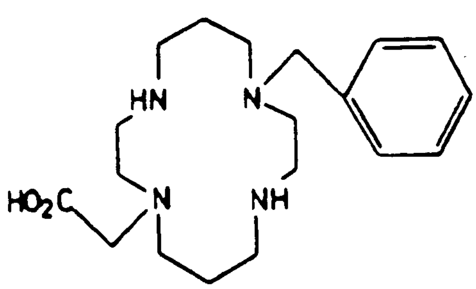

La préparation des dérivés de l'invention est essentiellement basée sur des condensations successives mettant en jeu les réactions classiques de la chimie organique.

Ainsi, on a avantageusement recours à l'une des réactions de condensation a) à c) suivantes pour la synthèse des dérivés de l'invention, et, plus particulièrement ceux de formule Ia.

a) Dans un premier mode de condensation, on fait réagir un dérivé réactif 1, plus spécialement un halogénure $R_1$-Z, avec une amine primaire 2 $H_2N$-$R_2$ ou une amine secondaire 3

P (ou $P_1$)
|
$H-N-R_2$

selon le schéma :

P (ou $P_1$)
|
$R_1-Z + H_2NR_2$ ou $H-N-R_2$

P (ou $P_1$)
|
-----> $R_1-NHR_2$ ou $R_1-N-R_2$

Dans l'halogénure $R_1Z$, Z représente plus spécialement du chlore ou du brome.

$R_1$ correspond à une partie du dérivé à synthétiser et est choisi parmi les groupes correspondant aux entités W-A-,

$$(W-A)_{x_1}-X-B-, \quad (W-A)_{x_1}-X-B-Y-C-,$$

ou -A-X-B-Y-C-Z,

$$-B-Y-(C-Z)_{x_2}$$

ou -C-Z.

Les dérivés <u>2</u> ou <u>3</u> sus-mentionnés sont alors choisis respectivement parmi les groupes dans lesquels

P (ou $P_1$)
|
$-N-R_2$,

ou $-N-R_2$ correspondent aux entités

$$-X-B-Y-(C-Z)_{x_2}, \quad -Y-(C-Z)_{x_2},$$

-Z ou W-,

$$(W-A)_{x_1}-X-, \text{ et } (W-A)_{x_1}-X-B-Y-,$$

dans lesquels W, A, X, B, Y, C, Z, P, $P_1$, $x_1$ et $x_2$ ont les significations indiquées ci-dessus lorsque y = 0.

Cette réaction de condensation est avantageusement réalisée en milieu basique.

Les réactifs sont mis en oeuvre selon des quantités équimolaires, et la température est de préférence de 80 à 120 °C.

b) Selon un autre type de condensation, on fait réagir un aldéhyde <u>4</u> de formule $R_1$-CHO dans laquelle $R_1$ a la signification indiquée ci-dessus en a), avec une amine primaire <u>2</u> telle qu'indiquée ci-dessus en a), ce qui conduit à une base de Schiff, puis on effectue une étape de réduction. Le schéma de cette

condensation peut être illustré comme suit :

$R_1$-CHO + $H_2$N-$R_2$ ---> $R_1$-CH = N-$R_2$ ---> $R_1$-$CH_2$-NH-$R_2$

Dans le cas des condensations du type a) ou b) sus-mentionnées, la fonction -COR est, le cas échéant, protégée à l'aide de groupes protecteurs appropriés, tels qu'un ester, ladite fonction coordinante étant déprotégée à l'aide d'une réaction d'hydrolyse après la réaction de condensation.

En variante, l'introduction de la fonction coordinante du type -COR soit sur les dérivés intermédiaires utilisés dans les condensations a) et b) sus-mentionnées, soit sur les dérivés finaux obtenus par ces condensations a) et b) (réalisées dans ce dernier cas à partir de dérivés intermédiaires ne comportant pas de groupe -COR), est avantageusement réalisée par condensation (ci-après désignée par condensation (α) d'un halogénure d'ester ou d'acide du type Z-$(CH_2)_x$-COO(H, R′) dans lequel x, Z et R′ sont tels que définis ci-dessus, sur le dérivé aminé intermédiaire ou final (dont certaines fonctions amines sont, le cas échéant protégées) selon le schéma:

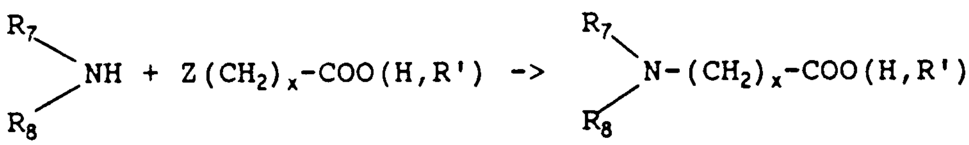

$R_7$ et $R_8$ représentant respectivement un atome d'hydrogène, ou un des groupes indiqués ci-dessus pour $R_1$.

Lorsque les dérivés intermédiaires utilisés dans les condensations a) et b) sus-mentionnées ne comportent pas de groupe -COR, il est particulièrement avantageux de réaliser les condensations a) et b) sus-mentionnées à l'aide d'une amine primaire 2 $H_2$N-$R'_2$ (dans le cas des condensations a) et b)) ou d'une amine
secondaire 3

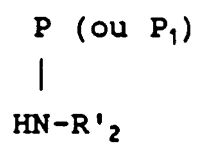

(dans le cas de la condensation a)), ces dérivés aminés étant choisis respectivement, en fonction de $R_1$, parmi les groupes
pour lesquels -NH-$R'_2$, ou

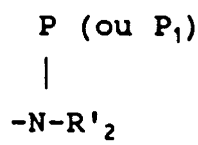

correspondent aux entités

$$-X-B-Y-(C-Z)_{x_2},\quad -Y-(C-Z)_{x_2},$$

-Z ou W-,

$$(W-A)_{x_1}-X-,\ \text{et}\ \ (W-A)_{x_1}-X-B-Y-,$$

dans lesquels un des constituants W, A, X, B, Y, C et Z est substitué par un groupe nitrile du type

$$-(CH_2)_x-CN,$$

x ayant la signification indiquée ci-dessus.

Après l'étape de condensation, et le cas échéant de réduction, le groupe nitrile est hydrolysé en fonction acide -COOH, cette dernière subissant, le cas échéant, une étape de fonctionnalisation pour obtenir un groupe -COR.

A titre illustratif, l'obtention des dérivés aminés du type $H_2N-R'_2$, et

$$\begin{array}{c} P\ (ou\ P_1) \\ | \\ HN-R'_2 \end{array}$$

est réalisée de la manière suivante (ci-après désignée par condensation ($\beta$) : condensation de l'acrylonitrile avec une amine primaire du type $R_4-NH_2$ dans laquelle $R_4$ correspond à l'une des entités X-B-Y-C-, Y-C-, -B-Y, -A-X-B-Y, selon le schéma :

$R_4-NH_2$ + $CH_2=CHCN$ -> $R_4-NH-CH_2-CH_2-CN$.

Dans le cas où $R_4$ représente une amine primaire $H_2N-R_5-$, (la fonction $NH_2$ étant, le cas échéant, protégée à l'aide d'une groupe protecteur du type tosyle, mésityle, ou phtalimide, puis déprotégée après condensation avec l'acrylonitrile) $R_5$ correspondant à l'une des entites -B-Y-C-, -C-, -B-, -A-X-B-, la quantité d'acrylonitrile utilisée dans la réaction decrite ci-dessus doit être en défaut par rapport à celle de la diamine $H_2N-R_5-NH_2$ (rapport molaire acrylonitrile/diamine de l'ordre de 1:3) pour obtenir le nitrile de formule :

$H_2N-R_5-NH-CH_2-CH_2-CN$

Il faut préciser que la simple condensation ($\beta$) de l'acrylonitrile sur un dérivé d'amine de type $R_4-NH_2$ dans les conditions définies ci-dessus, suivie de l'hydrolyse selon ($\gamma$) de la fonction nitrile en fonction acide, permet d'obtenir directement un dérivé de l'invention si le dérivé d'amine de départ comprend 4 fonctions azotées coordinantes.

En variante, la synthèse de dérivés de l'invention peut être réalisée à partir d'un dinitrile, par exemple un dinitrile de formule NC-A-X-B-Y-C-CN qui après hydrogénation conduit au dérivé de formule $H_2N-CH_2-A-X-B-Y-CH_2-NH_2$. A titre illustratif, un dinitrile particulièrement intéressant à utiliser en tant que dérivé intermédiaire peut être obtenu par condensation (ci-après désignée par condensation ($\delta$)) de l'acrylonitrile sur une diamine de formule $H_2N-B-NH_2$, l'acrylonitrile étant en excès par rapport à la diamine (rapport molaire acrylonitrile/diamine de l'ordre de 3:1) ce qui conduit à l'obtention du dinitrile de formule $NC-(CH_2)_2-NH-B-NH-(CH_2)_2-CN$. Une étape supplémentaire d'hydrogénation du dinitrile en présence de nickel de Raney conduit à l'obtention d'un dérivé de l'invention de formule $H_2N-(CH_2)_3-NH-B-NH-(CH_2)_3-NH_2$.

Le cas échéant, préalablement à l'étape d'hydrogénation sus-mentionnée, le dinitrile est soumis à une, ou plusieurs, étape(s) de condensation avec un halogénure du type $R_1-Z$ ayant la signification indiquée ci-dessus, ou avec un halogénure d'acide ou d'ester selon la méthode ($\alpha$) pour introduire une fonction coordinante du type -COR sur le dinitrile.

c) Une autre condensation, utilisable dans la synthèse des dérivés de l'invention, comprend la réaction d'un ou de deux dérivés réactifs $\underline{1}$ de formule $R_1-Z$, dans laquelle $R_1$ représente W-A-, W-A-X-B-, X-B-, -B-Y, -B-Y-C-Z, avec une pipérazinone, suivie d'une réaction d'hydrolyse de la fonction amide.

Cette condensation donne lieu soit à une monosubstitution selon le schéma :

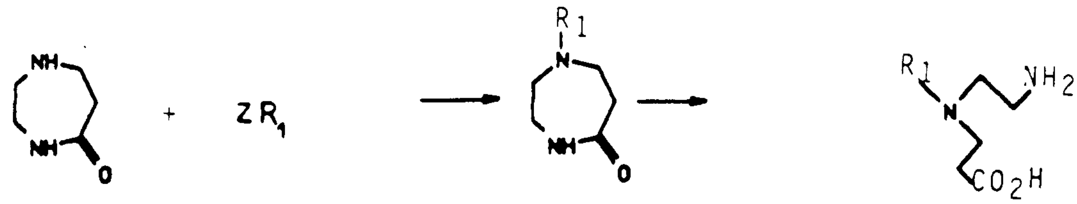

, soit à une disubstitution selon le schéma :

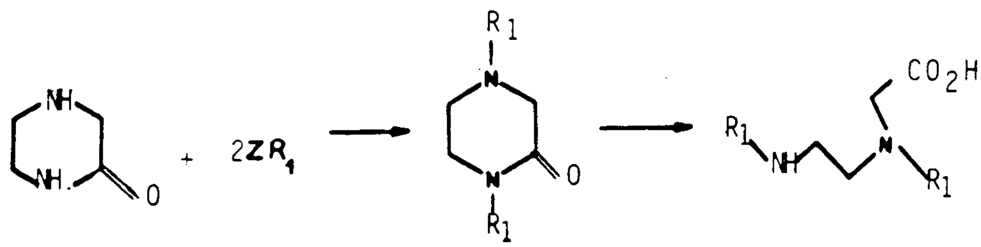

d) Pour obtenir des dérivés cycliques de formule IIa, monosubstitués, on effectue la condensation d'un dérivé 5 portant deux fonctions amine primaire correspondant à la formule $H_2N-T_1-R_9-T_2-NH_2$, avec un dialdéhyde 6, correspondant à la formule $OHC-T_1-R_{10}-T_2-CHO$, $T_1$ et $T_2$, identiques ou différents, représentant une chaîne alcoylène $-(CH_2)_x-$, dans laquelle x représente un nombre entier de 1 à 3, le cas échéant substituée par un, ou plusieurs, groupe P, ou par un, ou plusieurs, groupe $P_1$, P et $P_1$ étant tels que définis ci-dessus, $R_9$ et $R_{10}$, identiques ou différents, ont les significations indiquées ci-dessus pour X ou Y lorsque y = 1. Puis on soumet le produit cyclisé résultant à une étape de réduction.

Pour préparer la diamine 5, on a avantageusement recours au dinitrile correspondant,de formule $NC-T_1-R_9-T_2-CN$, soumis à une étape de réduction.

La réduction est effectuée à l'aide d'un agent réducteur en présence de catalyseur d'hydrogénation, le cas échéant, sous pression.

Des résultats satisfaisants sont obtenus en opérant avec de l'hydrogène. Le catalyseur est constitué par exemple par le nickel de Raney.

Le produit sous forme de dinitrile permet d'introduire aisément, selon les réactions classiques de la chimie organique, les substitutions souhaitées sur les entités présentes sur la chaîne.

Pour favoriser la réaction du dialdéhyde avec la diamine, on utilise avantageusement un réactif de couplage. Parmi les agents appropriés, on citera le chlorure de nickel hexahydraté.

Pour l'étape de réduction, on a recours à un agent réducteur utilisé de préférence avec un catalyseur d'hydrogénation.

Un mode de synthèse d'un dérivé cyclique de l'invention sera plus particulièrement détaillé dans les exemples de réalisation de dérivés de l'invention qui suivent.

Les dérivés définis ci-dessus forment des complexes métalliques capables de fixer réversiblement l'oxygène.

L'invention vise donc des complexes métalliques, le cas échéant sous forme d'hydrates, formés par un dérivé d'amine tel que défini ci-dessus associé à un métal.

Des métaux appropriés à la mise en oeuvre de l'invention comprennent les métaux de transition, en particulier le cobalt, le chrome, le fer, le manganèse, le ruthénium, le rhodium, le cuivre, le nickel, le palladium, l'osmium, et le platine.

Des métaux de transition préférés sont choisis parmi le cobalt, le cuivre, le nickel, le manganèse et le fer.

Dans les complexes métalliques de l'invention, les métaux de transition préférés, indiqués ci-dessus, sont, sous forme réduite, à l'état d'oxydation +2 ou +1 avant la réaction avec l'oxygène.

Les complexes métalliques mis en oeuvre selon l'invention sont donc chargés positivement. Leur charge est équilibrée par un contre-ion ; il s'agit d'un anion inerte vis-à-vis des réactions chimiques ou électrochimiques utilisées pour désorber l'oxygène, pour le transporter ou le régénérer. Des anions particulièrement appropriés sont choisis parmi les halogénures, les sulfates, les phosphates, les nitrates, les

carbonates, les perchlorates, les tétrafluoroborates, les hexafluorophosphates.

Les halogénures sont de préférence des ions bromure ou des ions chlorure.

Ils peuvent être également constitués par des iodures ou des fluorures.

Les anions peuvent être également des anions organiques, par exemple des acétates, des citrates, des triflates ou des méthane-sulfonates.

D'une manière avantageuse, de tels complexes métalliques présentent une solubilité en milieu aqueux supérieure ou égale à 0,5M et préférentiellement de l'ordre de 1M.

A une concentration de 1M, la viscosité de la solution aqueuse de ces complexes est inférieure ou égale à environ 2 fois la viscosité de l'eau.

En outre, ces complexes métalliques sont capables de fixer l'oxygène lorsqu'ils sont mis en présence d'un mélange gazeux où la pression partielle d'oxygène est avantageusement de l'ordre de 0,2bar (c'est-à-dire équivalent à la pression atmosphérique), notamment à la température de 15 ° C.

Le coefficient de diffusion de ces complexes dans le milieu n'excède pas 5 fois le coefficient de diffusion de l'oxygène dissous dans le même milieu.

La durée de vie de ces complexes est avantageusement supérieure à un mois, et peut atteindre une année ou plus.

Pour former ces complexes, on fait avantageusement réagir, en l'absence d'oxygène, le métal sous forme de sel avec un composé polyazoté de l'invention. Des sels permettant d'effectuer aisément la réaction de complexation sont notamment des acétates, des nitrates, des halogénures ou des sulfates.

La réaction est avantageusement réalisée en solution aqueuse. On utilise plus spécialement des quantités équimolaires de composés polyazotés et de sel métallique.

Selon le pH auquel se trouve la solution et selon le schéma de coordination favorisé par le métal, des équilibres différents interviennent entre le composé polyazoté, ou encore ligand, le métal et la forme complexée.

Si l'on désigne par LH le ligand sous sa forme neutre et par $M^{2+}$, le métal, on observe entre autres les équilibres suivants :

$$LH + M^{2+} \leftrightarrows (LHM)^{2+} \; ; \; L^{-} + M^{2+} \leftrightarrows (LM)^{+} \; ; \; LH_2^{+} + M^{2+} \leftrightarrows (LH_2M)^{3+} \; ; \; 2LH_2^{+} + M^{2+} \rightleftarrows ((LH_2)_2M)^{4+}.$$

A chaque équilibre correspond une constante dite constante de métallation.

La quantité de complexe formé résulte d'un équilibre de coordination et est donc fonction de la température à laquelle la solution est maintenue, du rapport des concentrations initiales des deux entités réactives et du pH de la solution.

Le pH de la solution est d'autant plus important qu'il conditionne la réactivité du ligand avec le sel métallique. En effet, les fonctions amine, lorsqu'elles sont protonées, ne coordinent que très faiblement un métal, alors que lorsqu'elles sont déprotonées, elles participent pleinement à la réaction de coordination. Par conséquent, les formes préférées des dérivés de l'invention seront du type LH ou $L^{-}$ .Le choix du pH de la solution aqueuse pour la préparation du complexe métallique se fera donc pour chaque produit par évaluation préalable des constantes de protonation du ligand.

L'étude par les inventeurs des complexes métalliques des dérivés de formule I et ceux de formule $\alpha$ a mis en évidence leurs propriétés de fixation réversible de l'oxygène moléculaire.

Grâce à leur stéréochimie, au nombre et à la combinaison des groupes donneurs d'électrons (fonctions coordinantes) et, le cas échéant, des groupes électro-attracteurs (halogène, groupe nitro, groupe alcoxy), l'ensemble de ces complexes métalliques, appelés ci-après complexes II et notés X, sont particulièrement utiles comme transporteurs d'oxygène. Ces complexes présentent avantageusement une affinité pour l'oxygène moléculaire comprise entre $10^4$ et $10^7$ $mole^{-1}.1$ et des durées de vie supérieures à 1 mois, en milieu aqueux.

Il est intéressant d'observer que l'affinité du dérivé de formule

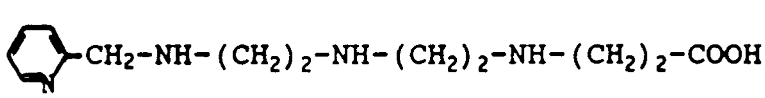

est de $10^7$ $mole^{-1}.l$ alors que le produit correspondant possédant un autre groupe pyridyle à la place de la fonction -COOH possède une affinité de $10^{11}$ $mole^{-1}.l$ (MARTELL et al, J. Chem.Soc. Chem. Commun. (1984) 335). Ce résultat souligne l'intérêt de la présence d'un groupe -COOH, ou d'un dérivé d'un tel groupe, dans les composés de l'invention.

L'invention vise, selon un autre aspect, un procédé de séparation et de production d'oxygène, à partir d'un mélange gazeux le contenant, comprenant la mise en oeuvre de tels complexes II, le cas échéant, sous forme de sels, comme définis ci-dessus, et/ou sous forme d'hydrates.

Le procédé de l'invention est caractérisé en ce qu'il comprend

- la mise en contact en solution aqueuse ou partiellement aqueuse du mélange de gaz avec un complexe métallique II tel que défini ci-dessus, dans des conditions permettant l'absorption de l'oxygène de ce mélange de gaz, ce qui conduit à la formation d'un dimère qui, dans sa forme neutre, répond à la formule $XO_2X$ dans laquelle X représente une molécule de complexe métallique,
- la désorption de l'oxygène fixé dans le dimère (réalisé dans un compartiment différent de celui utilisé pour l'étape d'absorption), et
- la récupération de l'oxygène désorbé.

Dans l'étape de mise en contact, le complexe métallique II est en solution aqueuse ou partiellement aqueuse, à une concentration de 0,1M à 1M à un pH de 6 à 12, de préférence de 7 à 9. Le complexe peut être formé in situ en ajoutant un dérivé d'amine et un sel métallique dans un milieu aqueux ou partiellement aqueux.

Par solution partiellement aqueuse, on entend un mélange d'eau et de solvant organique miscible à l'eau Comme exemples de solvant organique, on citera des solvants de type alcool tels que le méthanol et l'éthanol ou des solvants de type amide tels que le diméthyl formamide.

En variante le complexe métallique est en solution aqueuse ou partiellement aqueuse, cette solution étant immobilisée sur une membrane hydrophile perméable à l'oxygène. Cette membrane comprend un support formé d'un film polymère non perméable aux liquides. Des polymères utilisables comprennent les polysulfones, les polyamides, les polyesters, les polyoléfines, les polycarbonates, des polyhydrocarbures halogénés, des polyorganosilanes, des polyorganosiloxanes, des polyvinyls, et des polyimides.

L'une des faces de la membrane est en contact avec le mélange gazeux renfermant l'oxygène, la désorption de l'oxygène fixé par le complexe métallique étant effectuée sur l'autre face.

La désorption de l'oxygène fixé sur le complexant, est réalisée par exemple par le vide. Lorsque le complexe métallique est utilisé en solution, on abaisse la pression partielle de l'oxygène dans l'atmosphère environnant la solution, cette pression partielle est par exemple abaissée de 0,2 à 0,03 bar.

Lorsque le complexe métallique est en solution immobilisée sur une membrane, on abaisse la pression partielle et/ou on applique une différence de température de part et d'autre de la membrane.

La différence de pression partielle de part et d'autre de la membrane est par exemple de 0,1 bar.

La différence de température est de l'ordre de 10 °C.

Selon un autre mode de réalisation de l'étape de désorption, on oxyde par voie électrochimique, le dimère $XO_2X$ en appliquant un potentiel approprié entre deux électrodes d'une cellule électrochimique.

La fixation de l'oxygène par un complexe métallique en solution aqueuse peut se schématiser selon les équilibres suivants, où X représente le complexe métallique :

$X + 0_2 \rightarrow XO_2$

$XO_2 + X \rightarrow XO_2X$

La forme dimérique $XO_2X$, résultant de la complexation de $O_2$ par deux complexes métalliques est la forme prépondérante en milieu aqueux.

De tels dimères entrent également dans le cadre de l'invention.

Il sera fait référence dans la suite de ce texte aux figures 1 à 4 dans lesquelles :

- La figure 1 : représente la capacité d'absorption en pourcentage du ligand L7 complexé à l'ion $Co^{2+}$ en fonction du pH de la solution du complexe $(L7)Co^{2+}$ avant introduction de dioxygène. La quantité d'oxygène absorbé est mesurée par volumétrie.
- La figure 2 : représente la diminution de la capacite d'absorption de $O_2$ (ou dégradation) des complexants pentadentates linéaires du cobalt en fonction du temps.
- La figure 3 : représente la diminution de la capacité d'absorption de $O_2$ (ou dégradation) des complexants tetradentés linéaires ou pentadentés branchés du cobalt en fonction du temps.
- La figure 4 : illustre l'évolution de l'intensité de la bande de transfert du dioxygène :
  . la courbe A représente le spectre UV-Visible du ligand L6 seul,
  . la courbe B représente le spectre UV-Visible du complexe $(L6)CoO_2Co(L6)$ sous atmosphère de dioxygène,
  . la courbe C représente le spectre UV-Visible de la solution précédente (courbe B) après barbotage d'azote.

Les exemples qui suivent illustrent la synthèse de dérivés polyazotés ou ligands de l'invention selon l'un des modes de condensation décrits ci-dessus ainsi que les conditions d'utilisation des complexes métalliques de l'invention pour l'absorption du dioxygène suivie de la description du dioxygène fixé. Il va de soi que ces exemples ne revêtent aucun caractère limitatif.

1) Les exemples 1a et 1b illustrent le cas particulier d'obtention d'un dérivé de l'invention par simple mise en oeuvre de la méthode de condensation ($\beta$) suivie de l'hydrolyse ($\gamma$).

- Exemple 1a : SYNTHESE DU LIGAND DE FORMULE L1

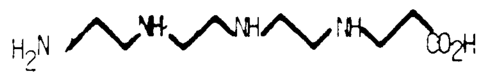

**1ère étape**

Préparation du nitrile selon le mode de condensation ($\beta$)

A 10 ml d'acrylonitrile (0,152 mole) (JANSSEN CHIMICA, réf. 14963.25), on ajoute 60 g de triéthylènetétramine (0,41 mole) (JANSSEN CHIMICA, réf.15792.78) sur une durée de 30 minutes environ. La solution bleue formée est alors agitée à température ambiante pendant 24 heures. L'excès de triéthylènetétramine est chassé par distillation sous pression (= 0,1 mmHg) et le liquide résiduel est utilisé sans autre purification.

**2ème étape**

Hydrolyse du nitrile en acide selon la réaction ($\gamma$)

Le nitrile obtenu est porté à reflux pendant 10 heures 30 minutes dans 100 ml d'eau et 50 ml d'acide sulfurique concentré (d = 1,83). Après refroidissement et concentration, l'acide est précipité par ajout d'acétone et refroidissement. Le précipité est filtré et lavé à l'acétone. Le solide obtenu après filtration est recristallisé dans un mélange eau/acide sulfurique/acétone. Après filtration, rinçage à l'acétone puis séchage, 15 g d'acide sont recueillis sous forme de poudre blanche.

RMN$^1$H ($D_2O$) : $\delta$2,88(t,2H), 3,41(t,2H), 3,51(m,12H).

Suivant un mode opératoire identique au précédent, le dérivé de formule L'1

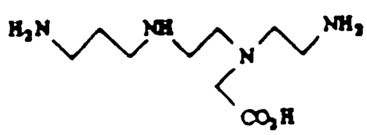

est obtenu par condensation du dérivé de formule

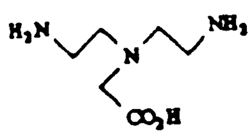

avec l'acrylonitrile, suivie d'une étape d'hydrolyse.

2) Les exemples 2a à 2j illustrent la synthèse de dérivés de l'invention selon la condensation b) par réaction d'un aldéhyde avec une amine primaire du type $R'_2-NH_2$ comportant un groupe nitrile.

Ces exemples de synthèse 2a à 2j présentent une première étape de synthèse commune du nitrile de formule $N_1$

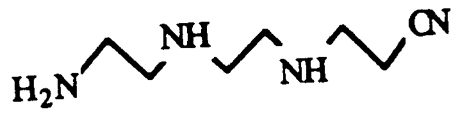

La préparation du nitrile $N_1$ est réalisée selon le mode de condensation ($\beta$) de la manière suivante : à 0,304 mole d'acrylonitrile on ajoute sur une durée d'une heure environ 0,923 mole de diéthylènetriamine (JANSSEN CHIMICA, réf.11431.82). Le milieu réactionnel est ensuite laissé sous agitation à température ambiante pendant 48 heures. L'excés de diéthylènetriamine est éliminé par distillation sous pression réduite (= 0,1 mmHg). Le liquide jaune résiduel correspondant au produit désiré est alors utilisé sans autre purification.

On recueille ainsi 39 g d'un liquide épais incolore dont le point d'ébullition est de 130-140 °C sous 0,05 mmHg.

Rdt : 85 %.

- Exemple 2a : SYNTHESE DU DERIVE DE FORMULE L2

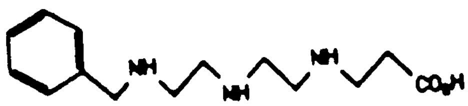

**1ère étape**

Préparation du nitrile N1

**2ème étape**

Condensation du nitrile N1 sur le benzaldéhyde et réduction de l'imine

On porte à reflux pendant 30 minutes, un mélange de 7,8 g du nitrile N1 (0,05 mole) avec 5,1 ml de benzaldéhyde (0,05 mole) (JANSSEN CHIMICA, réf.10522.46) et 40 ml d'éthanol absolu. Après évaporation de l'alcool, le produit est hydrogéné en présence de 1 g de Pd/C à 10 % dans 60 ml d'éthanol absolu dégazé pendant 4 jours. Le catalyseur est éliminé par filtration et le filtrat est évaporé à sec. L'huile résiduelle orange est utilisée sans autre purification.

**3ème étape**

Hydrolyse du nitrile obtenu à l'étape précédente en acide

L'huile obtenue est portée à reflux pendant 2 heures 30 minutes dans 120 ml d'eau et 25 ml d'acide sulfurique concentré (d = 1,83). L'acide est précipité par ajout d'acétone et refroidissement pendant 1 jour à -15 °C. Le précipité est filtré et recristallisé dans un mélange eau/acide sulfurique/acétone. Après filtration, lavage par l'acétone puis séchage, 2,74 g d'acide sont recueillis sous forme de poudre blanche.

RMN$^1$H ($D_2O$) : δ2,87(t,2H), 3,40(t,2H), 3,53(t,8H), 4,42(s,2H), 7,5(s,5H)

L'analyse élémentaire indique la présence de deux molécules d'acide sulfurique et d'une molécule d'eau par molécule de dérivé 2 dans le solide obtenu.

- Exemple 2b : SYNTHESE DU DERIVE DE FORMULE L3

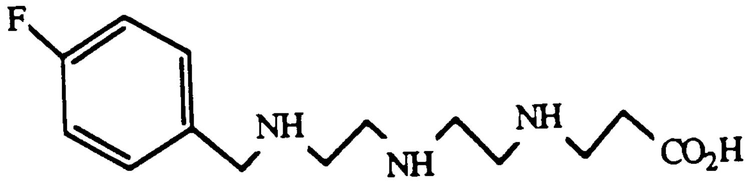

**1ère étape**

Préparation du nitrile N1

**2ème étape**

Condensation du nitrile N1 obtenu sur le fluoro-4benzaldéhyde et réduction de l'imine.

On porte à reflux pendant 25 minutes, 7,3 g du nitrile N1 (46,8 mmole) et 5,8 g de fluoro-4benzaldéhyde (46,8 mmole) dans 20 ml d'éthanol absolu. Après évaporation de l'alcool, le produit est hydrogèné pendant quatre jours en présence de 1,5 g de Pd/C à 10 % dans 40 ml d'alcool éthylique absolu dégazé. Le catalyseur est éliminé par filtration et le filtrat est évaporé à sec. Le produit est précipité sous forme de chlorhydrate par ajout de 80 ml d'acide chlorhydrique 6N. Le solide blanc formé est récupéré par filtration puis recristallisé dans l'éthanol à 90 %. Après filtration puis sèchage, 11 g de produit sont recueillis sous forme de poudre blanche.

RMN$^1$H ($D_2O$) : $\delta$ 3,03 (t, 2H, *CH*$_2$-CN); 3,52 (m, 10H, NH-*CH*$_2$-*CH*$_2$-NH, *CH*$_2$-$CH_2$CN); 4,31 (s,2H,*CH*$_2$-$C_6H_4F$); 7,21 (t, 2H, *H*$_2$-Phenyl); 7,49 (m,2H, *H*$_3$-Phenyl)

**3ème étape**

Hydrolyse du nitrile obtenu à l'étape précédente en acide

On porte à reflux durant 1 h 7,8 g du nitrile obtenu à la 2ème étape (20,88 mmole) dans 50 ml d'eau et 30 ml d'acide sulfurique concentré (d = 1,83). L'acide est précipité par ajout d'éthanol et refroidissement à -15 °C pendant deux jours. Après filtration, lavage par l'éthanol puis séchage, 8 g d'acide sont recueillis sous forme de poudre blanche.

RMN$^1$H ($D_2O$):$\delta$ 2,87 (t, 2H, -*CH*$_2$-$CO_2H$); 3,39 (t, 2H, −*CH*$_2$-$CH_2$-$CO_2H$); 3,53 (s, 8H, NH-*CH*$_2$ *CH*$_2$-NH);4,3 (s, 2H, *CH*$_2$-$C_6H_4F$); 7,2 (t, 2H, *H*$_2$-Phenyl); 7,51 (m, 2H, *H*$_3$-Phenyl)

- Exemple 2c : SYNTHESE DU DERIVE DE FORMULE L4

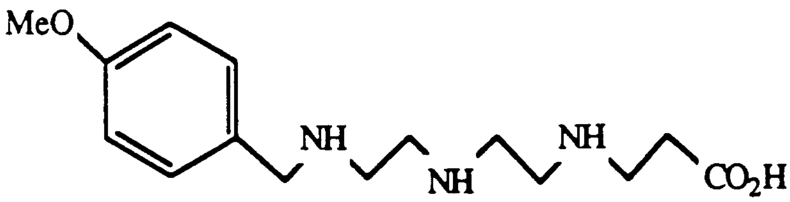

**1ère étape**

Préparation du nitrile N1

**2ème étape**

Condensation du nitrile N1 obtenu sur le méthoxy-4-benzaldéhyde et réduction de l'imine.

On porte à reflux pendant 20 minutes 8 g du nitrile N1 (51 mmole) et 6,2 g de méthoxy-4-benzaldéhyde (51 mmole) dans 20 ml d'éthanol absolu. Après évaporation de l'alcool, le produit est hydrogèné pendant cinq jours en présence de 1,5 g de Pd/C à 10 % dans 40 ml d'alcool éthylique absolu dégazé. Le catalyseur est éliminé par filtration et le filtrat est évaporé à sec. Le produit est précipité sous forme de chlorhydrate par ajout de 80 ml d'acide chlorhydrique 6N puis refroidissement à -15 °C pendant quatre jours. Le solide blanc formé est récupéré par filtration et lavé à l'éthanol absolu. Après sèchage, 11 g de produit sont recueillis sous forme de poudre blanche.

RMN$^1$H ($D_2O$):$\delta$ 3,02 (t, 2H, *CH₂*-CN); 3,47 (m, 10H, NH-*CH₂CH₂*NH,*CH₂*--CH₂-CN); 3,85 (s,3H, *CH₃*-OC₆H₄); 4,26 (s, 2H, *CH₂*-C₆H₄OMe); 7,06 (d, 2H, *H₂*--Phenyl); 7,43 (d, 2H, *H₃*-Phenyl)

**3ème étape**

Hydrolyse du nitrile obtenu à l'étape précédente en acide

On porte à reflux durant 1 h 3,43 g du nitrile obtenu à la 2ème étape (8,9 mmole) dans 30 ml d'eau et 15 ml d'acide sulfurique concentré (d = 1,83). L'acide est précipité par ajout d'éthanol et refroidissement à -15 °C pendant une nuit. Après filtration, rinçage par l'éthanol puis séchage, 2,9 g d'acide sont recueillis sous forme de poudre grise.

RMN$^1$H ($D_2O$):d 2,87 (t, 2H, -*CH₂*-CO₂H); 3,40 (t, 2H, -*CH₂*--CH₂-CO₂H); 3,52 (m, 8H, NH-*CH₂*--*CH₂*--NH); 3,84 (s,3H, *CH₃*-OC₆H₄); 4,26 (s,2H, *CH₂*--C₆H₄OMe); 7,07 (s,2H, *H₂*--Phenyl); 7,46(d, 2H, *H₃*-Phenyl)

- Exemple 2d : SYNTHESE DU DERIVE DE FORMULE L5

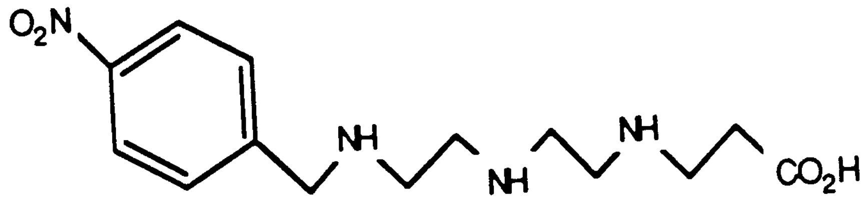

**1ère étape**

Préparation du nitrile N1

**2ème étape**

Condensation du nitrile N1 obtenu sur le nitro-4benzaldéhyde et réduction de l'imine.

On porte à reflux durant 30 minutes 8 g du nitrile N1 (51 mmole) et 6,2 ml de nitro-4benzaldéhyde (51 mmole) dans 20 ml d'éthanol absolu. Après evaporation de l'alcool, le produit est hydrogèné pendant cinq jours en présence de 1,5 g de Pd/C à 10 % dans 40 ml d'alcool éthylique absolu dégazé. Le catalyseur est éliminé par filtration et le filtrat est évaporé à sec. Le produit est précipité sous forme de chlorhydrate par ajout de 80 ml d'acide chlorhydrique 6N puis refroidissement à -15 °C pendant quatre jours. Le solide blanc formé est récupéré par filtration et lavé à l'éthanol absolu. Après sèchage, 9,5 g de produit sont recueillis sous forme de poudre beige.

**3ème étape**

Hydrolyse du nitrile obtenu à l'étape précédente en acide

On porte à reflux durant 1 h 30 9,5 g du nitrile obtenu à la 2ème étape (8,9 mmole) dans 30 ml d'eau et 20 ml d'acide sulfurique concentré (d = 1,83). L'acide est précipité par ajout d'éthanol et refroidissement à -15 °C pendant une nuit. Après filtration, rinçage par l'éthanol puis séchage, l'acide est recueilli sous forme de poudre grise. Après recristallisation dans un mélange éthanol/eau/acide sulfurique puis séchage, 4,7 g d'acide sont recueillis sous forme de poudre blanche.

RMN$^1$H ($D_2O$): $\delta$ 2,87 (t, 2H, $CH_2$-$CO_2H$); 3,40 (t, 2H, $CH_2$-$CH_2$-$CO_2H$); 3,53 (t, 8H, NH-$CH_2$-$CH_2$-NH-$CH_2$-$CH_2$-NH); 4,42 (s, 2H, $C_4H_4N$-$CH_2$); 7,42 (d, 2H, $H_5$ - Ph,$H_3$- Ph); 7,61 (d, 2H, $H_2$-Ph,$H_5$- Ph);

- Exemple 2e : SYNTHESE DU DERIVE DE FORMULE L6

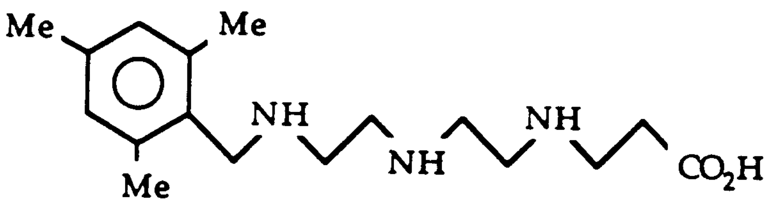

**1ère étape**

Préparation du nitrile N1

**2ème étape**

Condensation du nitrile N1 obtenu sur le mésitaldéhyde et réduction de l'imine.

On porte à reflux durant 30 minutes 7,8 g du nitrile N1 (0,05 mole) et 7,4 ml de mésitaldéhyde (0,05 mmole) dans 40 ml d'éthanol absolu. Après évaporation de l'alcool, le produit est hydrogèné en présence de 1 g de Pd/C à 10 % dans 60 ml d'éthanol absolu dégazé pendant 4 jours. Le catalyseur est éliminé par filtration et le filtrat est évaporé à sec. L'huile résiduelle orange est utilisée sans autre purification.

**3ème étape**

Hydrolyse du nitrile obtenu à l'étape précédente en acide

L'huile obtenue à la 2ème étape est portée à reflux pendant 2 h 30 dans 50 ml d'eau et 25 ml d'acide sulfurique concentré (d = 1,83). L'acide est précipité par ajout d'acétone et refroidissement pendant 1 jour à -15 °C. Le précipité est filtré et recristallisé dans un mélange acide sulfurique dilué à 5%/éthanol. Après filtration, lavage par l'acétone puis séchage, 3,8 g d'acide sont recueillis sous forme de poudre blanche.

RMN$^1$H ($D_2O$): $\delta$ 2,27 (s, 3H, $CH_3$-Ph) 2,38 (s, 6H, $CH_3$-Ph) 2,88 (t, 2H, $CH_2$-$CO_2H$) 3,42 (t, 2H, $CH_2$-$CH_2CO_2H$) 3,54-3,65 (m, 8H, NH-$CH_2$- $CH_2$ - NH) 4,40 (s, 2H, $CH_2$ - Ph) 7,04 (s, 2H, H - Ph)

- Exemple 2f : SYNTHESE DU DERIVE DE FORMULE L7

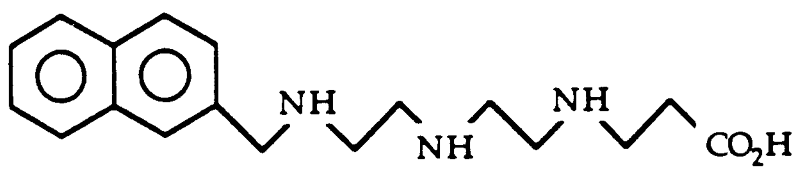

**1ère étape**

Préparation du nitrile N1

**2ème étape**

Condensation du nitrile N1 obtenu sur le naphtal-2aldéhyde et réduction de l'imine en amine.

On porte à reflux durant 30 minutes 7,8 g du nitrile N1 (0,05 mole) et 7,8 ml de naphtal-2aldéhyde (0,05 mole) dans 40 ml d'éthanol absolu. Après évaporation de l'alcool, le produit est hydrogèné en présence de 1 g de Pd/C à 10 % dans 60 ml d'éthanol absolu dégazé pendant 4 jours. Le catalyseur est éliminé par filtration et le filtrat est évaporé à sec. L'huile résiduelle orange est utilisée sans autre purification.

**3ème étape**

Hydrolyse du nitrile obtenu à l'étape précédente en acide

L'huile obtenue à la 2ème étape est portée à reflux pendant 1 h 30 dans 60 ml d'eau et 25 ml d'acide sulfurique concentré (d = 1,83). L'acide est précipité par ajout d'acétone et refroidissement pendant 1 jour à -15 °C. Le précipité est filtré et recristallisé dans un mélange eau/acide sulfurique/éthanol. Après filtration, lavage par l'acétone puis séchage, 4,2 g d'acide sont recueillis sous forme de poudre blanche.

RMN[1]H ($D_2O$): δ 2,86 (t, 2H, *CH*$_2$-$CO_2H$) 3,375 (t, 2H, *CH*$_2$-$CH_2$-$CO_2H$) 3,55 (m, 8H, NH-*CH*$_2$-*CH*$_2$-NH) 4,5 (s, 2H, *CH*$_2$ - napht) 7,61 (m, 3H, H - napht) 8,02 (m, 4H, H - napht)

- Exemple 2g : SYNTHESE DU DERIVE DE FORMULE L8

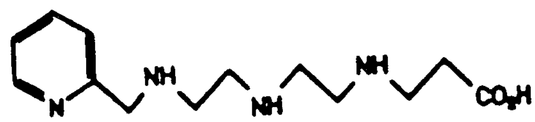

**1ère étape**

Préparation du nitrile N1

**2ème étape**

Condensation du nitrile N1 obtenu sur le pyridine-2carboxaldéhyde et réduction de l'imine.

On porte à reflux durant 30 minutes 8,2 g du nitrile N1 (52,56 mmole) et 5 ml de pyridine-2carboxaldéhyde (JANSSEN CHIMICA, réf.13182.87) dans 40 ml d'éthanol absolu. Après évaporation de l'alcool, le produit est hydrogèné en présence de 1 g de Pd/C à 10 % dans 40 ml d'alcool éthylique absolu dégazé pendant 4 jours. Le catalyseur est éliminé par filtration et le filtrat est évaporé à sec. L'huile résiduelle orange est utilisée sans autre purification.

**3ème étape**

Hydrolyse du nitrile obtenu à l'étape précédente en acide

L'huile obtenue à la 2ème étape est portée à reflux pendant 2 h 30 dans 200 ml d'eau et 50 ml d'acide sulfurique concentré (d = 1,83). L'acide est précipité par ajout d'acétone et refroidissement pendant 2 jours à -15 °C. Le précipité beige est filtré et recristallisé dans un mélange eau/acide sulfurique/acétone. Après filtration, lavage par l'acétone puis séchage, 10 g d'acide sont recueillis sous forme de poudre beige.

RMN $^1$H($D_2O$ : δ 2,89(t, 2H) ; 3,42(t, 2H); 3,6(m, 8H) ; 4,63(s, 2H) ; 7,87(t, 1H) ; 7,96(d, 1H) ; 8,37(t, 1H) ; 8,77(d, 1H).

- Exemple 2h : SYNTHESE DU DERIVE DE FORMULE L9

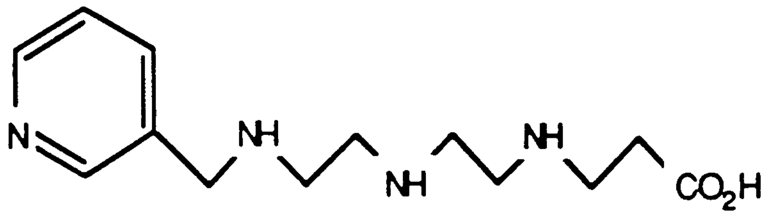

**1ère étape**

Préparation du nitrile N1

**2ème étape**

Condensation du nitrile N1 obtenu sur le pyridine-3carboxaldéhyde et réduction de l'imine en amine.

On porte à reflux durant 30 minutes 7,8 g du nitrile N1 (0,05 mole) dans 4,7 ml de pyridine-3carboxaldéhyde (0,05 mole) et 40 ml d'éthanol absolu. Après évaporation de l'alcool, le produit est hydrogèné en présence de 1 g de Pd/C à 10 % dans 40 ml d'alcool éthylique absolu dégazé pendant 4 jours. Le catalyseur est éliminé par filtration et le filtrat est évaporé à sec. L'huile résiduelle orange est utilisée sans autre purification.

**3ème étape**

Hydrolyse du nitrile obtenu à l'étape précédente en acide

L'huile obtenue à la 2ème étape est portée à reflux pendant 2 h 30 dans 120 ml d'eau et 25 ml d'acide sulfurique concentré (d = 1,83). L'acide est précipité par ajout d'acétone et refroidissement pendant 1 jour à -15 °C. Le précipité beige est filtré et recristallisé dans un mélange acide sulfurique dilué à 5%/éthanol. Après filtration, lavage par l'acétone puis séchage, 2,5 g d'acide sont recueillis sous forme de poudre blanche.

RMN$^1$H ($D_2O$): δ 2,87 (t, 2H, $CH_2$- $CO_2H$); 3,39 (t, 2H, $CH_2$ -$CH_2$-$CO_2H$): 3,56-3,67 (m, 8H, NH-$CH_2$-$CH_2$- NH-$CH_2$-$CH_2$- NH); 4,61 (s, 2H, $C_4H_4N$-$CH_2$); 8,17 (t, 1H, $H_5$ - Py); 8,81 (d, 1H, $H_4$ - Py); 8,88 (d, 1H, $H_5$ - Py); 9,05 (s, 1H, $H_2$- Py)

- Exemple 2i : SYNTHESE DU DERIVE DE FORMULE L10

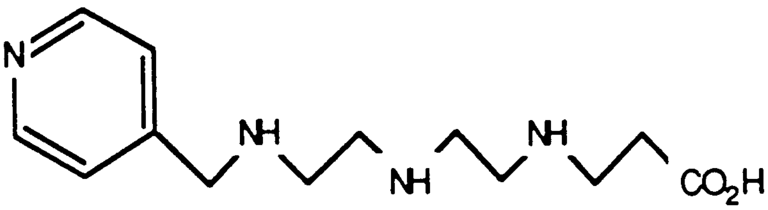

**1ère étape**

Préparation du nitrile N1

**2éme étape**

Condensation du nitrile N1 obtenu sur le pyridine-4carboxaldéhyde et réduction de l'imine en amine.

On porte à reflux durant 30 minutes 7,8 g du nitrile N1 (0,05 mole) dans 4,77 ml de pyridine-4carboxaldéhyde (0,05 mole) et 40 ml d'éthanol absolu. Après évaporation de l'alcool, le produit est hydrogéné en présence de 1 g de Pd/C à 10 % dans 60 ml d'éthanol absolu dégazé pendant 4 jours. Le catalyseur est éliminé par filtration et le filtrat est évaporé à sec. L'huile résiduelle orange est utilisée sans autre purification.

**3ème étape**

Hydrolyse du nitrile obtenu à l'étape précédente en acide

L'huile obtenue à la 2ème étape est portée à reflux pendant 1 h 30 dans 120 ml d'eau et 25 ml d'acide sulfurique concentré (d = 1,83). L'acide est précipité par ajout d'acétone et refroidissement pendant 1 jour à -15 °C. Le précipité est filtré et recristallisé dans un mélange eau/acide sulfurique/éthanol. Après filtration, lavage par l'acétone puis séchage, 2,3 g d'acide sont recueillis sous forme de poudre blanche.

RMN$^1$H ($D_2O$): $\delta$ 2,88 (t, 2H, $CH_2$- $CO_2H$) ; 3,41 (t, 2H, $CH_2$ -$CH_2$-$CO_2H$) ; 3,55-3,70 (m, 8H, NH-$CH_2$-$CH_2$- NH-$CH_2$-$CH_2$- NH); 4,67 (s, 2H, $C_4H_4N$-$CH_2$); 8,23 (d, 1H, $H_3$- Py); 8,23 (d, 1H, $H_5$- Py); 8,89 (d, 1H, $H_2$ - Py); 8,89 (d, 1H, $H_5$- Py)

- Exemple 2j : SYNTHESE DU DERIVE DE FORMULE L11

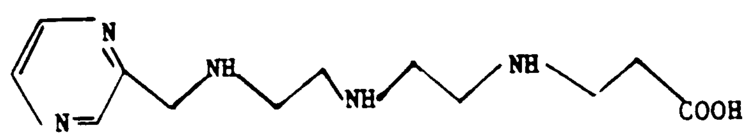

**1ère étape**

Préparation du nitrile N1

**2ème étape**

Condensation du nitrile N1 sur le pyrimidine-2carboxaldéhyde et réduction de l'imine

On porte à reflux pendant 20 à 30 minutes 0,05 mole du nitrile N1 avec 0,05 mole de pyrimidine-2carboxaldéhyde dans 50 ml d'éthanol absolu. Après évaporation de l'alcool, le produit est hydrogéné

pendant 3 à 5 jours en présence de 2 g de Pd/C à 10 % dans 50 ml d'éthanol absolu dégazé. Le catalyseur est éliminé par filtration et le filtrat est évaporé à sec.

L'huile résiduelle obtenue correspondant au produit désiré est utilisée sans autre purification.

**3ème étape**

Hydrolyse du nitrile obtenu à l'étape précédente en acide

L'huile résiduelle obtenue précédemment est portée à reflux pendant 1 à 2 heures dans 60 à 120 ml d'eau et 30 à 60 ml d'acide sulfurique concentré (d = 1,83). L'acide est précipité par ajout d'éthanol et refroidissement pendant 1 à 4 jours. Le précipité est filtré et recristallisé dans un mélange eau/acide sulfurique/alcool. Après filtration, lavage par l'éthanol absolu puis séchage, l'acide est recueilli sous forme de poudre blanche.

En partant des aldéhydes et des amines primaires substituées par un groupe carboxyle, mentionnés dans le tableau I, les produits suivants ont été obtenus suivant le mode de condensation b).

TABLEAU I

| | | | |
|---|---|---|---|
| amine primaire | 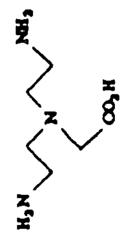 | 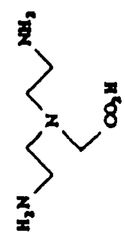 | 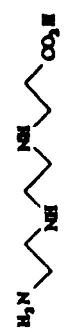 |
| aldéhyde | 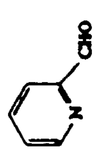 | 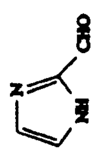 | 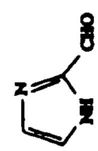 |
| Dérivé obtenu | 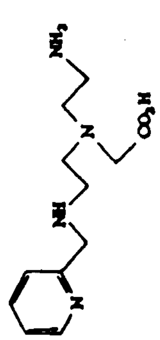 | 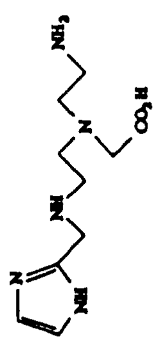 | 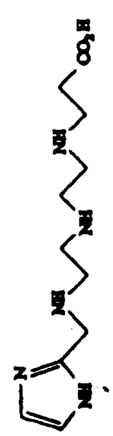 |

3) <u>Les exemples 3a à 3c</u> qui suivent décrivent des synthèses réalisées selon un protocole identique à celui des exemples du paragraphe 2) précédent, et qui comprennent une première étape commune de synthèse d'un nitrile de formule N2.

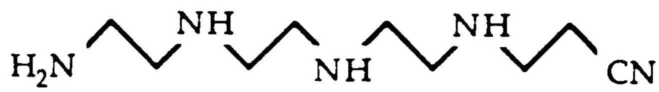

La préparation du nitrile $N_2$ est réalisée selon le mode de condensation ($\beta$) de la manière suivante :

à 0,304 mole d'acrylonitrile, on ajoute sur une durée d'une heure environ 0,923 mole de triéthylènetétramine. Le milieu réactionnel est ensuite maintenu sous agitation à température ambiante pendant 48 heures. L'excès de triéthylènetétramine est éliminé par distillation sous pression réduite (=0,1 mmHg). Le liquide jaune résiduel correspondant au produit désiré est alors utilisé sans autre purification.

- Exemple 3a : SYNTHESE DU DERIVE DE FORMULE L12

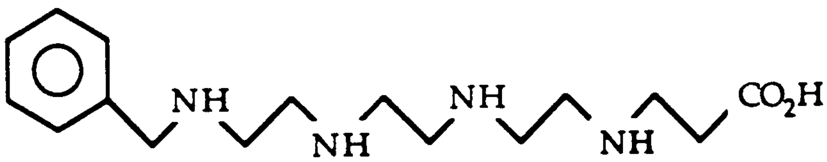

**1ère étape**

Préparation du nitrile N2

**2ème étape**

Condensation du nitrile N2 sur le benzaldéhyde et réduction de l'imine

On porte à reflux durant 25 minutes 9,95 g du nitrile N2 (50 mmole) et 5,1 ml de benzaldéhyde (50,2 mmole) dans 40 ml d'éthanol absolu. Après évaporation de l'alcool, le produit est hydrogèné pendant 4 jours en présence de 1,5 g de Pd/C à 10 % dans 40 ml d'alcool éthylique absolu dégazé. Le catalyseur est éliminé par filtration et le filtrat est évaporé à sec. Le produit est précipité sous forme de chlorhydrate par ajout de 80 ml d'acide chlorydrique 6N. Le solide blanc formé est récupéré par filtration puis recristallisé dans l'éthanol à 90 %. Après filtration puis séchage, 13,5 g de produit sont recueillis sous forme de poudre blanche.

**3ème étape**

Hydrolyse du nitrile obtenu à l'étape précédente en acide

On porte à reflux durant 1 heure 8 g du nitrile obtenu à la 2ème étape, dans 60 ml d'eau et 30 ml d'acide sulfurique concentré (d = 1,83). L'acide est précipité par ajout d'éthanol et refroidissement à -15 °C pendant 2 jours. Après filtration, lavage par l'éthanol puis séchage, 8 g d'acide sont recueillis sous forme de poudre blanche.

RMN$^1$H ($D_2O$):$\delta$ 2,89 (t, 2H, -*$CH_2$* - $CO_2H$) 3,43 (t, 2H, -*$CH_2$* -$CH_2$-$CO_2H$) 3,57 (m, 12H, NH - $CH_2CH_2$-NH) 4,3 (s, 2H, $CH_2$- Ph) 7,5 (s, 5H, H - Ph)

- Exemple 3b : SYNTHESE DU DERIVE DE FORMULE L13

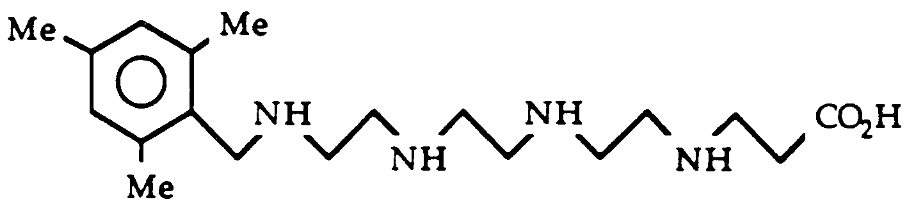

**1ère étape**

Préparation du nitrile N2

**2ème étape**

Condensation du nitrile N2 sur le mésitaldéhyde et réduction de l'imine en amine.

On porte à reflux durant 20 minutes 10 g du nitrile N2 (50,3 mmole) et 7,5 g de mésitaldéhyde (50,6 mmole) dans 40 ml d'éthanol absolu. Après évaporation de l'alcool, le produit est hydrogèné pendant cinq jours en présence de 1,5 g de Pd/C à 10 % dans 40 ml d'alcool éthylique absolu dégazé. Le catalyseur est éliminé par filtration et le filtrat est évaporé à sec. Le produit est précipité sous forme de chlorhydrate par ajout de 100 ml d'acide chlorhydrique 6N puis refroidissement à -15 °C pendant quatre jours. Le solide blanc formé est récupéré par filtration et lavé à l'éthanol absolu. Après séchage, 14 g de produit sont recueillis sous forme de poudre blanche.

**3ème étape**

Hydrolyse du nitrile obtenu à l'étape précédente

On porte à reflux pendant 1 heure 14 g de nitrile obtenu à l'étape précédente, dans 100 ml d'eau et 50 ml d'acide sulfurique concentré (d = 1,83). L'acide est précipité par ajout d'éthanol et refroidissement à -15 °C pendant une nuit. Après filtration, rinçage par l'éthanol puis séchage 5,6 g d'acide sont recueillis sous forme de poudre grise.

RMN$^1$H ($D_2O$) : $\delta$ 2,26 (s, 3H, $CH_3$ - Ph) 2,38 (s, 6H, $CH_3$ - Ph) 2,88 (t, 2H,*$CH_2$* -$CO_2H$) 3,41 (t, 2H, -*$CH_2$* -$CH_2$-$CO_2H$) 3,59 (m, 12H, NH-*$CH_2$*--*$CH_2$* - NH) 4,40 (s, 2H, $CH_2$ - Ph) 7,04 (s, 2H, H - Ph)

- Exemple 3c : SYNTHESE DU DERIVE DE FORMULE L14

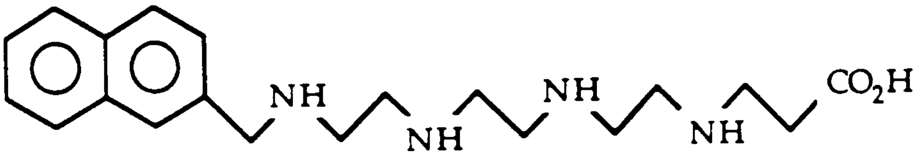

**1ère étape**

Préparation du nitrile N2

**2ème étape**

Condendation du nitrile N2 sur le naphtal-2aldéhyde et réduction de l'imine en amine

On porte à reflux durant 30 minutes, 10 g du nitrile N2 (50,3 mmole) et 7,9 g de naphtal-2aldéhyde (50,6 mmole) dans 40 ml d'éthanol absolu. Après évaporation de l'alcool, le produit est hydrogèné pendant 5 jours en présence de 1,5 g de Pd/C à 10 % dans 60 ml d'alcool éthylique absolu dégazé. Le catalyseur est éliminé par filtration et le filtrat est évaporé à sec. Le produit est précipité sous forme de chlorohydrate par ajout de 100 ml d'acide chlorhydrique 6N puis refroidissement à -15 ° C pendant 4 jours. Le solide blanc formé est récupéré par filtration et lavé à l'éthanol absolu. Après séchage, 8,6 g de produit sont recueillis sous forme de poudre beige.

**3ème etape**

Hydrolyse du nitrile obtenu à l'étape suivante en acide

On porte à reflux durant 1 heure 30, 8,6g du nitrile obtenu à la 2ème étape, dans 60 ml d'eau et 30 ml d'acide sulfurique concentré (d = 1,83). L'acide est précipité par ajout d'éthanol et refroidissement à -15 ° C pendant une nuit. Après filtration, rinçage par l'éthanol puis séchage, l'acide est recueilli sous forme de poudre grise. Après recristallisation dans un mélange éthanol/acide sulfurique puis séchage, 5,2 g d'acide sont recueillis sous forme de poudre blanche.

RMN$^1$H ($D_2O$): $\delta$ 2,88 (t, 2H, *$CH_2$*- $CO_2H$) 3,42 (t, 2H, *$CH_2$* -$CH_2$-$CO_2H$) 3,59 (m, 12H, NH-*$CH_2$* - CH-NH) 4,51 (s, 2H, $CH_2$ - napht) 7,64 (m, 3H, H - napht) 8,03 (m, 4H, H - napht)

4) Les exemples 4a.1, 4a.2 et 4b qui suivent comprennent une première étape commune de synthèse du dérivé aminé de formule B1 selon le mode de condensation b),

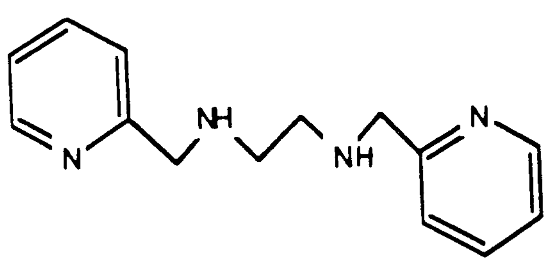

suivie d'une étape de condensation avec un halogénure d'acide selon le mode de condensation ($\alpha$).

Cette première étape de préparation du dérivé bis-1,6(pyridyl-2)diaza-2,5 hexane de formule B1 est réalisée de la manière suivante :

On porte à reflux pendant 20 minutes, 3,5 ml d'éthylènediamine (52,4 mmole) (JANSSEN CHIMICA, réf.11842.08) et 10 ml de pyridine-2 carboxaldéhyde (105,2 mmole) dans 40 ml d'éthanol absolu. Après évaporation de l'alcool, le produit est hydrogèné pendant cinq jours en présence de 1,5 g de Pd/C à 10 % dans 60 ml d'éthanol absolu. Le catalyseur est éliminé par filtration et le filtrat est évaporé à sec. L'huile résiduelle est utilisée sans autre purification.

RMN$^1$H($D_2O$) à pH = 1: $\delta$ 3,63 (s, 4H, NH-*$CH_2$*- *$CH_2$*- NH); 4,66 (s, 4H, $C_4H_4N$-*$CH_2$*);7,90 (t, 2H, *$H_5$* -

Py); 7,97 (d, 2H, $H_3$- Py); 8,41 (t, 2H, $H_4$- Py); 8,77 (d, 2H, $H_6$- Py);

- Exemple 4a : SYNTHESE DU DERIVE DE FORMULE L15

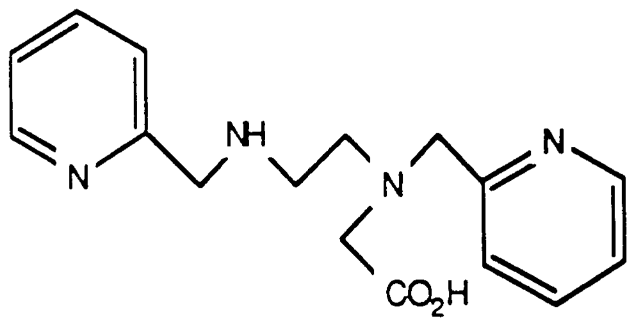

. Exemple 4a.1 :

**1ère étape**

Synthèse du bis-1,6(pyridyl-2)diaza-2,5 hexane (B1)

**2ème étape**

Condensation de l'acrylonitrile sur l'amine B1 obtenue

L'amine B1 obtenue est portée à reflux dans 20 ml d'acétonitrile. A cette solution est ensuite ajoutée goutte à goutte sur 1 heure environ une solution de 1 ml d'acrylonitrile dans 10 ml d'acétonitrile. L'addition terminée, le chauffage à reflux est prolongé pendant 30 minutes puis le solvant est évaporé sous vide. L'huile résiduelle orange est utilisée sans autre purification.

**3ème étape**

Hydrolyse du nitrile obtenu à l'étape précédente en acide

L'huile obtenue est portée à reflux pendant 1 heure 30 minutes dans 20 ml d'eau et 15 ml d'acide sulfurique concentré ( d = 1,83). La solution est évaporée à sec puis neutralisée par la soude jusqu'à pH d'environ 10. la solution marron obtenue est alors lavée quatre fois par 150 ml de chloroforme. La phase aqueuse devenue orange est concentrée à 10 ml puis neutralisée par 30 ml d'acide chlorhydrique 6N. La suite des opérations suivantes est alors répétée quatre fois : concentration du filtrat, précipitation par ajout d'éthanol, filtration et récupération du filtrat. La totalité des sels minéraux présents dans la solution est ainsi éliminée. Le solide beige récupéré après par évaporation du solvant du dernier filtrat est recristallisé dans un mélange éthanol/acide chlorhydrique 6N. Après filtration puis séchage, 850 mg d'acide sont recueillis sous forme de fines aiguilles blanches.
RMN$^1$ H($D_2O$) : $\delta$ 2,9(t,2H,$CH_2$-$CO_2H$) ; 3,37(s,4H,$CH_2$- $C_5H_4N$) ; 3,42(t,2H,$CH_2$-$CH_2$-$CO_2H$) ; 3,58-3,72-(m,4H,NH- $CH_2$-$CH_2$-NH) ; 7,96(t,2H,$H_4$-Pyr) ; 8,08(d,2H,$H_3$-Pyr) ; 8,49(t,2H,$H_5$-Pyr) ; 8,82(d,2H,$H_6$-Pyr).

. Exemple 4a.2 :

**1ère étape**

Synthèse du 1,6-bis(2-pyridyl)-2,5-diazahexane (B1)

. Exemple 4a.2 :

**1ère étape**

Synthèse du 1,6-bis(2-pyridyl)-2,5-diazahexane (B1)

**2ème étape**

Condensation de l'acide bromoéthanoïque sur l'amine B1 obtenue:

A 12g d'amine B1 obtenue précédemment (50 mmole) portés à reflux dans 125ml d'éthanol et 25ml d'eau, est ajoutée goutte à goutte sur 1h30 environ une solution de 1,16g d'acide bromoéthanoïque (8,34 mmole) et 0,7g d'hydroxyde de lithium monohydraté (16,67 mmole) dans 35 ml d'eau. Le chauffage à reflux est prolongé pendant 9 heures après la fin de l'ajout. L'éthanol est évaporé sous vide. La solution aqueuse résiduelle orange est lavée sept fois par 50 ml de dichlorométhane. Le produit brut est recueilli par évaporation et est purifié après protonation par une solution à 5% d'acide chlorhydrique dans l'éthanol en effectuant une recristallisation dans un mélange éthanol/acide chlorhydrique dilué à 5%. On recueille ainsi 1,72g de poudre blanche correspondant au produit visé.

RMN$^1$H ($D_2O$) à pH = 9: δ 2,57 (t, 2H, NH-*CH*$_2$- $CH_2$- N- ); 2,70 (t, 2H, NH-$CH_2$-*CH*$_2$N-); 3,18 (s, 2H, *CH*$_2$- $CO_2H$); 3,72 (s, 2H, $C_4H_4N$-*CH*$_2$); 3,79 (s, 2H, $C_4H_4N$-*CH*$_2$'); 7,31 (t, 1H, *H*$_5$ - Py) ; 7,36 (t, 1H, *H*$_5$' - Py) ; 7,37 (d, 1H, *H*$_3$- Py); 7,52 (d, 1H, *H*$_3$'- Py); 7,79 (t, 1H, *H*$_4$- Py); 7,84 (t, 1H, *H*$_4$'- Py); 8,38 (d, 1H, *H*$_5$- Py); 8,43 (d, 1H, *H*$_5$'- Py);

- Exemple 4b : SYNTHESE DU DERIVE DE FORMULE L16

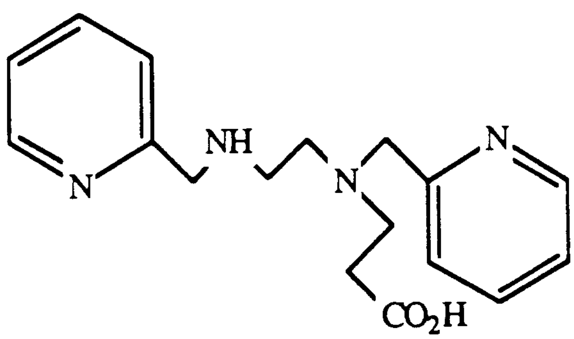

**1ère étape**

Synthèse du bis-1,6(pyridyl-2)diaza-2,5 hexane B1

**2ème étape**

Condensation de l'acide bromo-3propanoïque sur l'amine B1 obtenue:

A 12g d'amine B1 obtenue précédemment (50 mmole) portés à reflux dans 125ml d'éthanol et 25ml d'eau, est ajoutée goutte à goutte sur 2h environ une solution de 1,27g d'acide bromo-3propanoïque (8,3 mmole) et 0,7g d'hydroxyde de lithium monohydraté (16,67 mmole) dans 35 ml d'eau. Le chauffage à reflux est prolongé pendant 9 heures après la fin de l'ajout. L'éthanol est évaporé sous vide. La solution aqueuse résiduelle orange est lavée sept fois par 50 ml de dichlorométhane. Le produit brut est recueilli par

évaporation et est purifié après protonation par une solution à 5% d'acide chlorhydrique dans l'éthanol en effectuant une recristallisation dans un mélange éthanol/acide chlorhydrique dilué à 5%. On recueille ainsi 1,94g de poudre blanche correspondant au produit visé.

RMN $^1H$ ($D_2O$) à pH = 9 : δ 2,41 (t, 2H, NH-$CH_2$-$CH_2$- N-); 2,86 (t, 2H, NH-$CH_2$-$CH_2$- N- ); 2,88 (t, 2H,$CH_2$--$CH_2$-$CO_2H$); 3,18 (t, 2H, -$CH_2$-$CH_2$- $CO_2H$); 3,82 (s, 2H, $C_4H_4N$-$CH_2$); 4,17 (s, 2H, $C_4H_4N$-$CH_2$'); 7,40 (t, 1H, $H_5$ - Py); 7,41 (t, 1H, $H_5$'- Py); 7,43 (d, 1H, $H_3$- Py); 7,46 (d, 1H, $H_3$'- Py); 7,86 (t, 1H, $H_4$- Py); 7,88 (t, 1H, $H_4$'- Py); 8,49 (d, 1H, $H_5$- Py); 8,52 (d, 1H, $H_5$'- Py);

5) Les exemples 5 a et 5 b qui suivent décrivent des synthèses réalisées selon un protocole identique à celui des exemples du paragraphe 4) précédent, et qui comprennent une première étape commune de synthèse du dérivé aminé de formule B2.

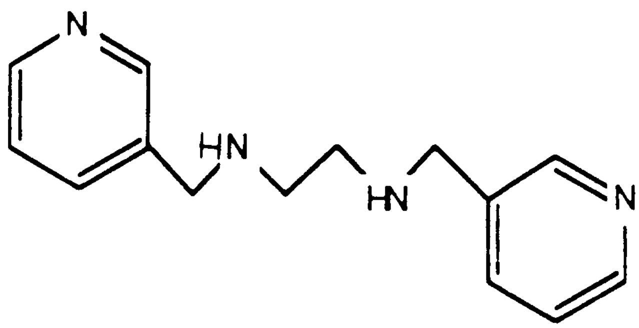

Cette première étape de préparation du derivé de formule B2 bis-1,6(pyridyl-3)diaza-2,5 hexane est réalisée de la manière suivante :

Un mélange de 3,5 ml d'éthylènediamine (52,4 mmole) et 10 ml de pyridine-3carboxaldéhyde (106 mmole) dans 40 ml d'éthanol absolu sont portés à reflux pendant 20 minutes. Après évaporation de l'alcool, le produit est hydrogèné pendant cinq jours en présence de 1,5g de Pd/C à 10% dans 60 ml d'éthanol absolu. Le catalyseur est éliminé par filtration et le filtrat est évaporé à sec. L'huile résiduelle est utilisée sans autre purification.

RMN $^1H$ ($D_2O$) à pH = 1 : δ 3,65 (s, 4H, NH-$CH_2$- $CH_2$- NH); 4,60 (s, 4H, $C_4H_4N$-$CH_2$);7,73 (t, 2H, $H_5$ - Py); 7,84 (d, 2H, $H_4$- Py); 8,57 (d, 2H, $H_5$- Py); 8,93 (s, 2H, $H_2$- Py) ;

- Exemple 5a : SYNTHESE DU DERIVE DE FORMULE L17

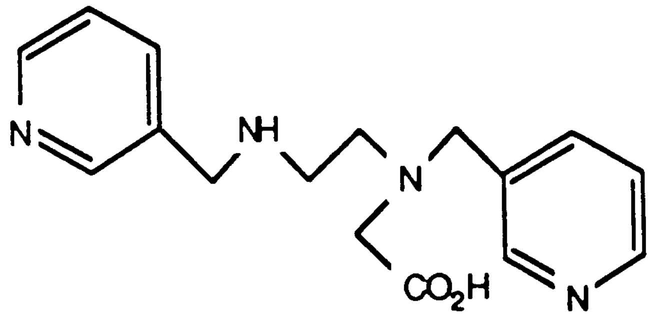

**1ère étape**

Synthèse du bis-1,6(pyridyl-3)diaza-2,5 hexane B2)

**2ème étape**

Condensation de l'acide bromoéthanoïque sur l'amine B2 obtenue:

A 12g d'amine B2 obtenue précédemment (50 mmole) portés à reflux dans 125ml d'éthanol et 25ml d'eau, est ajoutée goutte à goutte sur 1h30 environ une solution de 1,16g d'acide bromoéthanoïque (8,34 mmole) et 0,7g d'hydroxyde de lithium monohydraté (16,67 mmole) dans 35 ml d'eau. Le chauffage à reflux est prolongé pendant 10 heures après la fin de l'ajout. L'éthanol est évaporé sous vide. La solution aqueuse résiduelle orange est lavée sept fois par 50 ml de dichlorométhane. Le produit brut est recueilli par évaporation et est purifié après protonation par une solution à 5% d'acide chlorhydrique dans l'éthanol en effectuant une recristallisation dans un mélange éthanol/acide chlorhydrique dilué à 5%. On recueille ainsi 1,26g de poudre blanche correspondant au produit visé.

RMN$^1$H($D_2O$) à pH = 9: $\delta$ 2,59(s,2H,NH-$CH_2$-$CH_2$-N-); 2,72(t,2H,NH-$CH_2$-$CH_2$-N-);3,16(s,2H,$CH_2$-$CO_2$H); 3,68(s,2H,$C_5H_4$N-$CH_2$);3,70(s,2H,$C_5H_4$N-$CH_2$'); 7,35(t,1H,$H_5$-Py)-;7,39(t,1H,$H_5$'-Py); 7,81(t,1H,$H_4$-Py);7,84(t,1H,$H_4$'-Py); 8,41(d,1H,$H_6$-Py);8,45-(d,1H,$H_6$'-Py); 8,53(d,1H-$H_2$-Py);8,56(d,1H,$H_2$'-Py);

- Exemple 5b : SYNTHESE DU DERIVE DE FORMULE L18

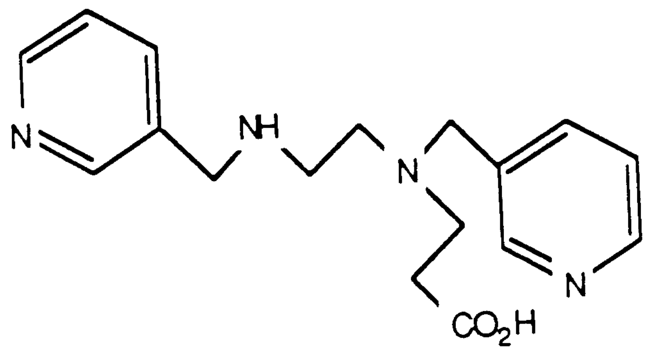

**1ère étape**

Synthèse du bis-1,6(pyridyl-3)diaza-2,5 hexane (B2)

**2ème etape**

Condensation de l'acide bromo-3propanoïque sur l'amine B2 obtenue:

A 12g d'amine B2 obtenue comme décrit plus haut (50 mmole) portés à reflux dans 125ml d'éthanol et 25ml d'eau, est ajoutée goutte à goutte sur 2h environ une solution de 1,27g d'acide bromo-3propanoïque (8,3 mmole) et 0,7g d'hydroxyde de lithium monohydraté (16,67 mmole) dans 35 ml d'eau. Le chauffage à reflux est prolongé pendant 9 heures après la fin de l'ajout. L'éthanol est évaporé sous vide et la solution aqueuse résiduelle orange est lavée sept fois par 50 ml de dichlorométhane. Le produit brut est recueilli par évaporation et est purifié après protonation par une solution à 5% d'acide chlorhydrique dans l'éthanol en effectuant une recristallisation dans un mélange éthanol/acide chlorhydrique dilué à 5%. On recueille ainsi 1,12g de poudre blanche correspondant au produit visé.

RMN$^1$H($D_2O$) à pH = 9 : $\delta$ 2,58(t,2H,NH-$CH_2$-$CH_2$-N-); 2,72(t,2H,NH-$CH_2$-$CH_2$-N-); 2,91(t,2H,$CH_2$-$CH_2$-$CO_2H$);3,16(t,2H,$CH_2$-$CH_2$-$CO_2H$); 3,65(s,2H,$C_5H_4N$-$CH_2$); 3,68(s,2H,$C_5H_4N$-CH'); 7,34(t,1H,$H_5$-Py);7,37(t,1H,$H_5$'-Py); 7,83(t,1H,$H_4$-Py);7,84(t,1H,$H_4$'-Py); 8,39(d,1H,$H_6$-Py);8,42(d,1H,$H_6$'-Py); 8,52(d,1H-$H_2$-Py);8,56(d,1H,$H_2$'-Py);

6) Les exemples 6a et 6b qui suivent comprennent une première étape commune de préparation d'un dinitrile selon la méthode de condensation ($\delta$) et une seconde étape commune de condensation selon le mode a) du bromure de benzyle sur le dinitrile sus-mentionné, suivie d'une étape de condensation avec un halogénure d'acide selon la méthode ($\alpha$) puis d'une étape d'hydrogénation des deux fonctions nitrile (selon la méthode ($\delta$)).

Les deux étapes communes sus-mentionnées sont réalisées de la manière suivante :

**1ère étape**

Préparation du dinitrile:

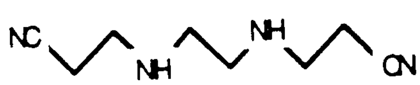

On ajoute 130 ml d'acrylonitrile (1,98 mole) sur une durée d'une heure environ à 65 ml d'éthylènediamine (0,35 mole). Le milieu réactionnel s'échauffe légèrement. Après agitation à température ambiante pendant 4 jours, on recueille un liquide incolore qui est utilisé sans autre purification.

RMN$^1$H($D_2O$): $\delta$ 2,65(t,4H,$CH_2$-CN); 2,73(s,4H,NH-$CH_2$-$CH_2$-NH); 2,90(t,4H,$CH_2$-$CH_2$-CN);

**2ème étape**

Préparation du composé S par condensation du bromure de benzyle sur le dinitrile obtenu à l'étape précédente

On porte à reflux dans 150 ml d'éthanol 15g du dinitrile obtenu précédemment (90 mmole) et 5,75g de carbonate de potassium (47 mmole). A cette solution est ajoutée sur une heure, une solution de 3ml de bromure de benzyle dans 15 ml d'éthanol. Le chauffage à reflux est alors prolongé pendant 3 heures après la fin de l'ajout. Le solvant est alors éliminé par évaporation sous vide et l'huile résiduelle orange dissoute dans 150 ml d'eau. Le nitrile benzylé est extrait par le dichlorométhane. Après séchage sur sulfate de magnésium, filtration et évaporation, 6,2g de produit brut sont recueillis. Le produit est alors purifié par chromatographie sur colonne et 3,6g de produit pur sont recueillis sous forme d'huile jaune.

RMN$^1$H($D_2O$): $\delta$ 2,43(t,2H,$CH_2$-CN);2,44(t,2H,$CH_2'$-CN); 2,67(s,4H,NH-$CH_2$-$CH_2$-NH); 2,81(t,2H,$CH_2$-$CH_2$-CN);2,81(t,2H,$CH_2'$-$CH_2$-CN); 3,64(s,2H,$CH_2$-Ph);7,32(s,5H,H-Ph)

- Exemple 6a : SYNTHESE DU DERIVE DE FORMULE L19

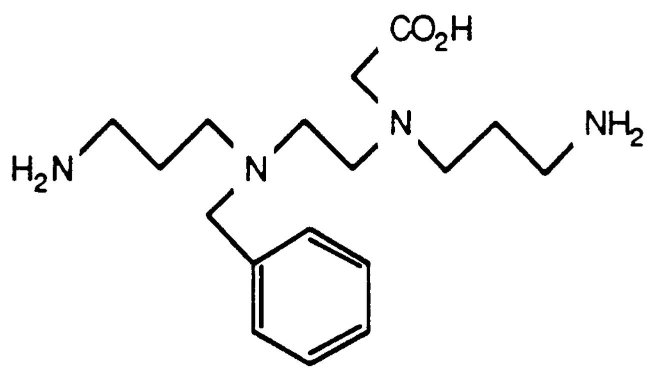

**1ère étape**

Préparation du dinitrile

**2ème étape**

Préparation du composé S par condensation du bromure de benzyle sur le dinitrile obtenu à l'étape précédente

**3ème étape**

Condensation de l'acide bromoéthanoïque sur le dinitrile benzylé S

On porte à reflux 3,1g de dinitrile benzylé S obtenu précédemment (12,1 mmole) dans 35 ml d'éthanol et 7 ml d'eau. A cette solution est ajoutée sur une heure environ, une solution de 1,65g d'acide bromoéthanoïque (11,8 mmole) et 1g d'hydroxyde de lithium monohydraté (23,8 mmole) dans 35 ml d'eau. Le chauffage à reflux est alors prolongé pendant 9 heures après la fin de l'ajout. Le solvant est alors éliminé par évaporation sous vide et l'huile résiduelle orange mise en solution dans 50ml d'eau. Le produit désiré est recueilli par extraction par le dichlorométhane et purifié par chromatographie liquide sur colonne.

RMN$^1$H($D_2O$): $\delta$ 2,43(t,2H,$CH_2$-CN);2,44(t,2H,$CH_2'$-CN); 2,67(s,4H,NH-$CH_2$-$CH_2$-NH); 2,81(t,2H,$CH_2$-$CH_2$-CN);2,81(t,2H,$CH_2'$-$CH_2$-CN); 3,12(t,2H,$CH_2CO_2H$); 3,64(s,2H,$CH_2$-Ph);7,32-(s,5H,H-Ph)

**4ème étape**

Réduction des fonctions nitrile par le dihydrogène en présence de nickel de Raney

Une solution de 2g du produit obtenu précédemment dans 30 ml d'éthanol et 1g de nickel de Raney sont placés dans un réacteur. L'hydrogénation est alors réalisée en établissant une pression d'hydrogène d'environ 50 bars pendant 24 heures. Le catalyseur est éliminé par filtration et le solvant par évaporation sous vide. L'huile résiduelle orange est purifiée par chromatographie liquide sur colonne. On recueille alors 1,2 g d'huile jaune pâle correspondant au produit attendu.

RMN$^1$H($D_2O$) pH = 9 : δ 1,72(quint,2H,$CH_2$-$CH_2$-$CH_2$-$NH_2$); 1,72(quint,2H,$CH_2$-$CH_2$'-$CH_2$-$NH_2$);2,63-(t,2H,NH-$CH_2$-$CH_2$-$NH_2$); 2,65(t,2H,NH-$CH_2$-$CH_2$'-$NH_2$); 2,68(t,2H,$CH_2$-$CH_2$-$CH_2$-$NH_2$);2,69(t,2H,$CH_2$'-$CH_2$-$CH_2$-$NH_2$);2,71(t,2H,$CH_2$-$NH_2$);2,73(t,2H,$CH_2$'-$NH_2$); 2,84(t,2H-$CH_2CO_2H$); 3,64(s,2H,$CH_2$-Ph);7,32(s,5H,H-Ph)

- Exemple 6b : SYNTHESE DU DERIVE DE FORMULE L20

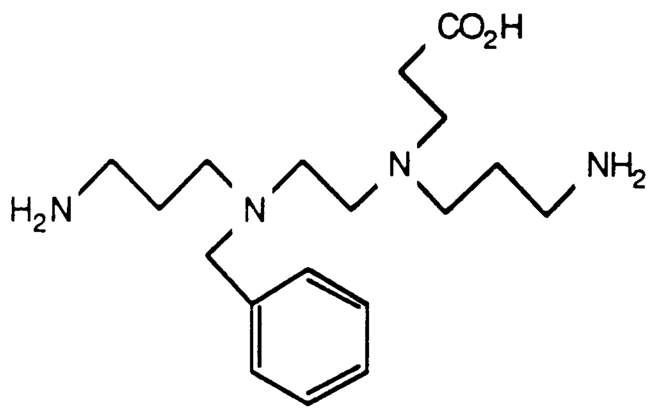

**1ère étape**

Préparation du dinitrile

**2ème étape**

Préparation du composé S par condensation du bromure de benzyle sur le dinitrile

**3ème étape**

Condensation de l'acide iodo-3propanoïque sur le dinitrile benzylé S

Le dinitrile benzylé S obtenu comme décrit plus haut (14 mmole) est porté à reflux dans 42 ml et 8,4 ml d'eau. A cette solution est ajoutée sur une heure environ, une solution de 2,8 g d'acide iodo-3propanoïque (14 mmole) et 1,18 g d'hydroxyde de lithium monohydraté (28 mmole) dans 35 ml d'eau. Le chauffage à reflux est alors prolongé pendant 9 heures après la fin de l'ajout. Le solvant est alors éliminé par évaporation sous vide et l'huile résiduelle orange mise en solution dans 50 ml d'eau. Le produit désiré est recueilli par extraction par le dichlorométhane et purifié par chromatographie liquide sur colonne.

RMN $^1$H ($D_2O$): δ 2,41 (t, 2H, *$CH_2$*- CN); 2,42 (t, 2H, *$CH_2$*'-CN); 2,68 (s, 4H, NH-*$CH_2$*-*$CH_2$*- NH); 2,79 (t, 2H, *$CH_2$*-$CH_2$-CN); 2,79 (t, 2H, *$CH_2$*'-$CH_2$-CN); 3,12 (t, 2H, -*$CH_2$*-$CO_2H$); 3,15 (t, 2H, -*$CH_2$*-$CH_2$-$CO_2H$); 3,66 (s, 2H, *$CH_2$*-Ph); 7,35 (s, 5H, *H*-Ph)

**4ème étape**

Réduction des fonctions nitrile par le dihydrogène en présence de nickel de Raney.

Une solution de 2 g du produit obtenu comme décrit plus haut dans 30 ml d'éthanol et 1 g de nickel de Raney est placée dans un réacteur. L'hydrogénation est alors réalisée en établissant une pression d'hydrogène d'environ 50 bars pendant 24 heures. Le catalyseur est éliminé par filtration et le solvant par évaporation sous vide. L'huile résiduelle orange est purifiée par chromatographie liquide sur colonne. On recueille alors 1,05 g d'huile jaune pâle correspondant au produit attendu.

RMN $^1H$ ($D_2O$) pH ≃ 9 : δ 1,70 (quint., 2H, $CH_2$-$CH_2$- $NH_2$); 1,70 (quint., 2H, $CH_2$-$CH_2'$-$CH_2$-$NH_2$); 2,59 (t, 2H, NH-$CH_2$-$CH_2$-NH); 2,61 (t, 2H, NH-$CH_2$-$CH_2'$- NH); 2,66 (t, 2H, $CH_2$-$CH_2$-$CH_2$-$NH_2$); 2,70 (t, 2H, $CH_2'$-$CH_2$- $CH_2$-$NH_2$); 2,73 (t, 2H, $CH_2$-$NH_2$) ; 2,74 (t, 2H, $CH_2'$-$NH_2$) ; 2,84 (t, 2H, -$CH_2$-$CO_2H$); 3,11 (t, 2H, -$CH_2$-$CO_2H$); 3,64 (s, 2H, $CH_2$-Ph); 7,33 (s, 5H,*H* -Ph)

7) - Exemple 7 : SYNTHESE DE DERIVES SELON LA CONDENSATION a) PAR REACTION D'UN CHLORURE D'ALCOYLE AVEC UNE AMINE

Les conditions générales utilisées sont notamment les suivantes :

On porte à reflux une solution de 0,1 mole d'amine dans 100 ml de toluène en présence de 0,1 mole d'amidure de sodium. On ajoute ensuite goutte à goutte une solution de 0,1 mole de chlorure d'alcoyle dans 100 ml de toluène et l'ébullition est maintenue pendant 1 heure. Après refroidissement, le mélange réactionnel est repris par 100ml d'eau. La phase organique est décantée, évaporée sous vide et le produit obtenu est dissous dans l'éthanol et précipité en faisant passer un courant de chlorure d'hydrogène. On recueille le produit après filtration, lavage à l'éthanol et recristallisation du brut obtenu dans un mélange eau/acide chlorhydrique/éthanol.

En opérant comme indiqué ci-dessus, les composés figurant dans le tableau II, ont été préparés en partant d'une amine HN($R_2$, $R_3$) et d'un chlorure $R_1$-Cl pour lesquels les significations des substituants sont respectivement indiquées

TABLEAU II

R 3 | R 2 | R 1 | dérivé obtenu

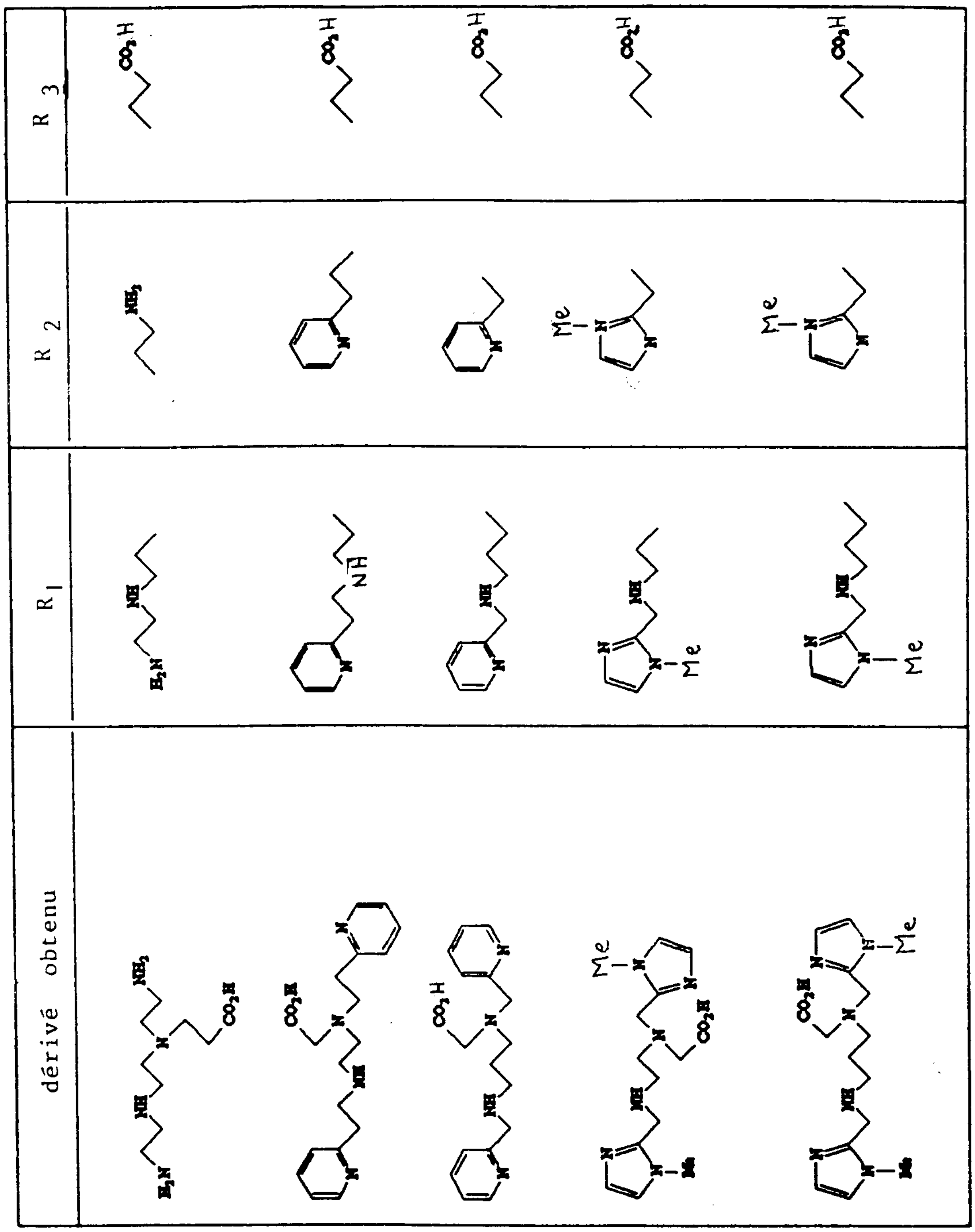

8) - Exemple 8 : SYNTHESE DE DERIVES SELON LA CONDENSATION c) PAR REACTION D'UNE PIPERAZINONE ET D'UN CHLORURE D'ALCOYLE

La réaction de condensation est effectuée notamment comme suit :

**1ère étape :**

Synthèse de la pipérazinone

On ajoute lentement (3 heures environ), 0,15 mole de chloroacétate d'éthyle (JANSSEN CHIMICA, réf.22041.22) dans 100 ml d'éthanol à une mole d'éthylènediamine dissoute dans 300 ml d'éthanol, puis 0,15 mole d'éthylate de sodium. Le précipité formé est éliminé par filtration, le filtrat est évaporé et l'excès d'éthylènediamine chassé par distillation. La pipérazinone est alors formée par chauffage à 200 °C sous pression réduite (5 mmHg) et recristallisée dans un mélange acétone/éther de pétrole.

**2ème étape :**

Condensation du chlorure d'acide sur la pipérazinone

On porte à reflux une solution de 0,1 mole de piperazinone dans 100 ml de toluène en présence de 0,1 mole d'amidure de sodium. On ajoute ensuite goutte à goutte une solution de 0,1 mole de chlorure d'alkyle dans 100 ml et poursuit l'ébullition pendant 1 heure. Après refroidissement, le mélange réactionnel est lavé à l'eau, évaporé à sec et le produit obtenu utilisé sans autre purification.

**3ème étape :**

Hydrolyse de la pipérazinone

Le produit décrit ci-dessus est porté à reflux pendant 10 heures dans 100 ml d'eau et 100 ml d'acide sulfurique concentré. Après concentration à 100 ml, l'acide est précipité par ajout d'éthanol et refroidissement à -15 °C. Le produit attendu est recueilli par filtration, lavage à l'éthanol et recristallisation dans un mélange eau/acide sulfurique/éthanol.

Dans les tableaux IIIa et IIIb, on indique les produits obtenus en partant des chlorures $R_1$-Z et de pipérazinone.

TABLEAU IIIa : monocondensation

| | | | | | |
|---|---|---|---|---|---|
| Piperazinone | 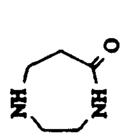 | 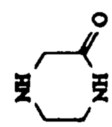 | 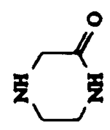 | 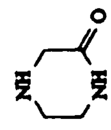 | 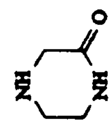 |
| chlorure d'alcoyle $R_1Cl$ | 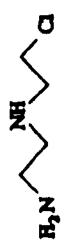 | 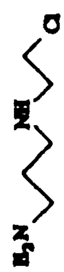 | 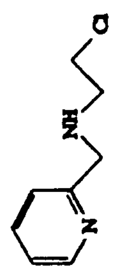 | 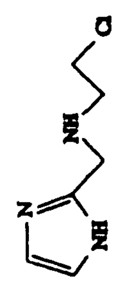 | 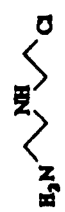 |
| Dérivé obtenu | 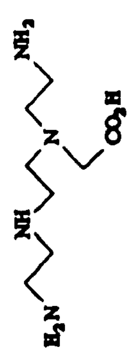 | 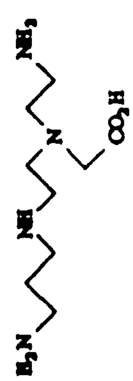 | 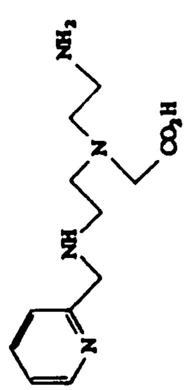 | 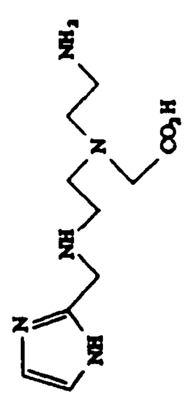 | 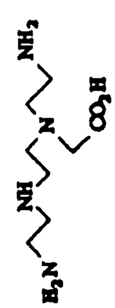 |

## TABLEAU IIIb : dicondensation

| Dérivé obtenu | chlorure d'alcoyle | Piperazinone |
| --- | --- | --- |
| 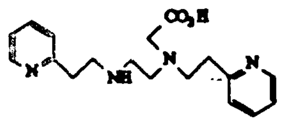 3 | 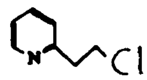 | 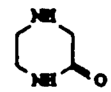 |
| 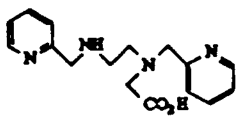 4 | 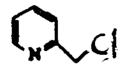 | 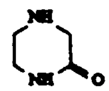 |
| 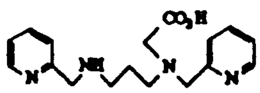 5 | 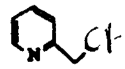 | 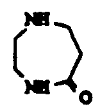 |
| 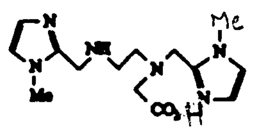 6 | 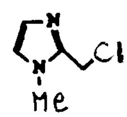 | 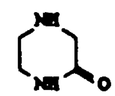 |
| 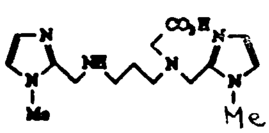 7 | 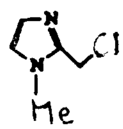 | 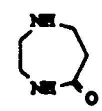 |
| 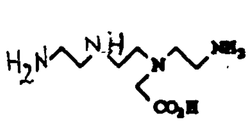 14 | 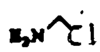 | 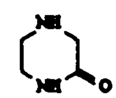 |

9) - Exemple 9 : Synthèse de dérivés par réaction d'une amine secondaire avec un halogénure d'ester.

On fait réagir du bromoacétate d'éthyle (JANSSEN CHIMICA, réf.15859.48) avec une amine secondaire ($R_2$, $R_3$)NH dans les conditions suivantes :

on porte à reflux pendant plusieurs heures (4 à 12 heures) 0,1 mole d'amine, 0,1 mole de bromoacétate d'éthyle et 0,1 mole de carbonate de sodium dans 200 ml d'éthanol. Par distillation, on chasse ensuite, 100 ml d'éthanol et la solution résiduelle est additionnée de glace pilée. Le précipité obtenu est recueilli par filtration, puis séché.

Le solide isolé, sans autre purification, est porté à reflux pendant environ 2 heures dans 50 ml d'eau et 50 ml d'acide sulfurique concentré (d = 1,83). L'acide formé est précipité par ajout d'éthanol et maintenu à -15 °C pendant 24 heures. Le précipité est ensuite filtré et recristallisé dans un mélange eau/acide sulfurique/éthanol. Après filtration, lavage par l'éthanol et séchage, l'acide est recueilli sous forme de poudre blanche.

On indique dans le tableau IV les produits obtenus selon l'amine de départ utilisée.

TABLEAU IV

| Dérivé obtenu | | Amine secondaire | Traitement |
|---|---|---|---|
| 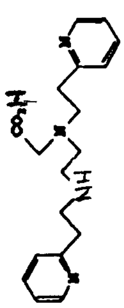 | 3 | 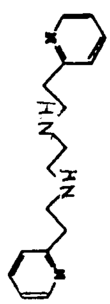 | hydrolyse |
| 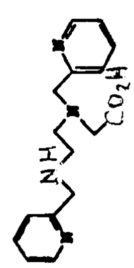 | 4 | 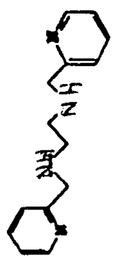 | hydrolyse |
| 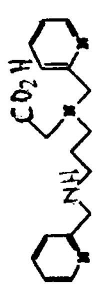 | 5 | 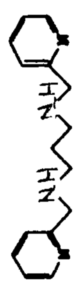 | hydrolyse |
| 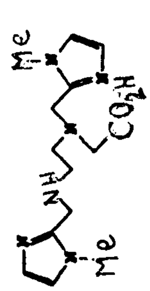 | 6 | 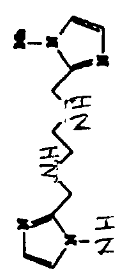 | hydrolyse |
| 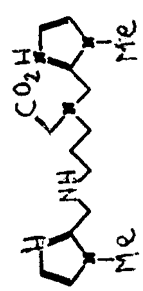 | 7 | 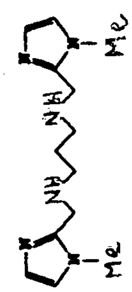 | hydrolyse |
| 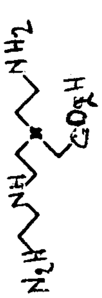 | 14 | 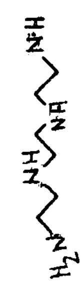 | hydrolyse |

10) - Exemple 10 : Synthèse selon la condensation d) d'un dérivé cyclique de formule

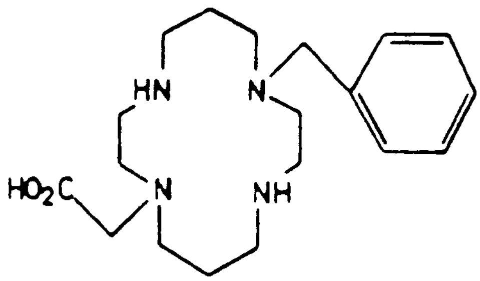

Cette synthèse est effectuée selon les étapes suivantes:

1 : réaction d'un dinitrile et du bromure de benzyle selon le schéma :

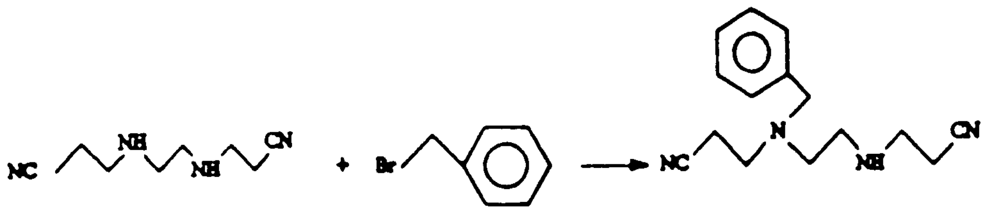

2 : réduction pour obtenir le dérivé diamino correspondant de formule

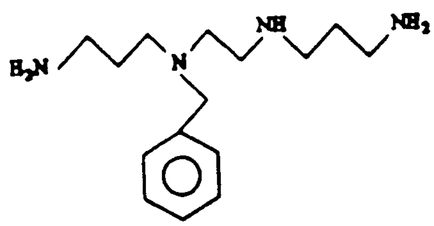

3 : condensation avec un dialdéhyde en présence de chlorure de nickel hexahydraté selon le schéma :

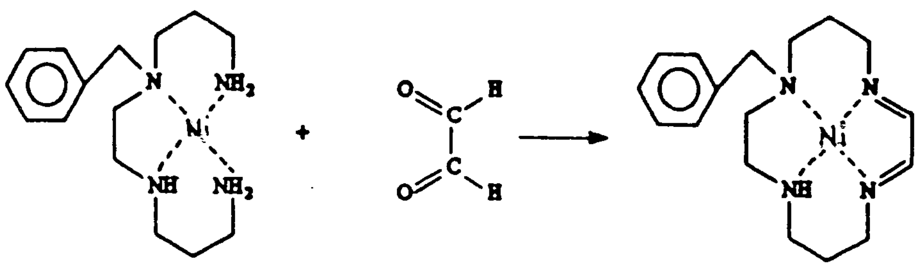

4 : Hydrogénation, suivie d'une réaction de décomplexation par traitement avec NaCN, selon :

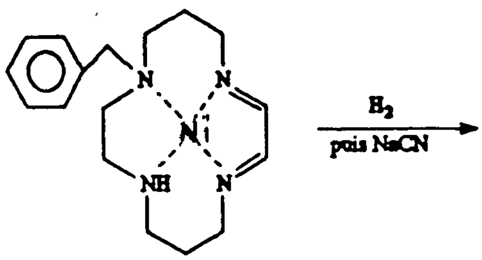


5 : Fixation d'une chaîne à terminaison carboxylique selon :

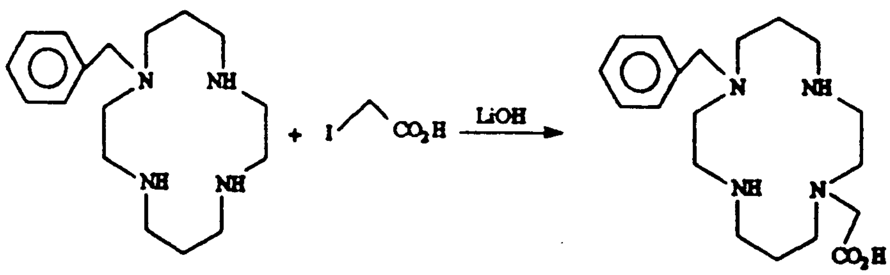


Ces étapes sont réalisées comme suit :

1 : réaction d'un dinitrile avec un bromure de benzyle

Après addition rapide de 35g de N,N'-bis(cyano-2-éthyl)-diamino-1,2 éthane dans 274 ml d'éthanol absolu, on ajoute lentement et goutte à goutte 24,8 ml (0,210 mole) de bromure de benzyle. On porte cette solution à reflux pendant 2 heures puis on laisse revenir à température ambiante. Le précipité blanc est éliminé par £iltration, le filtrat est évaporé et on isole 76,5 g d'une huile jaune.

On ajoute 111 ml d'eau distillée et 58 ml de chloroforme à l'huile obtenue et après agitation vigoureuse, on ajuste le pH à 8 par addition de 11 ml de soude à 30 %. La phase organique est alors isolée, séchée sur sulfate de magnésium, filtrée et évaporée. On isole 48 g d'huile jaune.

Après chromatographie sur silice sous moyenne pression, on isole 8,6 g d'une huile correspondant au produit de monobenzylation.

2 : Réduction

On procède à l'hydrogénation de 7 g de cette huile dissous dans 35 ml d'éthanol. Le catalyseur est éliminé par filtration sur Célite (marque déposée) et le filtrat est évaporé à sec.

On additionne 3 ml d'acide chlorhydrique concentré et 30 ml d'éthanol absolu. On isole le produit attendu sous forme de chlorhydrate.

3 : Condensation avec le dialdéhyde.

A 4 g de N-benzyl bis(amino-3 propyl) diamino-1,2 éthane dissous dans 60,6 ml d'eau, on ajoute 3,6 g de chlorure de nickel hexahydraté et on refroidit le milieu réactionnnel à 5 ° C. On additionne alors goutte à goutte 2,5 ml de glyoxal à 40 % en solution aqueuse.

4 : Réaction d'hydrogénation et de décomplexation.

Après une nuit à température ambiante on procède à la réaction d'hydrogénation sous une pression de 20 bars dans un autoclave, en présence de 1,5 g de nickel de Raney à 50 % dans l'eau.

Après avoir éliminé le nickel par filtration, on ajoute 3,8 g de cyanure de sodium au filtrat et on porte le tout à reflux pendant 2 heures. La solution est alors refroidie et extraite plusieurs fois au chloroforme. On

sèche alors la phase organique sur sulfate de magnésium et on évapore à sec pour récupérer le produit attendu.

5 : Greffage de la chaîne carboxylique

A 3 g (0, 01 mole) de cyclame N-benzylé, dissous dans 45 ml d'éthanol et 9 ml d'eau, on ajoute 0,168 g (0,004 mole) d'hydroxyde de lithium monohydraté. A 3 °C, on additionne ensuite goutte à goutte 0,002 mole d'acide bromoacétique dans 7 ml d'eau. La solution est portée à reflux pendant 21 heures puis les solvants sont éliminés par évaporation. Le produit obtenu est redissous dans 10 ml d'eau et 15 ml de chloroforme. Après plusieurs extractions au chloroforme, la phase aqueuse est isolée et concentrée. Après ajout de 3 ml d'acide chlorhydrique concentré goutte à goutte, le cyclame disubstitué est précipité par addition de 30 ml d'éthanol absolu et maintien pendant 1 jour à -15 °C.

Le précipité est filtré et recristallisé dans un mélange éthanol/eau/HCl.

11) - Exemple 11 : MODE DE PREPARATION GENERALE DES COMPLEXES II DE FORMULE X = $LCo^{II}$.

Sous atmosphère d'argon, on dissout 0,2 mmole de ligand L dans 20 ml d'eau désionisée et dégazée. Le pH de la solution obtenue après dissolution totale de L est différent du pH de l'eau pure et dépend de la nature et du mode d'isolement de L (par exemple, dérivé à l'état pur obtenu par distillation ou sel d'acide isolé par précipitation acide).

Le pH de la solution est ensuite ajusté par addition d'acide ou de base à une valeur permettant la prédominance de la forme neutre LH ou déprotonée $L^-$ du ligand dans le milieu. L'introduction du sel de cobalt, préalablement conditionné sous argon, conduit rapidement à la formation du complexe $LCo^{II}$. La formation de $LCo^{II}$ est détectée et suivie par spectrophotométrie UV-Visible (intensité d'une bande caractéristique de transfert de charge $L \rightarrow Co^{II}$ entre 190 et 290 nm, selon le ligand mis en oeuvre). A titre d'exemple, le tableau V rassemble les longueurs d'onde de la bande de transfert de charge caractéristique du complexe $LCo^{II}$ pour divers ligands L ainsi que la valeur de pH pour laquelle l'intensité de cette bande est maximum.

Tableau V

| Ligand | λmax(nm) | pH |
|---|---|---|
| L1 | 197 | 6.9 |
| L2 | 206 | 8.7 |
| L6 | 204 | 6.8 |
| L9 | 255 | 5.5 |
| L10 | 255 | 5.1 |
| L12 | 212 | 5.1 |
| L14 | 266 | 5.0 |
| L16 | 250 | 8.0 |

Il est à noter que, l'ion $Co^{2+}$ adoptant habituellement en solution une coordination octaédrique, un ou deux coordinants supplémentaires (selon que le ligand polyazoté de l'invention est pentadentate ou tétradentate) complètent le schéma de coordination du coeur métallique. Ces coordinants peuvent être soit des contre-ions, par exemple chlorure ou acétate, des ions hydroxydes, $OH^-$ (cas d'un milieu suffisamment basique), ou bien des molécules d'eau.

La formation des complexes $LCo^{II}$ est également suivie par spectroscopie RNM$^1$H. Après complexation les signaux des divers protons du ligand L résonnent dans une gamme de fréquences de 250 ppm à -100 ppm comparée à la gamme 0 à 10 ppm du ligand seul. Cette variation très importante des fréquences de résonance des protons de $LCo^{II}$ résulte de la distribution de densité de charge non appariée du coeur métallique aux divers atomes de carbone du ligand L (on rappelle que la configuration électronique externe du cation $Co^{2+}$ est $3d^7$).

12) - Exemple 12 : PREPARATION DU COMPLEXE METALLIQUE DE FORMULE

$Co^{II}(C_6H_5CH_2\text{-}NH\text{-}(CH_2)_2NH(CH_2)_2NH(CH_2)_2COOH)$

On dissout 96 mg de [$C_6H_5CH_2NH(CH_2)_2NH(CH_2)_2NH-(CH_2)_2NH-(CH_2)_2-COOH$, $2H_2SO_4$, $H_2O$] dans 20 ml d'eau, à un pH de 7,47, sous argon, à 22°C. Après dissolution totale du ligand (ou encore dérivé de l'invention), le pH est de 2,25.

L'ajout de 50 mg d'acétate de cobalt tétrahydraté à la solution induit une évolution du pH qui atteint une valeur d'équilibre de 3,51.

L'ajout de soude à cette solution permet ensuite d'ajuster le pH à la valeur souhaitée.

Pour une concentration en complexe métallique de $7.10^{-3}$M, on ajuste le pH à une valeur de 7,37.

Chacune des étapes de cette préparation est effectuée sous atmosphère inerte et les réactifs sont préalablement conditionnés de façon à ne contenir aucune trace d'oxygène.

Si ces étapes sont conduites en présence d'oxygène, le complexe métallique réagit avec l'oxygène pour conduire au dimère péroxo $LCoO_2CoL$ dans lequel L représente le dérivé d'amine de l'invention.

13) - Exemple 13 : DETERMINATION DE LA QUANTITE D'OXYGENE ABSORBE PAR UN COMPLEXANT DE L'INVENTION

En présence d'oxygène les complexes $LCo^{II}$ conduisent rapidement à un composé $\mu$-peroxo $LCoO_2CoL$ selon :

$LCo + O_2 <=> LCoO_2$
$LCoO_2 + LCo <=> LCoO_2CoL$

La quantité d'oxygène absorbé ainsi que la cinétique d'absorption dépendent de plusieurs paramètres dont on notera en particulier le pH. Ceci est illustré par la Figure 1 dans le cas du ligand L7.

Les mesures d'absorption sont effectuées par volumétrie, par potentiométrie ou par spectrophométrie UV-Visible.

14) - Exemple 14 : DETERMINATION DE LA QUANTITE D'OXYGENE ABSORBE PAR UN COMPLEXANT DE L'INVENTION

**A. Par volumétrie :**

. Méthode générale

On effectue une mesure directe de la quantité d'oxygène absorbé par une solution de complexe métallique en opérant comme suit :

Dans un ballon tricol de 100 ml, 20 ml d'une solution aqueuse de ligand à la concentration de $10^{-2}$M et un pH connu, est en équilibre avec un mélange gazeux contenant de l'oxygène à une pression partielle connue, le tout étant maintenu à une température constante de 25°C.

Un tube capillaire préalablement calibré, contenant un index de mercure, est relié au ballon, de même qu'un capteur de pression et un doigt en verre coudé, capable de contenir un sel métallique.

Lorsque l'ensemble des différentes parties du montage est à l'équilibre et isolé de l'extérieur par un système de fermeture étanche, et lorsque le sel métallique est introduit par rotation du doigt coudé, dans la solution maintenue sous agitation, l'abaissement de la pression à l'intérieur du montage, provoqué par l'absorption de l'oxygène contenu dans le mélange gazeux environnant la solution, est instantanément compensé par le déplacement de l'index de mercure dans le capillaire. Lorsque l'index est stabilisé, un nouvel équilibre est atteint et la mesure du volume correspondant est une mesure directe du volume de gaz absorbé. Il est alors possible d'évaluer les concentrations respectives de $XO_2X$ et X et ainsi d'évaluer la constante $K0_2$ (une correction est cependant nécessaire à apporter en raison de la variation de la pression partielle de $0_2$ dans l'atmosphère avant et après équilibre).

. Résultats obtenus avec le ligand $pF(C_6H_4)-CH_2-NH-(CH_2)_2-NH-(CH_2)_2-NH-(CH_2)_2-COOH$ (pF = parafluoro)

On ajoute 0,053 g d'acétate de cobalt à 20 ml d'une solution aqueuse contenant ce ligand à une concentration de $10^{-2}$M, à un pH de 7,56. On observe un déplacement de volume de 0,294 ml. Dans les conditions de température et de pression de l'expérience, ce volume correspond à 1,19 $10^{-5}$ mole d'oxygène absorbé.

En tenant compte des équilibres de protonation et métallation du complexe, il est alors possible de calculer une constante $K0_2$ de 2,6 x $10^4$.

Cette valeur est confirmée par une expérience réalisée avec une solution de ce même ligand, de même concentration, à un pH initial de 8,10, avant introduction du sel de cobalt. Le déplacement est alors de 0,464 ml, ce qui correspond à un absorption de 1,86 x $10^{-5}$ mole de $O_2$.

**B. Titrage potentiométrique**

Par titrage potentiométrique d'une solution contenant un ligand et un sel métallique, en équilibre avec un mélange gazeux contenant de l'oxygène, il est possible d'évaluer la constante $KO_2$ en calculant la différence avec le titrage réalisé en anaérobie.

**C. Par spectrophotométrie UV visible.**

Une solution aqueuse contenant un ligand et un sel métallique, quelque soit leur concentration respective, présente un spectre d'absorption rigoureusement plat entre 300 et 500 nm, lorsque cette solution est maintenue sous atmosphère dépourvue d'oxygène.

Lorsque cette solution est mise en contact avec l'air ou un mélange gazeux contenant de l'oxygène, un large pic d'absorption apparaît entre 300 et 500 nm, et plus généralement entre 300 et 400 nm. L'intensité de ce pic d'absorption varie en fonction du pH de la solution et de la pression partielle d'oxygène de l'atmosphère en équilibre avec la solution.

Le tableau VI rassemble les longueurs d'onde de ce pic caractéristique du complexe $LCoO_2CoL$ (ou bande de transfert de charge cobalt → dioxygène), ainsi que les valeurs de pH des solutions utilisées pour effectuer les mesures

Tableau VI

| Ligand | λmax(nm) | pH |
|---|---|---|
| L1 | 314 | 4.6 |
| L2 | 370 | 7.0 |
| L3 | 368 | 5.9 |
| L4 | 368 | 6.2 |
| L6 | 375 | 6.0 |
| L7 | 371 | 5.8 |
| L8 | 328 | 6.3 |
| L9 | 370 | 5.7 |
| L10 | 365 | 7.7 |
| L12 | 315 | 5.5 |
| L13 | 358 * | 6.5 |
| L14 | 320 * | 6.7 |
| L16 | 374 | 6.0 |

* épaulement ; λ mal défini

Les spectres UV visible enregistrés pour des ligands de l'invention en présence d'acétate de cobalt, en équilibre avec l'oxygène de l'air, à deux pH différents, montrent que le rapport des intensités maximales des pics correspond parfaitement au rapport des volumes de $O_2$ absorbé, mesurés pour ces mêmes ligands dans des conditions de pH et température identiques à celles des enregistrements de spectres.

<u>Stabilité dans le temps des complexes dioxygénés $LCoO_2CoL$</u> :

Les complexes dioxygénés $LCoO_2CoL$ évoluent dans le temps avec formation d'espèces qui ne fixent pas l'oxygène. Ces espèces résultent :

- soit d'une oxydation centrométallée du complexe $\mu$-peroxo conduisant à une espèce $LCo^{III}$,
- soit d'une oxydation du ligand organique qui modifie la structure, donc les propriétés du complexant qui en résulte,
- soit d'un échange de ligands ou coordinants (autres que L) du cobalt conduisant à une espèce coordinativement saturée et stable.

La perte des propriétés complexantes de $LCo^{II}$ vis-à-vis de $O_2$ (ou dégradation) est suivie dans le temps par la mesure de la diminution de l'intensité de la bande de transfert de charge cobalt → dioxygène des complexes $\mu$-peroxo $LCoO_2CoL$.

Le mode et la vitesse de dégradation dépendent considérablement de la structure du ligand L. Ainsi, les ligands pentadentates linéaires conduisent à des solutions totalement dégradées (qui ne fixent plus le dioxygène) en quelques heures, tandis que les ligands tétradentates linéaires ou pentadentates branchés nécessitent plusieurs mois.

L'étude RMN des solutions dégradées pour les ligands pentadentates linéaires montre que la dégradation est du type oxydation centrométallée (formation d'un complexe $LCo^{III}$).

Cependant de telles solutions sont particulièrement appropriées pour la séparation du dioxygène d'un mélange de gaz le contenant si la désorption est effectuée par la voie électrochimique. En effet, dans ce cas, les complexes de $Co^{III}$ sont réduits dans le compartiment cathodique de la cellule électrochimique en complexes de $Co^{II}$, ces derniers fixant à nouveau le dioxygène.

Ces observations sont rapportées Figures 2 et 3 respectivement pour deux séries de ligands sélectionnées. Sur ces figures, on observe en particulier la dégradation totale en 2,5 heures du ligand pentadentate linéaire L8 et la dégradation partielle du ligand tétradentate L2 (réduction de 75 % de la capacité initiale en 85 jours) ou bien du ligand pentadentate branché L16 (réduction de 70 % de la capacité initiale en 110 jours).

15) - Exemple 15 : DESORPTION DE L'OXYGENE COMPLEXE

La désorption de l'oxygène complexé peut s'effectuer par abaissement de la pression partielle de $O_2$ si la l'affinité, $KO_2$, est inférieure à $10^7$, ou par oxydation électrochimique.

Ces deux méthodes sont testées pour les ligands décrits dans cette invention.

**1. Abaissement de la pression partielle de $O_2$.**

La mesure de la quantité d'oxygène désorbé par abaissement de la pression partielle de $O_2$ est effectuée par spectrophotométrie UV visible.

L'intensité du pic d'absorption entre 300 et 400 nm étant directement proportionnelle à la concentration en forme $XO_2X$, la différence des intensités de ce pic enregistré pour une solution en équilibre avec une atmosphère riche en oxygène puis appauvrie en oxygène, est une mesure directe de la quantité d'oxygène désorbé.

Ce type de mesure est illustré Figure 4.

**2. Désorption par oxydation électrochimique.**

L'oxydation électrochimique de $XO_2X$ entraîne la désorption de $O_2$ selon :

$$XO_2X \rightarrow 2X^+ + O_2 + 2e^-$$

L'évaluation du potentiel à appliquer pour que l'oxydation ait lieu et que la désorption intervienne, est effectuée à l'aide d'une cellule électrochimique d'une capacité volumique de 20 ml, constituée de deux compartiments séparés par une membrane de type anionique (RAI4O35). Les électrodes utilisées sont celles qui ont été évaluées comme permettant le meilleur transfert électronique. Classiquement, on enregistre le courant qui traverse la cellule pour un potentiel imposé entre les deux électrodes. Les courbes intensité/potentiel résultant de cette étude permettent de déterminer le potentiel minimal qu'il est nécessaire d'appliquer pour que la réaction d'oxydoréduction intervienne. La quantité d'oxygène désorbé est ensuite évaluée en reliant le compartiment anodique de la cellule électrochimique à un volumètre (BROOKS EMERSON), qui permet une lecture directe du volume d'$O_2$ désorbé à tout instant au cours de l'électrolyse.

## Revendications

**1.** Dérivés polyazotés correspondant à la formule générale suivante :

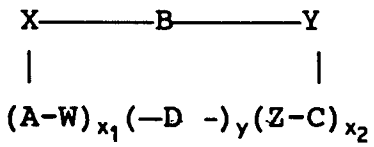

(I)

dans laquelle y vaut 0 ou 1,

a) lorsque y = 0 :

. $x_1$ et $x_2$ valent 0 ou 1, $x_1$ et $x_2$ ne pouvant pas valoir 0 simultanément ;

. les constituants A, B et C, identiques ou différents, représentent :

- une chaîne alcoylène $-(CH_2)_x-$, dans laquelle x représente un nombre entier de 1 à 4, le cas échéant substituée par un, ou plusieurs, groupe P, ou par un, ou plusieurs, groupe $P_1$, $P_1$ représentant un groupe alcoyle de 1 à 4 atomes de carbone, le cas échéant substitué par un, ou plusieurs, groupe P, P représentant :
  * un groupe -COR dans lequel R représente un hydroxyle (-OH), une amine primaire, ou substituée par un, ou deux, groupe alcoyle de 1 à 4 atomes de carbone, une fonction -OR' dans laquelle R' représente soit un groupe alcoyle de 1 à 4 atomes de carbone, soit un cycle aromatique de 6 à 14 atomes de carbone le cas échéant substitué en position ortho et/ou méta et/ou para par un atome d'halogène, un groupe alcoyle de 1 à 4 atomes de carbone, alcoxy de 1 à 4 atomes de carbone, hydroxy, un groupe aryle, un hétérocycle aromatique, un groupe nitro, un groupe $-(CH_2)_t$-COR dans lequel R a la signification indiquée ci-dessus, et t représente un nombre entier de 0 à 4, ou une amine primaire ou substituée par un, ou deux, groupe alcoyle de 1 à 4 atomes de carbone ;
  * un cycle aromatique de 6 à 14 atomes de carbone, le cas échéant substitué en position ortho et/ou méta et/ou para par un atome d'halogène, un groupe alcoyle de 1 à 4 atomes de carbone, alcoxy de 1 à 4 atomes de carbone, hydroxy, un groupe aryle, un hétérocycle aromatique, un groupe nitro, un groupe $-(CH_2)_t$-COR dans lequel R a la signification indiquée ci-dessus, et t représente un nombre entier de 0 à 4, ou une amine primaire ou substituée par un, ou deux, groupe alcoyle de 1 à 4 atomes de carbone ;
  * un hétérocycle aromatique, notamment azoté, de 4 à 12 atomes de carbone, le cas échéant substitué en position ortho, et/ou méta, et/ou para, par un atome d'halogène, un groupe alcoyle de 1 à 4 atomes de carbone, alcoxy de 1 à 4 atomes de carbone, hydroxy, un hétérocycle aromatique, un groupe aryle, un groupe nitro, un groupe $-(CH_2)_t$-COR dans lequel R a la signification indiquée ci-dessus et t représente un nombre entier de 0 à 4, ou une amine primaire ou substituée par un, ou deux, groupe alcoyle de 1 à 4 atomes de carbone,
  * un groupe amine primaire,ou substitué par un, ou deux, groupe alcoyle de 1 à 4 atomes de carbone,
  * un groupe CN,
  * un groupe alcoyle de 1 à 4 atomes de carbone,
  * un groupe alcoxy de 1 à 4 atomes de carbone,
  * une fonction hydroxyle,
  * un groupe nitro,
  * un atome d'halogène,
- un cycle aromatique de 6 à 14 atomes de carbone dont 2 atomes de carbone sont engagés respectivement dans une liaison avec W et X dans le cas de A, ou avec X et Y dans le cas de B, ou avec Y et Z dans le cas de C, ce cycle aromatique étant le cas échéant substitué par un, ou plusieurs, groupe P et/ou $P_1$, P et $P_1$ ayant les significations indiquées ci-dessus,
- un groupe du type

$$-(CH_2)_{x_3}-V-(CH_2)_{x_4}-$$

dans lequel $x_3$ et $x_4$ sont des nombres entiers variant de 1 à 3, et V représente un cycle

aromatique de 6 à 14 atomes de carbone, le cas échéant substitué par un, ou plusieurs, groupe P et/ou $P_1$, P et $P_1$ ayant les significations indiquées ci-dessus,

. les constituants X et Y, identiques ou différents, représentent respectivement :

- lorsque $x_1$ et $x_2$ valent respectivement 1 :
  * un groupe

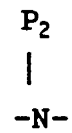

dans lequel $P_2$ représente un atome d'hydrogène ou le groupe P ou $P_1$, P et $P_1$ ayant les significations indiquées ci-dessus,

  * un hétérocycle aromatique azoté, de 4 à 12 atomes de carbone dont 2 atomes de carbone sont engagés respectivement dans une liaison avec A et B dans le cas de X, ou avec B et C dans le cas de Y, cet hétérocycle étant le cas échéant, substitué par un, ou plusieurs, groupe P et/ou $P_1$, P et $P_1$ ayant les significations indiquées ci-dessus,
- lorsque $x_1 = 0$ ou lorsque $x_2 = 0$, : le substituant Y ou X respectivement terminal représente
  * un groupe -COR dans lequel R a la signification indiquée ci-dessus.
  * un hétérocycle aromatique azoté, de 4 à 12 atomes de carbone, le cas échéant substitué en position ortho, et/ou méta, et/ou para, par un, ou plusieurs, groupe P et/ou $P_1$, P et $P_1$ ayant les significations indiquées ci-dessus,
  * un groupe CN,
  * un groupe

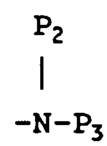

dans lequel $P_2$ et $P_3$, identiques ou différents, représentent un atome d'hydrogène ou le groupe P ou $P_1$, P et $P_1$ ayant les significations indiquées ci-dessus,

. W et Z, identiques ou différents, représentent respectivement les groupes indiqués ci-dessus pour X et Y lorsque $x_1$ et $x_2$ valent respectivement zéro, ou encore un cycle aromatique de 6 à 14 atomes de carbone, le cas échéant substitué par un, ou plusieurs, groupe P et/ou $P_1$, P et $P_1$ ayant les significations indiquées ci-dessus, à l'exclusion des dérivés de formule

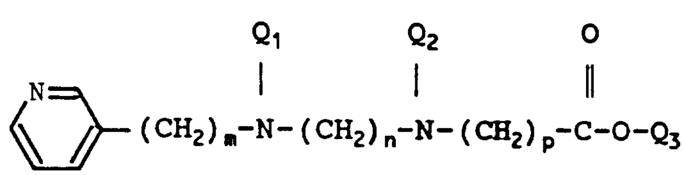

dans laquelle :

- m et p sont des nombres entiers variant de 1 à 4,
- n est un nombre entier variant de 2 à 4,
- $Q_1$ et $Q_2$, identiques ou différents, représentent un atome d'hydrogène ou un groupe alcoyle de 1 à 4 atomes de carbone,
- $Q_3$ représente un atome d'hydrogène, un groupe alcoyle de 1 à 4 atomes de carbone, ou un groupe phényle ou phényle substitué avec un, deux, ou trois substituants choisis indépendamment entre le chlore et un groupe alcoyle de 1 à 3 atomes de carbone ;

b) lorsque $y = 1$,

. $x_1$ et $x_2$ valent 1

. D a la même signification que A, B et C définis ci-dessus,

. W, X, Y et Z, identiques ou différents, représentent respectivement :

* les groupes indiqués ci-dessus pour X et Y lorsque $x_1$ et $x_2$ valent respectivement 1, ou

* un cycle aromatique de 6 à 14 atomes de carbone dont 2 atomes de carbone sont engagés respectivement dans une liaison avec A et D dans le cas de W, ou avec B et A dans le cas de X, ou avec C et B dans le cas de Y, ou avec D et C dans le cas de Z, ce cycle aromatique étant le cas échéant substitué par un, ou plusieurs groupe P et/ou $P_1$, P et $P_1$ ayant les significations indiquées ci- dessus, ou

* un groupe du type

$$-(CH_2)_{x_3}-V-(CH_2)_{x_4}-$$

dans lequel $x_3$ et $x_4$ sont des nombres entiers variant de 1 à 3, et V représente un cycle aromatique de 6 à 14 atomes de carbone, le cas échéant substitué par un, ou plusieurs, groupe P et/ou $P_1$, P et $P_1$ ayant les significations indiquées ci-dessus,

* un groupe

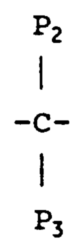

dans lequel $P_2$ et $P_3$ ont les significations indiquées ci-dessus, étant entendu que, aussi bien lorsque y = 0 ou 1, les dérivés polyazotés correspondant comportent 4 ou 5 fonctions coordinantes dont une est représentée par le groupe -COR , R ayant la signification indiquée ci-dessus, les autres fonctions coordinantes étant des groupes azotés choisis parmi:

- les groupes

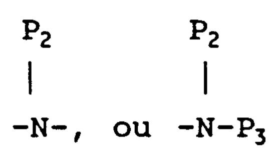

dans lesquels $P_2$ et $P_3$ ont les significations indiquées ci-dessus,

- les hétérocycles aromatiques azotés de 4 à 12 atomes de carbone, le cas échéant substitués par un, ou plusieurs, groupe P et/ou $P_1$ tels que définis ci-dessus,

sous réserve que lorsque les dérivés polyazotés comportent 4 fonctions coordinantes,

* soit les trois groupes azotés sont des groupes

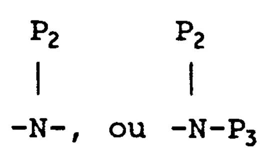

tels que définis ci-dessus, les dérivés polyazotés correspondant comportant alors un cycle aromatique de 6 à 14 atomes de carbone, le cas échéant substitué par un, ou plusieurs, groupe P et/ou $P_1$ tels que définis ci-dessus,

* soit un au moins parmi les trois groupes azotés représente un hétérocycle aromatique azoté, le cas échéant substitué par un, ou plusieurs, groupe P et/ou $P_1$ tels que définis ci-dessus,

les dérivés de formule $\alpha$ :

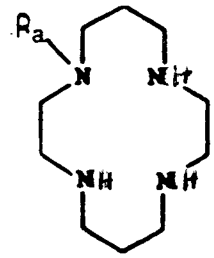

dans laquelle $R_a$ représente :

. un groupe $-(CH_2)_n-COOH$ dans lequel n est un nombre entier variant de 1 à 3.

. ou un groupe

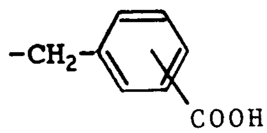 ,

les dérivés de formule $\beta$ tels que définis à la revendication 3,
étant exclus.

**2.** Dérivés polyazotés selon la revendication 1, caractérisés en ce qu'ils ne comportent qu'un seul groupe -COR, R ayant la signification indiquée dans la revendication 1.

**3.** Dérivés selon l'une des revendications 1 et 2, à l'exclusion des dérivés de formule $\beta$:

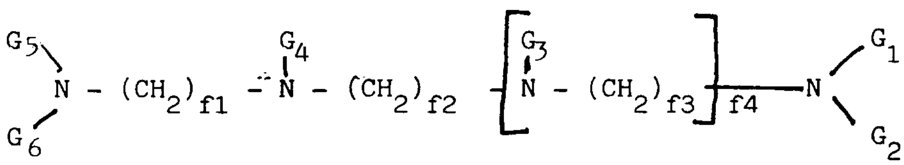 $\beta$

dans laquelle :

- $G_1$, $G_2$, $G_3$, $G_4$, et $G_5$ identiques ou différents, représentent H, ortho-$CH_2C_6H_4OH$, $-CH_2-COOH$ ou

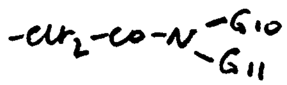 ;

- $G_6$ représente H, ou ortho-$CH_2C_6H_4OH$,
  ou un reste

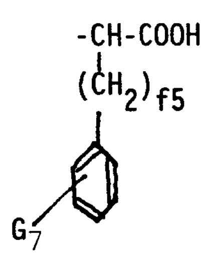

$G_7$ représentant $NO_2$, $NH_2$,

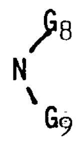

$G_8$ et $G_9$, identiques ou différents, étant H ou un radical alkyle en $C_1$-$C_4$, le maléimide ou une halotriazine substituée,

sous réserve que lorsque $G_6$ est H, l'un de $G_1$, $G_2$, $G_3$, $G_4$ ou $G_5$ est
$-CH_2-COOH$,

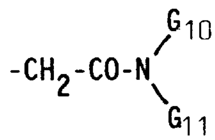

$G_{10}$ et $G_{11}$ identiques ou différents, représentant H ou un radical alkyle en $C_1$-$C_4$ ;

- $f_1$, $f_2$ et $f_3$ représentent 2 ou 3 ;
- $f_4$ représente 0 ou 1 ;
- $f_5$ représente un entier compris entre 0 et 10.

**4.** Dérivés polyazotés selon l'une des revendications 1 à 3, correspondant à la formule suivante :

$(W-A)_{x1}-X-B-Y-(C-Z)_{x2}$ (Ia),

dans laquelle W, A, X, B, Y, C, Z, $x_1$ et $x_2$ ont les significations indiquées dans la revendication 1 lorsque y = 0.

**5.** Dérivés selon la revendication 4, caractérisés en ce que $x_1 = x_2 = 1$ et correspondant à la formule suivante :

W-A-X-B-Y-C-Z (Ib)

**6.** Dérivés selon la revendication 4, caractérisés en ce que $x_1 = 0$ et correspondant à la formule suivante :

X-B-Y-C-Z (Ic)

**7.** Dérivés selon la revendication 4, caractérisés en ce que $x_2 = 0$ et correspondant à la formule suivante:

W-A-X-B-Y (Id)

**8.** Dérivés selon l'une quelconque des revendications 4 à 7, caractérisés en ce qu'ils comportent 4 fonctions coordinantes.

**9.** Dérivés selon la revendication 8, correspondant à la formule Ib, caractérisés en ce que l'un de W ou de Z représente le groupe -COR ,R ayant la signification indiquée dans la revendication 1, tandis que l'autre de W ou de Z, ainsi que X et Y, représentent respectivement un groupe azoté,

de préférence :

- l'un de W ou de Z représente :
  - . soit un groupe pyridyle, pyrimidyle ou imidazolyle
  - . soit un groupe aminobenzyle
- X et Y représentent respectivement un groupe -NH- ,
- A,B et C représentent respectivement un groupe $-(CH_2)_x-$ , x étant un nombre entier variant de 1 à 4.

**10.** Dérivés selon la revendication 8, correspondant à la formule Ic, caractérisés en ce que :
- l'un de X ou de Z représente le groupe -COR , R ayant la signification indiquée dans la revendication 1, tandis que l'autre de X ou de Z ainsi que Y, représentent un groupe azoté, et
- l'un de B, ou de C, ou de Y est substitué par un groupe alcoyle de 1 à 4 atomes de carbone substitué par un groupe azoté, de préférence :
- l'un de X ou de Z représente :
  . soit un groupe pyridyle, pyrimidyle ou imidazolyle,
  . soit un groupe aminobenzyle,
  . soit une amine primaire $-NH_2$,
- Y représente un groupe

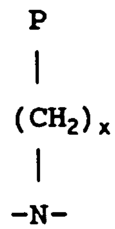

dans lequel x représente un nombre entier de 1 à 4, et P représente :
  . soit un groupe pyridyle, pyrimidyle ou imidazolyle,
  . soit un groupe aminobenzyle,
  . soit une amine primaire $-NH_2$,
- B et C représentent respectivement un groupe $-(CH_2)_x-$, x étant défini ci-dessus.

**11.** Dérivés selon la revendication 8, correspondant à la formule Id, caractérisés en ce que :
- l'un de W ou de Y représente le groupe -COR tel que défini ci-dessus, tandis que l'autre de W ou de Y, ainsi que X, représentent un groupe azoté, et
- l'un de A, ou de B, ou de X est substitué par un groupe alcoyle de 1 à 4 atomes de carbone substitué par un groupe azoté,
  de préférence :
- l'un de W ou de Y représente.
  . soit un groupe pyridyle, pyrimidyle ou imidazolyle,
  . soit un groupe aminobenzyle,
  . soit une amine primaire $-NH_2$,
- X représente un groupe

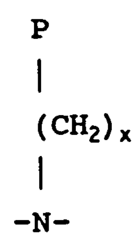

dans lequel x représente un nombre entier variant de 1 à 4 et P représente :
  . soit un groupe pyridyle, pyrimidyle ou imidazolyle,
  . soit un groupe aminobenzyle,
  . soit une amine primaire $-NH_2$
- A et B représentent respectivement un groupe $-(CH_2)-_x$, x étant défini ci-dessus.

**12.** Dérivés selon l'une quelconque des revendications 4 à 7, caractérisés en ce qu'ils comportent 5 fonctions coordinantes.

**13.** Dérivés selon la revendication 12, correspondant à la formule Ib, caractérisés en ce que:
  a)

- W, X, Y, et Z représentent un groupe azoté,
- l'un de W ou de Z est substitué par un groupe alcoyle de 1 à 4 atomes de carbone substitué par un groupe -COR, R ayant la signification indiquée dans la revendication 1,
  de préférence :
- l'un de W ou de Z représente :
  . soit un groupe pyridyle, pyrimidyle ou imidazolyle,
  . soit un groupe aminobenzyle,
  . soit une amine primaire $-NH_2$,
- tandis que l'autre de W ou de Z représente un groupe $-NH-(CH_2)_x-COR$ dans lequel R et x ont les significations indiquées dans la revendication 1,
- X et Y représentent respectivement un groupe -NH- ,
- A, B et C représentent respectivement un groupe $-(CH_2)-_x$, x étant défini ci-dessus,
  ou encore,

b)
- l'un de W ou de Z représente le groupe -COR, R ayant la signification indiquée dans la revendication 1,
- tandis que l'autre de W et de Z, ainsi que X et Y représentent un groupe azoté, et
- l'un de A, X, B, Y, ou C est substitué par un groupe alcoyle de 1 à 4 atomes de carbone substitué par un groupe azoté, de préférence :
- l'un de W ou de Z représente :
  . soit un groupe pyridyle, pyrimidyle ou imidazolyle,
  . soit un groupe aminobenzyle,
  . soit une amine primaire $-NH_2$,
- l'un de X ou de Y représente -NH- tandis que - l'autre de X et de Y représente

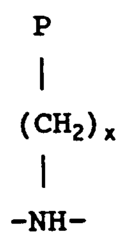

dans lequel x représente un nombre entier variant de 1 à 4 et P représente :
  . soit un groupe pyridyle, pyrimidyle ou imidazolyle,
  . soit un groupe aminobenzyle,
  . soit une amine primaire $-NH_2$,
- A, B et C représentent respectivement $-(CH_2)_x-$,x étant défini ci-dessus.

**14.** Dérivés selon l'une des revendications 1 et 2, correspondant à la formule générale suivante :

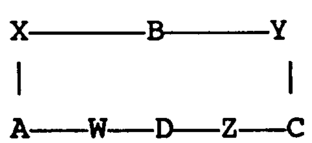

(IIa)

dans laquelle W, A, X, B, Y, C, Z et D ont les significations indiquées dans la revendication 1 lorsque y = 1.

**15.** Dérivés selon la revendication 14, caractérisés en ce qu'ils comportent 5 fonctions coordinantes.

**16.** Dérivés selon la revendication 15, caractérisés en ce que :
- W, X, Y et Z représentent respectivement un groupe azoté,
- l'un de W, X, Y ou Z étant substitué par un groupe alcoyle de 1 à 4 atomes de carbone lui-même substitué par le groupe -COR, R ayant la signification indiquée dans la revendication 1, de préférence :

- W, X, Y et Z représentent respectivement
  - * soit un groupe pyridyle, pyrimidyle ou imidazolyle, le cas échéant substitué par un groupe alcoyle de 1 à 4 atomes de carbone lui-même substitué par le groupe -COR, R ayant la signification indiquée dans la revendication 1,
  - * soit un groupe -NH-, ou

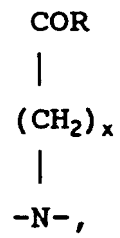

x et R ayant les significations indiquées dans la revendication 1.

**17.** Méthode de préparation des dérivés selon l'une quelconque des revendications 1 à 16, caractérisée en ce qu'elle comprend la mise en oeuvre de l'une au moins des étapes de condensation suivantes, à savoir :

- la réaction d'un halogénure du type $R_1$-Z dans lequel Z représente un halogène,notamment du chlore ou du brome, et $R_1$ correspond à une partie du dérivé à synthétiser et est choisi parmi les groupes correspondant aux entités W-A-,

$$(W\text{-}A)_{x_1}\text{-}X\text{-}B\text{-},\quad (W\text{-}A)_{x_1}\text{-}X\text{-}B\text{-}Y\text{-}C\text{-},$$

ou -A-X-B-Y-C-Z,

$$\text{-}B\text{-}Y\text{-}(C\text{-}Z)_{x_2}$$

ou -C-Z avec amine primaire 2 $H_2N$-$R_2$ ou une amine secondaire 3

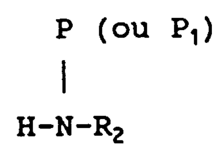

selon le schéma :

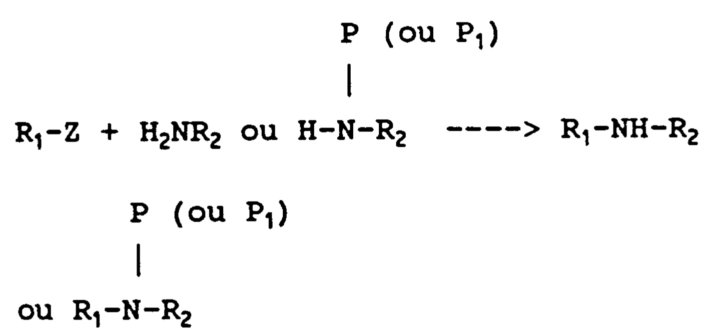


les dérivés d'amine 2 et 3 sus-mentionnés étant alors respectivement choisis parmi les groupes dans lesquels -NH-$R_2$, ou

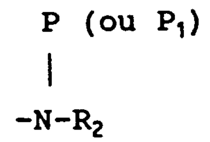

correspondent aux entités

$$-X-B-Y-(C-Z)_{x_2}, \quad -Y-(C-Z)_{x_2},$$

-Z ou W-,

$$(WA)_{x_1}-X-, \quad \text{et} \quad (W-A)_{x_1}-X-B-Y-,$$

dans lesquels W, A, Y, B, X, C, Z, P, $P_1$, $x_1$ et $x_2$ ont les significations indiquées dans la revendication 1 lorsque y = 0,

- la réaction d' un aldéhyde 4 de formule $R_1$-CHO dans laquelle $R_1$ a la signification indiquée ci-dessus, avec une amine primaire 2 telle qu'indiquée ci-dessus, suivie d'une étape de réduction, selon le schéma

$R_1$-CHO + $H_2$N-$R_2$ ---> $R_1$-CH = N-$R_2$ ---> $R_1$-$CH_2$-NH-$R_2$.

- la réaction de l'acrylonitrile avec le dérivé d'amine 2 ou 3, ce qui conduit à la formation d'une chaîne terminée par un groupe nitrile, suivie d'une étape d'hydrolyse pour transformer le groupe CN en un groupe -COOH, puis le cas échéant une étape de fonctionnalisation pour obtenir un groupe -COR,
- la réaction d'un halogénure de formule $R_1$-Z sus-mentionnée dans laquelle $R_1$ représente W-A-, W-A-X-B, X-B-, -B-Y, -B-Y-C-Z, avec une pipérazinone, suivie d'une réaction d'hydrolyse de la fonction amide, selon le schéma :

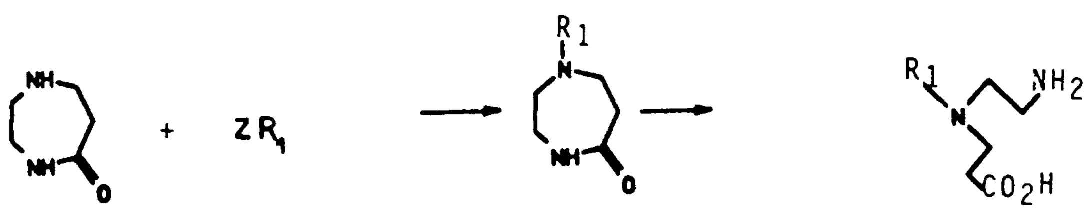

dans le cas d'une monocondensation,
ou selon le schéma

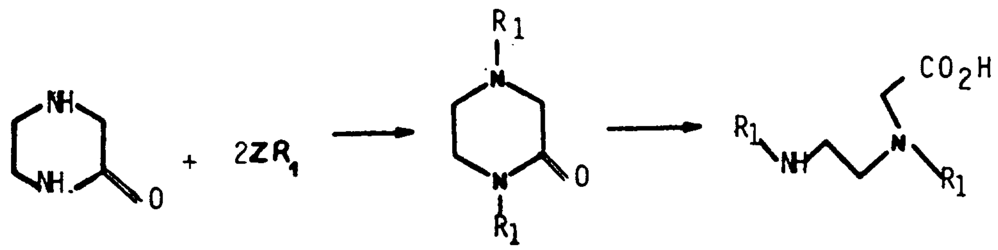

dans le cas d'une dicondensation,

- la condensation d'un dérivé 5 portant deux fonctions amine primaire, cette diamine 5 correspondant à la formule $H_2N-T_1-R_9-T_2-NH_2$, avec un dialdéhyde 6, correspondant à la formule $OHC-T_1-R_{10}-T_2-CHO$, $T_1$ et $T_2$,identiques ou différents, représentant une chaîne alcoylène $-(CH_2)_x-$, dans

laquelle x représente un nombre entier de 1 à 3, le cas échéant substituée par un, ou plusieurs, groupe P, ou par un, ou plusieurs, groupe $P_1$, P et $P_1$ étant tels que définis ci-dessus, $R_9$ et $R_{10}$, identiques ou différents, ont les significations indiquées dans la revendication 1 pour X ou Y lorsque y = 1, suivie d'une étape de réduction du produit cyclisé résultant de la condensation précédente.

**18.** Complexes métalliques caractérisés en ce qu'ils sont constitués d'un atome de métal, notamment de Co, Ni, Fe, Mn, Cu, et d'un dérivé selon l'une quelconque des revendications 1 à 16, y compris les dérivés de formule $\alpha$.

**19.** Méthode de préparation des complexes selon la revendication 18 caractérisée en ce qu'elle comprend la réaction entre le métal sous forme de sel, notamment sous forme d'acétate, de nitrate, ou d'halogénures, avec un dérivé selon l'une quelconque des revendications 1 à 16.

**20.** Procédé de séparation ou de production d'oxygène à partir d'un mélange de gaz le contenant, caractérisé en ce qu'il comprend :

- la mise en contact du mélange de gaz avec un complexe métallique selon la revendication 18, dans des conditions permettant l'absorption de l'oxygène du mélange de gaz, ce qui conduit à la formation d'un dimère, qui, dans sa forme neutre, répond à la formule $XO_2X$ dans laquelle X représente une molécule de complexe métallique,
- la désorption de l'oxygène fixé dans le dimère et
- la récupération de l'oxygène désorbé,

le complexe métallique étant de préférence en solution aqueuse ou partiellement aqueuse à une concentration de 0,1M à 1M à un pH de 6 à 8, ou en variante, étant

en solution immobilisée sur une membrane perméable à l'oxygène, cette membrane comprenant un support formé d'un film polymère non perméable aux liquides dont l'une des faces est en contact avec le mélange gazeux renfermant l'oxygène, la désorption de l'oxygène fixé par le complexe métallique étant effectuée par l'autre face,

la désorption étant réalisée de préférence par désorption électrochimique ou par le vide, en particulier par abaissement de la pression partielle de l'oxygène dans l'atmosphère environnant la solution, lorsque le complexe métallique est réalisé en solution aqueuse ou partiellement aqueuse, et/ou en appliquant une différence de température de part et d'autre d'une membrane lorsque le complexe métallique est en solution immobilisée sur une membrane.

**21.** Dimère de formule $XO_2X$ dans lequel X représente un complexe métallique selon la revendication 18.

## Claims

**1.** Polynitrogenous compounds corresponding to the following general formula :

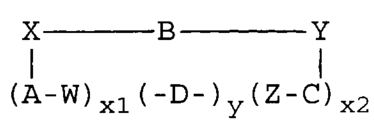

(I)

in which y = 0 or 1,

a) when y = 0 :

. $x_1$ and $x_2$ are equal to 0 or 1, $x_1$ and $x_2$ not being able to equal 0 simultaneously ;

. the constituents A, B and C, identical or different, represent :

- an alkylene chain $-(CH_2)_x-$, in which x represents a whole number from 1 to 4, substituted if necessary by one or more P groups, or by one or more $P_1$ groups, $P_1$ representing an alkyl group having 1 to 4 carbon atoms, substituted if necessary by one or more P groups, P representing :

  * a -COR group in which R represents a hydroxyl (-OH), a primary amine, or an amine substituted by one or two alkyl groups having 1 to 4 carbon atoms, an -OR' functional group in which R' represents either an alkyl group having 1 to 4 carbon atoms, or an aromatic ring having 6 to 14 carbon atoms substituted if necessary in the ortho and/or

meta and/or para position by a halogen atom, an alkyl group having 1 to 4 carbon atoms, an alkoxy group having 1 to 4 carbon atoms, hydroxy, an aryl group, an aromatic heterocyclic ring, a nitro group, a $-(CH_2)_t$-COR group in which R has the meaning given above, and t represents a whole number from 0 to 4, or a primary amine or an amine substituted by one or two alkyl groups having 1 to 4 carbon atoms ;

* an aromatic ring having 6 to 14 carbon atoms, substituted if necessary in the ortho and/or meta and/or para position by a halogen atom, an alkyl group having 1 to 4 carbon atoms, an alkoxy group having 1 to 4 carbon atoms, hydroxy, an aryl group, an aromatic heterocyclic ring, a nitro group, a $-(CH_2)_t$-COR group in which R has the meaning given above, and t represents a whole number from 0 to 4, or a primary amine or an amine substituted by one or two alkyl groups having 1 to 4 carbon atoms ;
* an aromatic heterocyclic ring, in particular containing nitrogen, having 4 to 12 carbon atoms, substituted if necessary in the ortho and/or meta and/or para position by a halogen atom, an alkyl group having 1 to 4 carbon atoms, an alkoxy group having 1 to 4 carbon atoms, hydroxy, an aromatic heterocyclic ring, an aryl group, a nitro group, a $-(CH_2)_t$-COR group in which R has the meaning given above, and t represents a whole number from 0 to 4, or a primary amine or an amine substituted by one or two alkyl groups having 1 to 4 carbon atoms,
* a primary amine group or an amine group substituted by one or two alkyl groups with 1 to 4 carbon atoms,
* a CN group,
* an alkyl group having 1 to 4 carbon atoms,
* an alkoxy group having 1 to 4 carbon atoms,
* a hydroxyl functional group,
* a nitro group,
* a halogen atom,

- an aromatic ring having 6 to 14 carbon atoms, 2 carbon atoms of which are engaged respectively in a bond with W and X in the case of A, or with X and Y in the case of B, or with Y and Z in the case of C, this aromatic ring being if necessary substituted by one or more P and/or $P_1$ groups, P and $P_1$ having the meanings given above,
- a group of the type $-(CH_2)_{x3}-V-(CH_2)_{x4}-$ in which x3 and x4 are whole numbers varying from 1 to 3, and V represents an aromatic ring having 6 to 14 carbon atoms, substituted if necessary by one or more P and/or $P_1$ groups, P and $P_1$ having the meanings given above,

. the constituents X and Y, identical or different, represent respectively :

- when $x_1$ and $x_2$ are respectively equal to 1 :
  * a

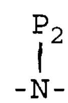

group in which $P_2$ represents a hydrogen atom or the P or $P_1$ group, P and $P_1$ having the meanings given above,

  * a nitrogen-containing aromatic heterocyclic ring, having 4 to 12 carbon atoms, two carbon atoms of which are engaged respectively in a bond with A and B in the case of X, or with B and C in the case of Y, this heterocyclic ring being if necessary substituted by one or more P and/or $P_1$ groups, P and $P_1$ having the meanings given above,
- when $x_1 = 0$ or when $x_2 = 0$ : the end substituent Y or X represents respectively
  * a -COR group in which R has the meaning given above,
  * a nitrogen-containing aromatic heterocyclic ring, having 4 to 12 carbon atoms, if necessary substituted in the ortho and/or meta and/or para position by one or more P and/or $P_1$ groups, P and $P_1$ having the meanings given above,
  * a CN group,

* a

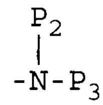

group in which $P_2$ and $P_3$, identical or different, represent a hydrogen atom or the P or $P_1$ group, P and $P_1$ having the meanings given above,

. W and Z, identical or different, represent respectively the groups indicated above for X and Y when $x_1$ and $x_2$ are respectively equal to zero, or an aromatic ring having 6 to 14 carbon atoms, substituted if necessary by one or more P and/or $P_1$ groups, P and $P_1$ having the meanings given above, with the exception of derivatives having the formula

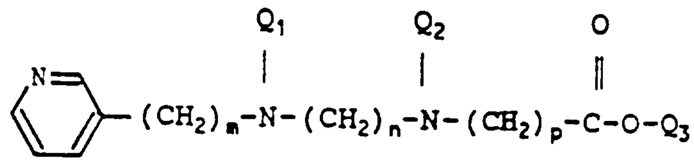

in which ;

- m and p are whole numbers varying from 1 to 4,
- n is a whole number varying from 2 to 4,
- $Q_1$ and $Q_2$, identical or different, represent a hydrogen atom or an alkyl group having 1 to 4 carbon atoms,
- $Q_3$ represents a hydrogen atom, an alkyl group having 1 to 4 carbon atoms or a phenyl group or a phenyl group substituted with one, two or three substituents selected independently from chlorine and an alkyl group having 1 to 3 carbon atoms ;

b) when y = 1,

. $x_1$ and $x_2$ equal 1,

. D has the same meaning as A, B and C defined above,

. W, X, Y and Z, identical or different, represent respectively :

* the groups given above for X and Y when $x_1$ and $x_2$ are respectively equal to 1, or

* an aromatic ring having 6 to 14 carbon atoms, 2 carbon atoms of which are engaged respectively in a bond with A and D in the case of W, or with B and A in the case of X, or with C and B in the case of Y, or with D and C in the case of Z, this aromatic ring being if necessary substituted by one or more P and/or $P_1$ groups, P and $P_1$ having the meanings given above, or

* a group of the type $-(CH_2)_{x3}-V-(CH_2)_{x4}-$ in which x3 and x4 are whole numbers varying from 1 to 3, and V represents an aromatic ring having 6 to 14 carbon atoms, substituted if necessary by one or more P and/or $P_1$ groups, P and $P_1$ having the meanings given above,

* a

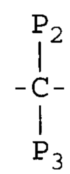

group in which $P_2$ and $P_3$ have the meanings given above, it being understood that, moreover when y = 0 or 1, the corresponding polynitrogenous derivatives contain 4 or 5 coordinating functional groups one of which is represented by the -COR group, R having the meaning given above, the other coordinating functional groups being nitrogen-containing groups selected from :

-

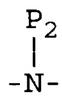 or 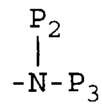

groups in which $P_2$ and $P_3$ have the meanings given above,

- nitrogen-containing aromatic heterocyclic rings having 4 to 12 carbon atoms, if necessary substituted by one or more P and/or $P_1$ groups such as defined above, except that when the polynitrogenous derivatives contain 4 coordinating functional groups,

* either the three nitrogen-containing groups are

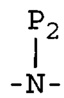 or 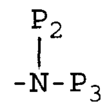

groups such as defined above, the corresponding polynitrogenous derivatives then having an aromatic ring with 6 to 14 carbon atoms, if necessary substituted by one or more P and/or $P_1$ groups such as defined above,

* or at least one of the three nitrogen-containing groups represents a nitrogen-containing aromatic heterocyclic ring, if necessary substituted by one or more P and/or $P_1$ groups, such as defined above,

derivatives having the formula $\alpha$ :

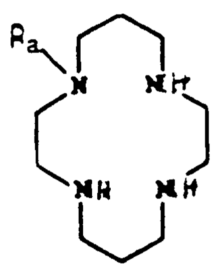

in which $R_a$ represents :

. a $-(CH_2)_n-COOH$ group in which n is a whole number varying from 1 to 3.

. or a

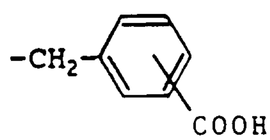

group,

derivatives of formula $\beta$, such as defined in Claim 3 being excluded.

**2.** Polynitrogenous derivatives according to claim 1, characterized in that they have only a single -COR group, R having the meaning given in claim 1.

**3.** Derivatives according to either of claims 1 and 2, with the exception of the derivatives of formula $\beta$ :

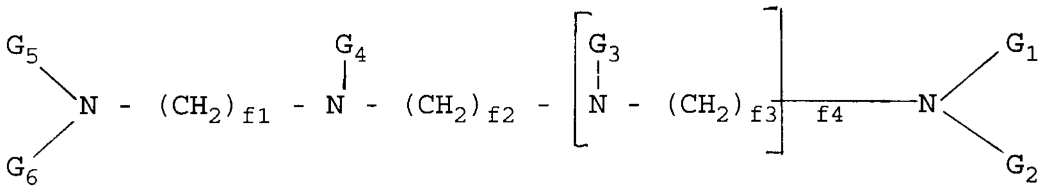

$\beta$

in which :

- $G_1$, $G_2$, $G_3$, $G_4$ and $G_5$, identical or different, represent H, ortho-$CH_2C_6H_4OH$, $-CH_2-COOH$ or

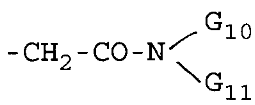

;

- $G_6$ represents H or ortho-$CH_2C_6H_4OH$,
  or a

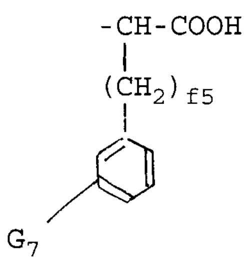

residue,
$G_7$ representing $NO_2$, $NH_2$,

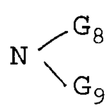

$G_8$ and $G_9$, identical or different, being H or an alkyl radical having $C_1$ - $C_4$, maleimide or a substituted halotriazine,
except that when $G_6$ is H, one of $G_1$, $G_2$, $G_3$, $G_4$ or $G_5$ is
$-CH_2-COOH$,

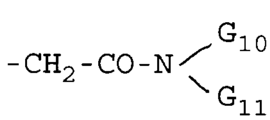

$G_{10}$ and $G_{11}$, identical or different, representing H or an alkyl radical having $C_1$ - $C_4$ ;

- $f_1$, $f_2$ and $f_3$ represent 2 or 3 ;
- $f_4$ represents 0 or 1 ;
- $f_5$ represents a whole number of between 0 and 10.

**4.** Polynitrogenous derivatives according to one of claims 1 to 3, corresponding to the following formula :

$(W-A)_{x1}-X-B-Y-(C-Z)_{x2}$ (Ia),

in which W, A, X, B, Y, C, Z, $x_1$ and $x_2$ have the meanings indicated above in claim 1 when $y = 0$.

**5.** Derivatives according to claim 4, characterized in that $x_1 = x_2 = 1$ and that they correspond to the following formula :

W-A-X-B-Y-C-Z (Ib)

**6.** Derivatives according to claim 4, characterized in that $x_1 = 0$ and that they correspond to the following formula :

X-B-Y-C-Z (Ic)

**7.** Derivatives according to claim 4, characterized in that $x_2 = 0$ and that they correspond to the following formula :

W-A-X-B-Y (Id)

**8.** Derivatives according to any one of claims 4 to 7, characterized in that they have 4 coordinating functional groups.

**9.** Derivatives according to claim 8, corresponding to formula Ib, characterized in that either W or Z represents the -COR group, R having the meaning given in Claim 1, while the other one of W or Z, as well as X and Y, represent respectively a nitrogen-containing group,

preferably :

- either W or Z represents :
  - . either a pyridyl, pyrimidyl or imidazolyl group
  - . or an aminobenzyl group
- X and Y represent respectively an -NH- group,
- A, B and C represent respectively a $-(CH_2)_x-$ group, x being a whole number varying between 1 and 4.

**10.** Derivatives according to claim 8, corresponding to formula Ic, characterized in that :

- either X or Z represents the -COR group, R having the meaning indicated in claim 1, while the other one of X or Z as well as Y, represent a nitrogen-containing group, and
- one of B or C or Y is substituted by an alkyl group having 1 to 4 carbon atoms, substituted by a nitrogen-containing group,

preferably :

- either X or Z represents :
  - . either a pyridyl, pyrimidyl or imidazolyl group
  - . or an aminobenzyl group
  - . or a primary amine $-NH_2$,
- Y represents a

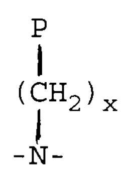

group in which x represents a whole number of 1 to 4, and P represents :

  - . either a pyridyl, pyrimidyl or imidazolyl group
  - . or an aminobenzyl group
  - . or a primary amine $-NH_2$,
- B and C represent respectively a $-(CH_2)_x-$ group, x being defined above.

**11.** Derivatives according to claim 8, corresponding to formula Id, characterized in that :

- either W or Y represent the -COR group as defined above, while the other one of W or Y, as well as X, represent a nitrogen-containing group, and
- one of A or B or X is substituted by an alkyl group having 1 to 4 carbon atoms, substituted by a nitrogen containing group,

  preferably :
- either W or Y represents :
  . either a pyridyl, pyrimidyl or imidazolyl group
  . or an aminobenzyl group
  . or a primary amine $-NH_2$,
- X represents a

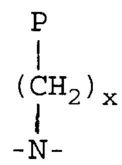

  group in which x represents a whole number varying from 1 to 4, and P represents :
  . either a pyridyl, pyrimidyl or imidazolyl group
  . or an aminobenzyl group
  . or a primary amine $-NH_2$,
- A and B represent respectively a $-(CH_2)_x-$ group, x being defined above.

**12.** Derivatives according to any one of claims 4 to 7, characterized in that they have 5 coordinating functional groups.

**13.** Derivatives according to claim 12, corresponding to formula Ib, characterized in that :

a)
  - W, X, Y and Z represent a nitrogen-containing group,
  - either W or Z is substituted by an alkyl group having 1 to 4 carbon atoms substituted by a -COR group, R having the meaning given in claim 1,

    preferably :
  - either W or Z represents :
    . either a pyridyl, pyrimidyl or imidazolyl group
    . or an aminobenzyl group
    . or a primary amine $-NH_2$,
  - while the other one of W or Z represents a $-NH-(CH_2)_x-COR$ group in which R and x have the meanings given in claim 1,
  - X and Y represent respectively an -NH- group,
  - A, B and C represent respectively a $-(CH_2)_x$ group, x being defined above,

    or,

b)
  - either W or Z represents the -COR group, R having the meaning given in claim 1,
  - while the other one of W or Z, as well as X and Y represent a nitrogen-containing group,
  - one of A, X, B, Y or C is substituted by an alkyl group having 1 to 4 carbon atoms substituted by a nitrogen-containing group.

    preferably :
  - either W or Z represents :
    . either a pyridyl, pyrimidyl or imidazolyl group
    . or an aminobenzyl group
    . or a primary amine $-NH_2$,
  - either X or Y represents -NH- while

- the other one of X and Y represents

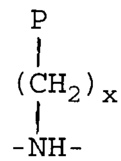

in which x represents a whole number varying between 1 and 4 and P represents :

. either a pyridyl, pyrimidyl or imidazolyl group

. or an aminobenzyl group

. or a primary amine $-NH_2$,

- A, B, and C represent respectively $-(CH_2)_x-$, x being defined above.

**14.** Derivatives according to either of claims 1 and 2, corresponding to the following general formula :

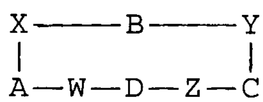

(IIa)

in which W, A, X, B, Y, C, Z and D have the meanings given in claim 1 when y = 1.

**15.** Derivatives according to claim 14, characterized in that they include 5 coordinating functional groups.

**16.** Derivatives according to Claim 15, characterized in that :

- W, X, Y and Z represent respectively a nitrogen-containing group,
- one of W, X, Y or Z being substituted by an alkyl group having 1 to 4 carbon atoms itself substituted by the -COR group, R having the meaning given in Claim 1,

  preferably :
- W, X, Y and Z represent respectively
  * either a pyridyl, pyrimidyl or imidazolyl group, if necessary substituted by an alkyl group having 1 to 4 carbon atoms itself substituted by the -COR group, R having the meaning given in claim 1,
- or an -NH- group, or

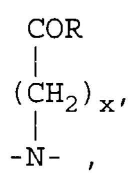

x and R having the meanings given in claim 1.

**17.** Method for the preparation of derivatives according to any one of claims 1 to 16, characterized in that it comprises putting into operation at least one of the following condensation stages, that is :

- reacting a halide of the type $R_1$-Z in which Z represents a halogen, particularly chlorine or bromine, and $R_1$ corresponds to part of the derivative to be synthesised and is selected from among the groups corresponding to the entities W-A-, $(W-A)_{x1}$-X-B-, $(W-A)_{x1}$-X-B-Y-C-, or -A-X-B-Y-C-Z, -B-Y-$(C-Z)_{x2}$ or -C-Z with primary amine <u>2</u> $H_2N-R_2$ or a secondary amine <u>3</u>

$$\begin{array}{c} P \text{ (or } P_1) \\ | \\ H\text{-}N\text{-}R_2 \end{array}$$

according to the equation :

$$R_1\text{-}Z + H_2NR_2 \text{ or } \begin{array}{c} P \text{ (or } P_1) \\ | \\ H\text{-}N\text{-}R_2 \end{array} \longrightarrow R_1\text{-}NH\text{-}R_2 \text{ or } \begin{array}{c} P \text{ (or } P_1) \\ | \\ R_1\text{-}N\text{-}R_2 \end{array}$$

the above-mentioned amine derivatives 2 and 3 being then selected respectively from among groups in which
$-NH-R_2$, or

$$\begin{array}{c} P \text{ (or } P_1) \\ | \\ -N\text{-}R_2 \end{array}$$

correspond to the entities $-X-B-Y-(C-Z)_{x2}$, $-Y-(C-Z)_{x2}$, -Z or W-, $(WA)_{x1}-X-$, and $(W-A)_{x1}-X-B-Y-$, in which W, A, Y, B, X, C, Z, P, $P_1$, $x_1$ and $x_2$ have the meanings given in claim 1, when y = 0.

- reacting an aldehyde 4 of formula $R_1-CHO$ in which $R_1$ has the meaning given above, with a primary amine 2 such as given above, followed by a reduction stage, according to the equation

  $R_1-CHO + H_2N-R_2 \rightarrow R_1-CH = N-R_2 \rightarrow R_1-CH_2-NH-R_2$.

- reacting acrylonitrile with the amine derivative 2 or 3, which leads to the formation of a chain terminated by a nitrile group, followed by a hydrolysis stage to convert the CN group into a -COOH group, and then if necessary a functionalization stage to obtain a -COR group,
- reacting a halide of formula $R_1-Z$ mentioned above in which $R_1$ represents W-A-, W-A-X-B, X-B-, -B-Y, -B-Y-C-Z, with a piperazinone, followed by a reaction in which the amide functional group is hydrolysed, according to the equation :

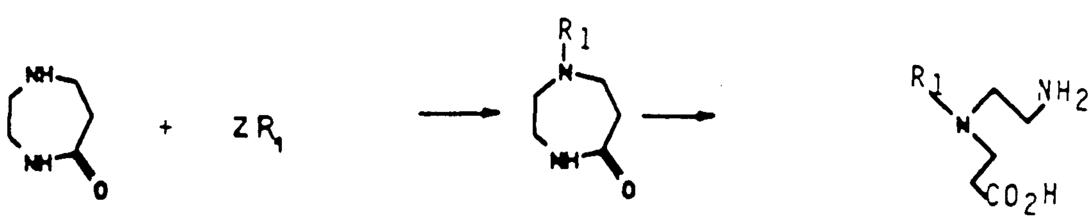

in the case of a monocondensation,
or according to the equation

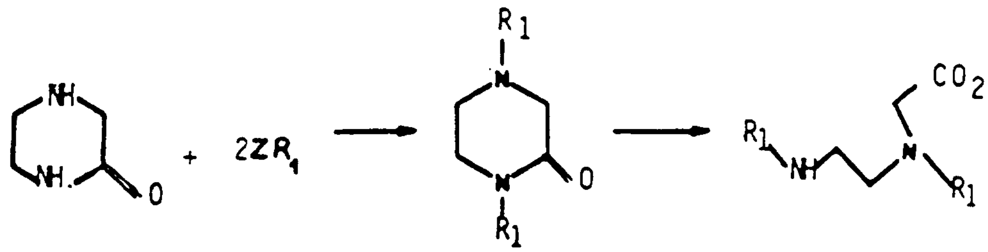

in the case of a dicondensation.

- condensing a derivative 5 carrying two primary amine functional groups, this diamine 5 corresponding to the formula $H_2N$-$T_1$-$R_9$-$T_2$-$NH_2$, with a dialdehyde 6, corresponding to the formula OHC-$T_1$-$R_{10}$-$T_2$-CHO, $T_1$ and $T_2$, identical or different, representing an alkylene chain $-(CH_2)_x-$, in which x represents a whole number from 1 to 3, substituted if necessary by one or more P groups, or by one or more $P_1$ groups, P and $P_1$ being such as defined above, $R_9$ and $R_{10}$, identical or different, have the meanings indicated in claim 1 for X or Y when y = 1, followed by a stage in which the cyclized product resulting from the preceding condensation is reduced.

**18.** Metal complexes characterized in that they consist of a metal atom, particularly Co, Ni, Fe, Mn and Cu, and a derivative according to any one of claims 1 to 16, including derivatives of formula $\alpha$.

**19.** Method for the preparation of complexes according to Claim 18, characterized in that it comprises reacting the metal, in salt form, particularly in the form of acetate, nitrate, or halides, with a derivative according to any one of Claims 1 to 16.

**20.** Process for the separation or production of oxygen from a gas mixture containing it, characterized in that it comprises :

- putting the gas mixture into contact with a metal complex according to Claim 18, under conditions enabling oxygen to be absorbed from the gas mixture, which leads to the formation of a dimer which, in its neutral form, corresponds to the formula $XO_2X$ in which X represents a metal complex molecule,
- desorbing the oxygen attached to the dimer
  and
- recovering the desorbed oxygen, the metal complex being preferably in aqueous or partially aqueous solution at a concentration of 0.1M to 1M at a pH of 6 to 8, or as a variant, being in solution immobilised on a membrane which is permeable to oxygen, this membrane comprising a support formed from a polymeric film not permeable to liquids one of the faces of which is in contact with the gaseous mixture containing oxygen, desorption of the oxygen fixed by the metal complex being carried out through the other face,
  desorption being preferably carried out by electrochemical desorption or by vacuum, in particular by lowering the partial pressure of oxygen in the atmosphere surrounding the solution, when the metal complex is made in aqueous or partially aqueous solution, and/or by applying a difference in temperature either side of a membrane when the metal complex is in solution immobilised on a membrane.

**21.** Dimer of formula $XO_2X$ in which X represents a metal complex according to claim 18.

## Patentansprüche

**1.** Polystickstoffderivate, die der folgenden allgemeinen Formel entsprechen:

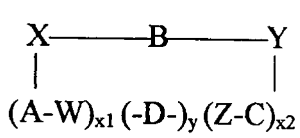

(I)

wobei y den Wert 0 oder 1 hat,

a) wenn y = 0:

- haben $x_1$ und $x_2$ den Wert 0 oder 1, wobei $x_1$ und $x_2$ nicht gleichzeitig den Wert 0 haben können;
- stehen die identischen oder unterschiedlichen Bestandteile A, B und C für:
  - eine Alkylenkette $-(CH_2)_x-$, in welcher x eine ganzzahlige Zahl zwischen 0 und 4 darstellt, gegebenenfalls substituiert durch eine oder mehrere P-Gruppen oder durch eine oder mehrere $P_1$-Gruppen, wobei $P_1$ eine Alkylgruppe mit 1 bis 4 Kohlenstoffatomen darstellt, gegebenenfalls durch eine oder mehrere P-Gruppen substituiert, wobei P darstellt:
    * eine [-COR]-Gruppe, in welcher R ein Hydroxyl (-OH), ein primäres oder durch eine oder zwei Alkylgruppen mit 1 bis 4 Kohlenstoffatomen substituiertes Amin, eine [-OR']-Funktion, in welcher R' entweder eine Alkylgruppe mit 1 bis 4 Kohlenstoffatomen oder einen aromatischen Ring mit 6 bis 14 Kohlenstoffatomen, gegebenenfalls in Ortho- und/oder Meta- und/oder Parastellung substituiert durch ein Halogenatom, eine Alkylgruppe mit 1 bis 4 Kohlenstoffatomen, eine Hydroxygruppe, eine Arylgruppe, einen aromatischen Heteroring, eine Nitrogruppe, eine $[-(CH_2)_t-COR]$-Gruppe, in der R die oben gegebene Bedeutung hat und t eine ganze Zahl zwischen 0 und 4 darstellt, oder ein primäres oder durch eine oder zwei Alkylgruppen mit 1 bis 4 Kohlenstoffatomen substituiertes Amin darstellt;
    * einen aromatischen Ring mit 6 bis 14 Kohlenstoffatomen, gegebenenfalls in Ortho- und/oder Meta- und/oder Parastellung substituiert durch ein Halogenatom, eine Alkylgruppe mit 1 bis 4 Kohlenstoffatomen, eine Alkoxygruppe mit 1 bis 4 Kohlenstoffatomen, eine Hydroxygruppe, eine Arylgruppe, einen aromatischen Heteroring, eine Nitrogruppe, eine $[-(CH_2)_t-COR]$-Gruppe, in der R die oben gegebene Bedeutung hat und t eine ganze Zahl zwischen 0 und 4 darstellt, oder ein primäres oder durch ein oder zwei Alkylgruppen mit 1 bis 4 Kohlenstoffatomen substituiertes Amin:
    * einen aromatischen, insbesondere stickstoffhaltigen, Heteroring mit 4 bis 12 Kohlenstoffatomen, gegebenenfalls in Ortho- und/oder Meta- und/oder Parastellung, substituiert durch ein Halogenatom, eine Alkylgruppe mit 1 bis 4 Kohlenstoffatomen, eine Alkoxygruppe mit 1 bis 4 Kohlenstoffatomen, eine Hydroxygruppe, einen aromatischen Heteroring, eine Arylgruppe, eine Nitrogruppe, eine $[-(CH_2)_t-COR]$-Gruppe, in welcher R die oben gegebene Bedeutung hat und t eine ganze Zahl zwischen 0 und 4 darstellt, oder ein primäres oder durch ein oder zwei Alkylgruppen mit 1 bis 4 Kohlenstoffatomen substituiertes Amin;
    * eine primäre oder durch ein oder zwei Alkylgruppen mit 1 bis 4 Kohlenstoffatomen substituierte Aminogruppe;
    * eine CN-Gruppe;
    * eine Alkylgruppe mit 1 bis 4 Kohlenstoffatomen;
    * eine Alkoxygruppe mit 1 bis 4 Kohlenstoffatomen;
    * eine Hydroxyl-Funktion;
    * eine Nitrogruppe;
    * ein Halogenatom;
  - einen aromatischen Ring mit 6 bis 14 Kohlenstoffatomen, von denen zwei Kohlenstoffatome jeweils in einer Bindung mit W und X im Falle von A oder mit X und Y im Falle von B oder mit Y und Z im Falle von C gebunden sind, wobei dieser aromatische Ring gegebenenfalls durch eine oder mehrere P- und/oder $P_1$-Gruppen substituiert ist, wobei P und $P_1$ die oben gegebenen Bedeutungen haben,
  - eine $-(CH_2)_{x3}-V-(CH_2)_{x4}$-Gruppe, in der $x_3$ und $x_4$ ganze Zahlen zwischen 1 und 3 sind und V einen aromatischen Ring mit 6 bis 14 Kohlenstoffatomen darstellt, gegebenenfalls durch eine oder mehrere P- und/oder $P_1$-Gruppen substituiert, wobei P und $P_1$ die oben gegebenen Bedeutungen haben,
- die identischen oder unterschiedlichen Bestandteile X und Y jeweils darstellen:
  - wenn $x_1$ und $x_2$ jeweils 1 sind:

* eine

$P_2$
|
-N-Gruppe,

in welcher $P_2$ ein Wasserstoffatom oder die P- oder $P_1$-Gruppe darstellt, wobei P und $P_1$ die oben gegebenen Bedeutungen haben,
* einen aromatischen, stickstoffhaltigen Heteroring mit 4 bis 12 Kohlenstoffatomen, von denen 2 Kohlenstoffatome jeweils in einer Bindung A und B im Falle von X oder mit B und C im Falle von Y gebunden sind, wobei der Heteroring gegebenenfalls durch eine oder mehrere P- und/oder $P_1$-Gruppen substituiert ist wobei P und $P_1$ die oben gegebenen Bedeutungen haben,

- wenn $x_1 = 0$ oder wenn $x_2 = 0$ ist, stellt der jeweilige Endsubstituent Y oder X dar:
  * eine [-COR]-Gruppe, in welcher R die obige Bedeutung hat,
  * einen aromatischen, stickstoffhaltigen Heteroring mit 1 bis 4 Kohlenstoffatomen, gegebenenfalls in Ortho- und/oder Meta- und/oder Parastellung durch eine oder mehrere P- und/oder $P_1$-Gruppen substituiert, wobei P und $P_1$ die oben gegebenen Bedeutungen haben,
  * eine CN-Gruppe,
  * eine

$P_2$
|
$P_3$-N-Gruppe,

in welcher die identischen oder unterschiedlichen $P_2$ und $P_3$ ein Wasserstoffatom oder die P- oder $P_1$-Gruppe darstellen, wobei P und $P_1$ die oben gegebenen Bedeutungen haben,

- die identischen oder unterschiedlichen W und Z jeweils die oben gegebenen Gruppen für X und Y, wenn $x_1$ und $x_2$ jeweils null sind, oder aber einen aromatischen Ring mit 6 bis 14 Kohlenstoffatomen, gegebenenfalls durch eine oder mehrere P- und/oder $P_1$-Gruppen substituiert, darstellen, wobei P und $P_1$ die oben gegebenen Bedeutungen unter Ausschluß der Derivate der Formel

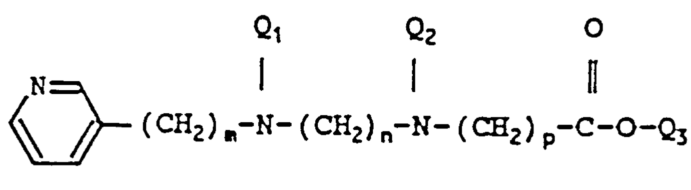

haben, in welcher:
- m und p ganze Zahlen zwischen 1 und 4 sind,
- n eine ganze Zahl zwischen 2 und 4 ist,
- die identischen oder unterschiedlichen $Q_1$ und $Q_2$ ein Wasserstoffatom oder eine Alkylgruppe mit 1 bis 4 Kohlenstoffatomen darstellen,
- $Q_3$ ein Wasserstoffatom, eine Alkylgruppe mit 1 bis 4 Kohlenstoffatomen oder eine Phenylgruppe oder ein Phenyl darstellt, das mit einem, zwei oder drei Substituenten substituiert ist, die unabhängig aus Chlor und einer Alkylgruppe mit 1 bis 3 Kohlenstoffatomen ausgewählt werden;

b) wenn y = 1:
- haben $x_1$ und $x_2$ den Wert 1,

- hat D die gleiche Bedeutung wie die oben festgelegten A, B und C,
- stellen die identischen oder unterschiedlichen W, X, Y und Z jeweils dar:
  - * die identischen obigen Gruppen für X und Y, wenn $x_1$ und $x_2$ jeweils den Wert 1 haben, oder
  - * einen aromatischen Ring mit 6 bis 14 Kohlenstoffatomen, von denen 2 Kohlenstoffatome jeweils in einer Bindung mit A und D im Falle von W oder mit B und A mit Falle von X oder mit C und B im Falle von Y oder mit D und C im Falle von Z gebunden sind, wobei dieser aromatische Ring gegebenenfalls durch eine oder mehrere P- und/oder $P_1$-Gruppen substituiert ist, wobei P und $P_1$ die oben gegebenen Bedeutungen haben, oder
  - * eine Gruppe der Art $-(CH_2)_{x3}-V-(CH_2)_{x4}-$, in welcher $x_3$ und $x_4$ ganze Zahlen zwischen 1 und 3 sind und V einen aromatischen Ring mit 6 bis 14 Kohlesstoffatomen darstellt, gegebenenfalls durch eine oder mehrere P- und/oder $P_1$-Gruppen substituiert, wobei P und $P_1$ die oben gegebenen Bedeutungen haben,
  - * eine

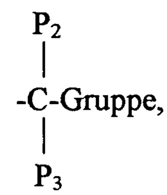

in welcher $P_2$ und $P_3$ die obigen Bedeutungen haben, wobei es sich versteht, daß sowohl für y = 0 oder 1 die entsprechenden Polystickstoffderivate 4 oder 5 koordinierende Funktionen aufweisen, von denen eine durch die [-COR]-Gruppe dargestellt ist, wobei R die oben gegebene Bedeutung hat und die anderen koordinierenden Funktionen stickstoffhaltige Gruppen sind, die man auswählt unter:

- den

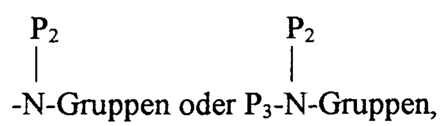

in welchen $P_2$ und $P_3$ die oben gegebenen Bedeutungen haben,
- aromatischen, stickstoffhaltigen Heteroringen mit 4 bis 12 Kohlenstoffatomen, gegebenenfalls durch eine oder mehrere P- und/oder $P_1$-Gruppen substituiert, wie oben definiert,

unter dem Vorbehalt, daß, wenn die Polystickstoffderivate 4 koordinierende Funktionen aufweisen,

* wobei die drei stickstoffhaltigen Gruppen

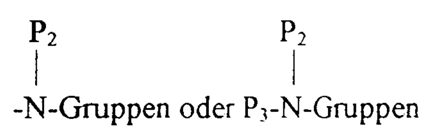

sind, wie oben definiert, und die entsprechenden Polystickstoffderivate einen aromatischen Ring mit 6 bis 14 Kohlenstoffatomen aufweisen, gegebenenfalls durch eine oder mehrere P und/oder $P_1$-Gruppen substituiert, wie oben definiert,

* oder zumindest eine der drei stickstoffhaltigen Gruppen einen aromatischen stickstoffhaltigen Heteroring darstellt, gegebenenfalls durch eine oder mehrere P-und/oder $P_1$-Gruppen substituiert, wie oben definiert,

die Derivate der Formel $\alpha$:

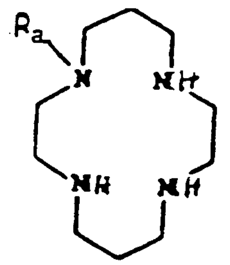

in welcher $R_a$ darstellt:

- eine $[-(CH_2)_n-COOH]$-Gruppe, in welcher n eine ganze Zahl zwischen 1 und 3 ist,
- oder eine Gruppe der Form

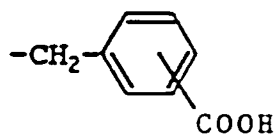

und die Derivate der Fomel $\beta$, wie in Anspruch 3 definiert, ausgeschlossen sind.

**2.** Polystickstoffderivate nach Anspruch 1, dadurch gekennzeichnet, daß sie nur eine [-COR]-Gruppe aufweisen, wobei R die in Anspruch 1 gegebene Bedeutung hat

**3.** Derivate nach einem der Ansprüche 1 und 2 unter Ausschluß von Derivater der Formel $\beta$:

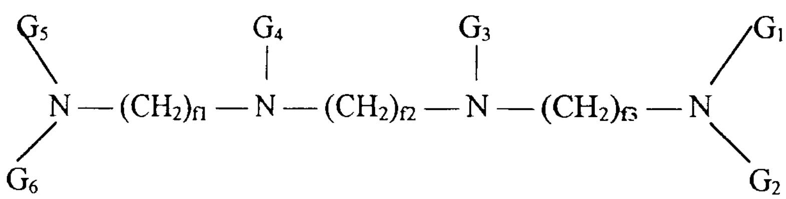

(β)

in welcher:

- die identischen oder unterschiedlichen $G_1$, $G_2$, $G_3$, $G_4$ und $G_5$ für H, $OrthoCH_2C_6H_4OH$, $-CH_2-COOH$ oder

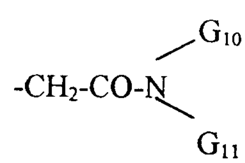

stehen,

- $G_6$ für H oder Ortho-$CH_2C_6H_4OH$ oder einen Rest der Form

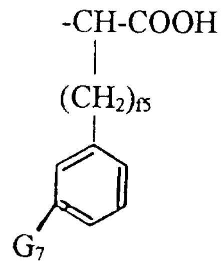

steht,

wobei $G_7$ für $NO_2$,

$NH_2$, 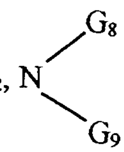

steht,

wobei die identischen oder unterschiedlichen $G_8$ und $G_9$ H oder ein Alkylradikal mit $C_1$-$C_4$, Maleimid oder ein substituiertes Halotriazin sind,

unter dem Vorbehalt, daß, wenn $G_6$ H ist, eines der $G_1$, $G_2$, $G_3$, $G_4$ oder $G_5$ -$CH_2$-COOH,

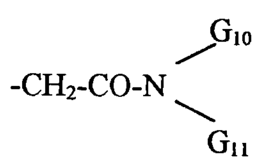

ist,

wobei die identischen oder unterschiedlichen $G_{10}$ und $G_{11}$ für H oder einen Alkylradikal mit $C_1$-$C_4$ stehen, und

- $f_1$, $f_2$ und $f_3$ die Zahlen 2 oder 3 darstellen,
- $f_4$ die Zahlen 0 oder 1 darstellt,
- $f_5$ eine ganze Zahl zwischen 0 und 10 darstellt.

**4.** Polystickstoffderivate nach einem der Ansprüche 1 bis 3 gemäß der folgenden Formel:

$(W\text{-}A)_{x1}\text{-}X\text{-}B\text{-}Y\text{-}(C\text{-}Z)_{x2}$ (Ia),

in welcher W, A, X, B, Y, C, Z, $x_1$ und $x_2$ die in Anspruch 1 gegebenen Bedeutungen haben, wenn y = 0.

**5.** Derivate nach Anspruch 4, dadurch gekennzeichnet, daß $x_1 = x_2 = 1$, was der folgenden Formel entspricht:

W-A-X-B-Y-C-Z (Ib).

**6.** Derivate nach Anspruch 4, dadurch gekennzeichnet, daß $x_1 = 0$, was der folgenden Formel entspricht:

X-B-Y-C-Z (Ic).

**7.** Derivate nach Anspruch 4, dadurch gekennzeichnet, daß $x_2 = 0$, was der folgenden Formel entspricht:

W-A-X-B-Y (Id).

**8.** Derivate nach einem der Ansprüche 4 bis 7, dadurch gekennzeichnet, daß sie 4 koordinierende Funktionen aufweisen.

**9.** Derivate nach Anspruch 8, die der Formel Ib entsprechen, dadurch gekennzeichnet, daß eines der Symbole W oder Z die [-COR]-Gruppe darstellt, wobei R die in Anspruch 1 gegebene Bedeutung hat, während das andere der Symbole W oder Z sowie X und Y jeweils eine stickstoffhaltige Gruppe darstellen,

wobei vorzugsweise:

- eines der Symbole W oder Z steht für:
  - entweder eine Pyridyl-, Pyrimidyl- oder Imidazolylgruppe
  - oder eine Aminobenzylgruppe
- X und Y jeweils eine -NH-Gruppe darstellen,
- A, B und C jeweils für eine $-(CH_2)_x$-Gruppe stehen, wobei x eine ganze Zahl zwischen 1 und 4 ist.

**10.** Derivate nach Anspruch 8, die der Formel Ic entsprechen, dadurch gekennzeichnet, daß:

- eines der Symbole X oder Z für die [-COR]-Gruppe steht, wobei R die in Anspruch 1 gegebene Bedeutung hat, während das andere der Symbole X oder Z sowie Y für eine stickstoffhaltige Gruppe stehen, und
- eines der Symbole B oder C oder Y durch eine durch eine stickstoffhaltige Gruppe substituierte Alkylgruppe mit 1 bis 4 Kohlenstoffatomen substituiert ist, wobei vorzugsweise:
- eines der Symbole X oder Z steht für:
  - entweder eine Pyridyl-, Pyrimidyl- oder Imidazolylgruppe,
  - oder eine Aminobenzylgruppe,
  - oder ein primäres Amin $-NH_2$,
- Y eine

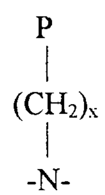

Gruppe darstellt,
in welcher x für eine ganze Zahl zwischen 1 bis 4 steht und P für:
  - entweder eine Pyridyl-, Pyrimidyl- oder Imidazolylgruppe,
  - oder eine Aminobenzylgruppe,
  - oder ein primäres Amin $-NH_2$,
- B und C jeweils für eine $-(CH_2)_x$- Gruppe stehen; wobei x oben definiert ist

**11.** Derivate nach Anspruch 8, die der Formel Id entsprechen, dadurch gekennzeichnet, daß:

- eines der Symbole W oder Y für die oben definierte [-COR]-Gruppe steht, während das andere der Elemente W oder Y sowie X für eine stickstoffhaltige Gruppe stehen, und
- eines der Elemente A oder B oder X durch eine durch eine stickstoffhaltige Gruppe substituierte Alkylgruppe mit 1 bis 4 Kohlenstoffatomen substituiert ist,

  wobei vorzugsweise:
- eines der Symbole W oder Y steht für:
  - entweder eine Pyridyl-, Pyrimidyl- oder eine Imidazolylgruppe,
  - oder eine Aminobenzylgruppe,
  - oder ein primäres Amin $-NH_2$,

- X eine

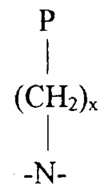

Gruppe darstellt, in welcher x für eine ganze Zahl zwischen 1 und 4 steht und P für:
- entweder eine Pyridyl-, Pyrimidyl- oder Imidazolylgruppe,
- oder eine Aminobenzylgruppe,
- oder ein primäres Amin $-NH_2$,

- A und B jeweils für eine $-(CH_2)_x$-Gruppe stehen, wobei x oben definiert ist.

**12.** Derivate nach einem der Anprüche 4 bis 7, dadurch gekennzeichnet, daß sie 5 koordinierende Funktionen aufweisen.

**13.** Derivate nach Anspruch 12, die der Formel Ib entsprechen, dadurch gekennzeichnet, daß:

a)
- W, X Y und Z für eine stickstoffhaltige Gruppe stehen,
- eines der Symbole W oder Z durch eine Alkylgruppe mit 1 bis 4 Kohlenstoffatomen substituiert ist, die durch eine [-COR]-Gruppe substituiert ist, wobei R die in Anspruch 1 gegebene Bedeutung hat,

  wobei vorzugsweise:
- eines der Symbole W oder Z steht für:
  - entweder eine Pyridyl-, Pyrimidyl- oder Imidazolylgruppe,
  - oder eine Aminobenzylgruppe,
  - oder ein primäres Amin $-NH_2$,
- während das andere der Symbole W oder Z für eine $[-NH-(CH_2)_x-COR]$-Gruppe steht, in welcher R und x die in Anspruch 1 gegebenen Bedeutungen haben,
- X und Y jeweils eine -NH-Gruppe darstellen,
- A B und C jeweils eine $-(CH_2)_x$-Gruppe darstellen, wobei x weiter oben definiert ist,

  oder aber

b)
- eines der Symbole W oder Z für die [-COR]-Gruppe steht, wobei R die in Anspruch 1 gegebene Bedeutung hat,
- während das andere der Symbole W und Z sowie X und Y eine stickstoffhaltige Gruppe darstellen und
- eines der Symbole A, X, B, Y oder C durch eine durch eine stickstoffhaltige Gruppe substituierte Alkylgruppe mit 1 bis 4 Kohlenstoffatomen substituiert ist,

  wobei vorzugsweise:
- eines der Symbole W oder Z steht für:
  - entweder eine Pyridyl-, eine Pyrimidyl- oder Imadazolylgruppe,
  - oder eine Aminobenzylgruppe,
  - oder ein primäres Amin $-NH_2$,
- wobei eines der Symbole X oder Y für -NH- steht, während
- das andere der Symbole X und Y für

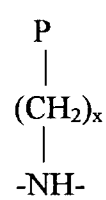

steht, in welchem x für eine ganze Zahl zwischen 1 und 4 steht und P für:
- entweder eine Pyridyl-, eine Pyrimidyl- oder Imadzolylgruppe,
- oder eine Aminobenzyl,
- oder ein primäres Amin $-NH_2$,

- A B und C jeweils für $-(CH_2)_x-$ stehen, wobei x oben definiert ist.

**14.** Derivate nach einem der Ansprüche 1 und 2, die der folgenden allgemeinen Formel entsprechen:

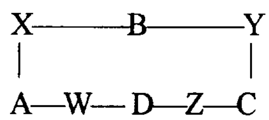

(IIa)

wobei W, A X B, Y, C, Z und D die in Anspruch 1 gegebenen Bedeutungen haben, wenn y = 1.

**15.** Derivate nach Anspruch 14, dadurch gekennzeichnet, daß sie 5 koordinierende Funktionen aufweisen.

**16.** Derivate nach Anspruch 15, dadurch gekennzeichnet, daß:
- W, X, Y und Z jeweils eine stickstoffhaltige Gruppe darstellen,
- eines der Symbole W, X, Y oder Z durch eine Alkylgruppe mit 1 bis 4 Kohlenstoffatomen substituiert ist, welche wiederum durch die [-COR]-Gruppe substituiert ist, wobei R die in Anspruch 1 gegebene Bedeutung hat,

  wobei vorzugsweise:
- W, X, Y und Z jeweils stehen für:
  * entweder eine Pyridyl-, Pyrimidyl- oder Imidazolylgruppe, gegebenenfalls durch eine Alkylgruppe mit 1 bis 4 Kohlenstoffatomen substituiert, die ihrerseits durch die [-COR]-Gruppe substituiert ist, wobei R die in Anspruch 1 gegebenen Bedeutung hat,
  * oder eine -NH-Gruppe oder eine

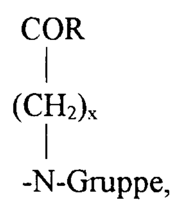

wobei x und R die in Anspruch 1 gegebenen Bedeutungen haben.

**17.** Verfahren zur Darstellung der Derivate nach einem der Ansprüche 1 bis 16, dadurch gekennzeichnet, daß es den Einsatz mindestens eines der folgenden Kondensationsschritte aufweist, nämlich:
- die Reaktion eines Halogenids vom Typ $R_1$-Z, wobei Z für ein Halogen, insbesondere Chlor oder Brom, steht und $R_1$ einem Teil des zu synthetisierenden Derivats entspricht und aus den Gruppen ausgewählt wird, die den Einheiten W-A-, $(W-A)_{x1}$-X-B-, $(W-A)_{x1}$-X-B-Y-C-, oder -A-X-B-Y-C-Z, -B-Y-$(C-Z)_{x2}$ oder -C-Z entspricht, und zwar mit einem primären Amin (2) $H_2N-R_2$ oder einem sekundären Amin (3)

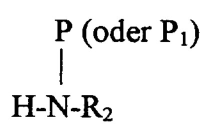

gemäß dem Schema:

$$R_1\text{-}Z + H_2NR_2 \text{ oder } H\text{-}\overset{\overset{\text{P (oder } P_1)}{|}}{N}\text{-}R_2 \text{ -----> } R_1\text{-}NH\text{-}R_2 \text{ oder } R_1\text{-}\overset{\overset{\text{P (oder } P_1)}{|}}{N}\text{-}R_2$$

wobei die oben erwähnten Aminderivate (2 und 3) dann jeweils unter den Gruppen ausgewählt werden, in welchen $-NH-R_2$ oder

$$\text{-}\overset{\overset{\text{P (oder } P_1)}{|}}{N}\text{-}R_2$$

den Einheiten $-X-B-Y-(C-Z)_{x2}$, $-Y-(C-Z)_{x2}$, -Z oder W-, $(WA)_{x1}-X-$, und $(W-A)_{x1}-X-B-Y-$ entsprechen, in welchen W, A, Y, B, X, C, Z, P, $P_1$, $x_1$ und $x_2$ die in Anspruch 1 gegebenen Bedeutungen haben, wenn y = 0,

- die Reaktion eines Aldehyds (4) der Formel $R_1$-CHO, in welcher $R_1$ die oben gegebene Bedeutung hat und zwar mit einen primären Amin (2), wie oben angegeben, gefolgt von einem Reduktionsschritt gemäß

$R_1$-CHO + $H_2N$-$R_2$ -----> $R_1$-CH = N-$R_2$ -----> $R_1$-$CH_2$NH-$R_2$,

- die Reaktion von Acrylnitril mit dem Aminderivat (2 oder 3), was zur Bildung einer durch eine Nitrilgruppe abgeschlossenen Kette führt, gefolgt von einem Hydrolyseschritt zur Umwandlung der CN-Gruppe in eine [-COOH]-Gruppe, woraufhin gegebenenfalls ein Funktionalisierungsschritt zur Erzeugung einer [-COR]-Gruppe folgt,
- die Reaktion eines Halogenids der oben erwähnten Formel $R_1$-Z, in welchem $R_1$ für W-A-, W-A-X-B, X-B-, -B-Y, -B-Y-C-Z steht, und zwar mit einem Piperazinon, woraufhin eine Hydrolysereaktion der Amidfunktion nach folgendem Schema folgt:

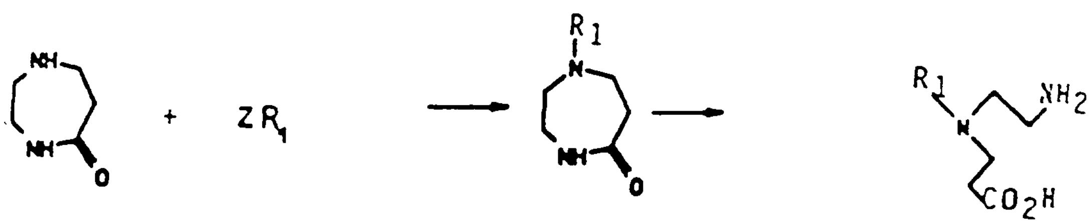

im Falle einer Monokondensation,
oder gemäß:

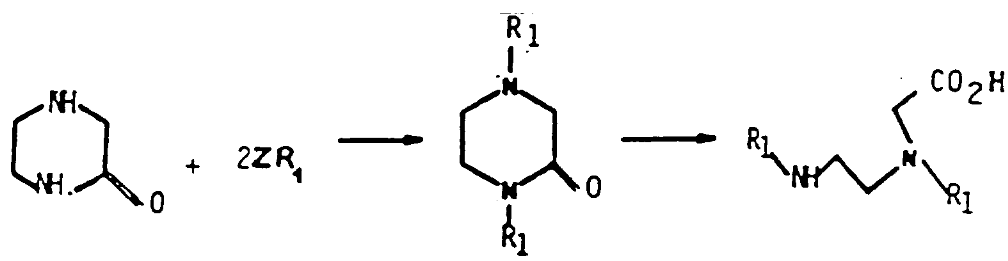

im Falle einer Dikondensation,

- die Kondensation eines Derivats (5), das zwei primäre Aminfunktionen trägt, wobei dieses Diamin (5) der Formel $H_2N\text{-}T_1\text{-}R_9\text{-}T_2\text{-}NH_2$ entspricht, und zwar mit einem Dialdehyd (6), das der Formel $OHC\text{-}T_1\text{-}R_{10}\text{-}T_2\text{-}CHO$ entspricht, wobei die identischen oder unterschiedlichen $T_1$ und $T_2$ eine Alkylenkette $-(CH_2)_x-$ darstellen, in welcher x für eine ganze Zahl von 1 bis 3 steht, gegebenenfalls substituiert durch eine oder mehrere P-Gruppen oder durch eine oder mehrere $P_1$-Gruppen, wobei P und $P_1$ wie oben definiert sind, und die identischen oder unterschiedlichen $R_9$ und $R_{10}$ die in Anspruch 1 gegebenen Bedeutungen für X oder Y haben, gefolgt von einem Reduktionsschritt des sich aus der vorhergehenden Kondensation ergebenden zyklisierten Produktes.

**18.** Metallkomplexe, dadurch gekennzeichnet, daß sie aus einem Metallatom, insbesondere aus Co, Ni, Fe, Mn, Cu, und einem Derivat nach einem der Ansprüche 1 bis 16 einschließlich der Derivate der Formel $\alpha$ bestehen.

**19.** Verfahren zur Herstellung von Komplexen nach Anspruch 18, dadurch gekennzeichnet, daß es die Reaktion zwischen dem Metall in Form eines Salzes, insbesondere in Form eines Acetats, eines Nitrats oder eines Halogenids mit einem Derivat nach einem der Ansprüche 1 bis 16 aufweist.

**20.** Verfahren zur Abtrennung oder Herstellung von Sauerstoff aus einem ihn enthaltenden Gasgemisch, dadurch gekennzeichnet, daß das Verfahren aufweist:

- das Zusammenbringen des Gasgemisches mit einem Metallkomplex nach Anspruch 18 unter Bedingungen, welche die Absorption des Sauerstoffs des Gasgemisches ermöglichen, was zur Bildung eines Dimers führt, das in seiner neutralen Form der Formel $XO_2X$ entspricht, in welcher X ein Metallkomplex-Molekül darstellt,
- die Desorption des in dem Dimer fixierten Sauerstoffs, und
- die Rückgewinnung des desorbierten Sauerstoffs,

wobei der Metallkomplex vorzugsweise in wäßriger oder teilweise wäßriger Lösung mit einer Konzentration von 0,1M bis 1M bei einem pH-Wert von 6 bis 8 vorliegt, oder aber stattdessen in einer Lösung vorliegt, die aufeiner für Sauerstoff durchlässigen Membran festgelegt ist, wobei diese Membran einen Träger aufweist, der aus einem für Flüssigkeiten nicht-durchdringbaren Polymerfilm gebildet ist, dessen eine Fläche mit dem den Sauerstoff enthaltenden gasförmigen Gemisch in Berührung ist, während die Desorption des mittels des Metallkomplexes fixierten Sauerstoffs durch die andere Fläche bewirkt wird,

wobei die Desorption vorzugsweise mittels elektrochemischer Desorption oder mittels eines Vakuums durchgeführt wird, insbesondere durch Senkung des Sauerstoff-Partialdruckes in der die Lösung umgebenden Atmosphäre, wenn der Metallkomplex in wäßriger oder teilweise wäßriger Lösung hergestellt wird, und/oder indem man eine Temperaturdifferenz auf beiden Seiten einer Membran anlegt, wenn der Metallkomplex in einer auf einer Membran festgelegten Lösung gelöst ist.

**21.** Dimer der Formel $XO_2X$, in welchem X für einen Metallkomplex nach Anspruch 18 steht.

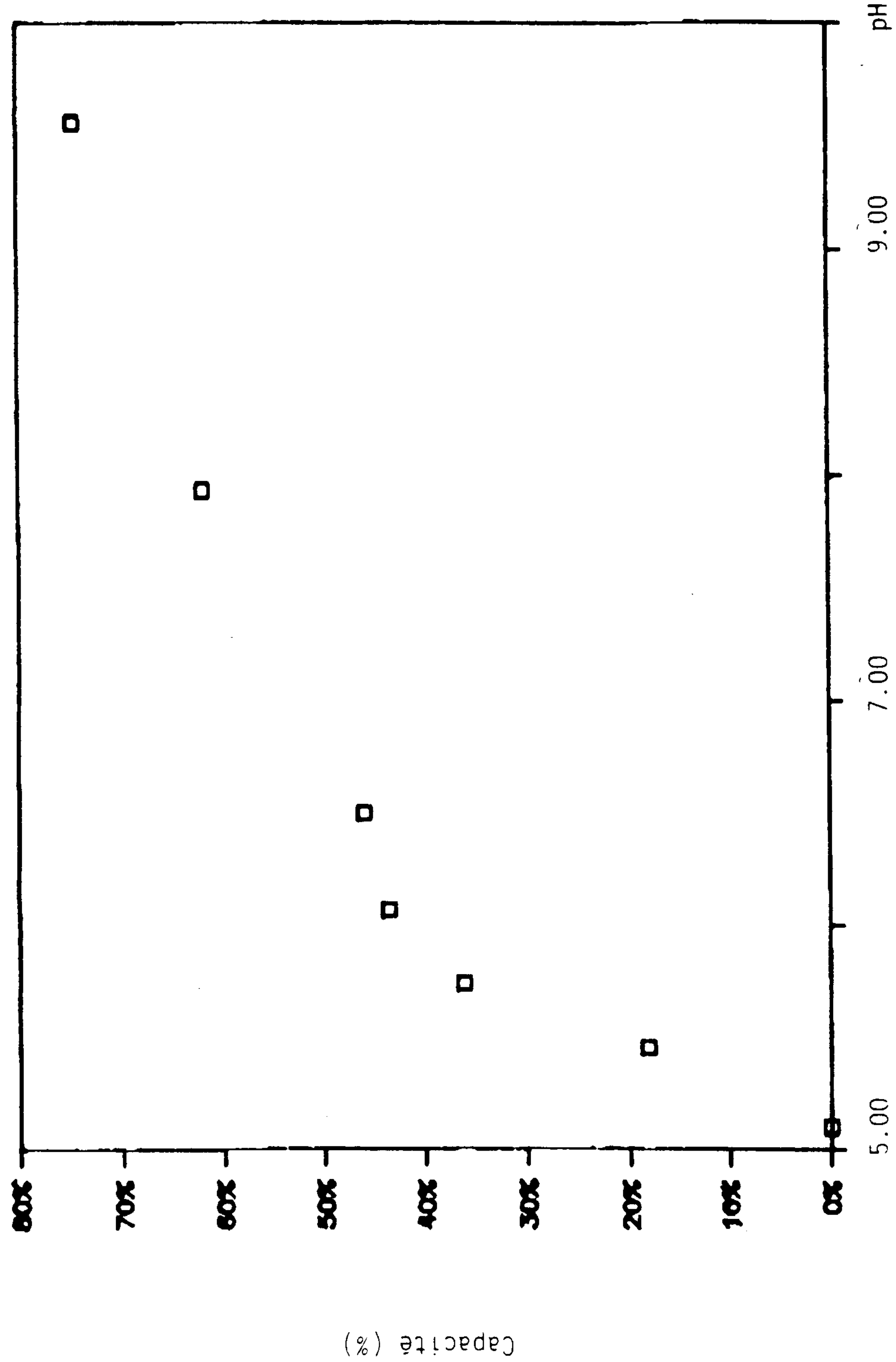
pH
9.00
7.00
5.00
80%
70%
60%
50%
40%
30%
20%
10%
0%
Capacité (%)

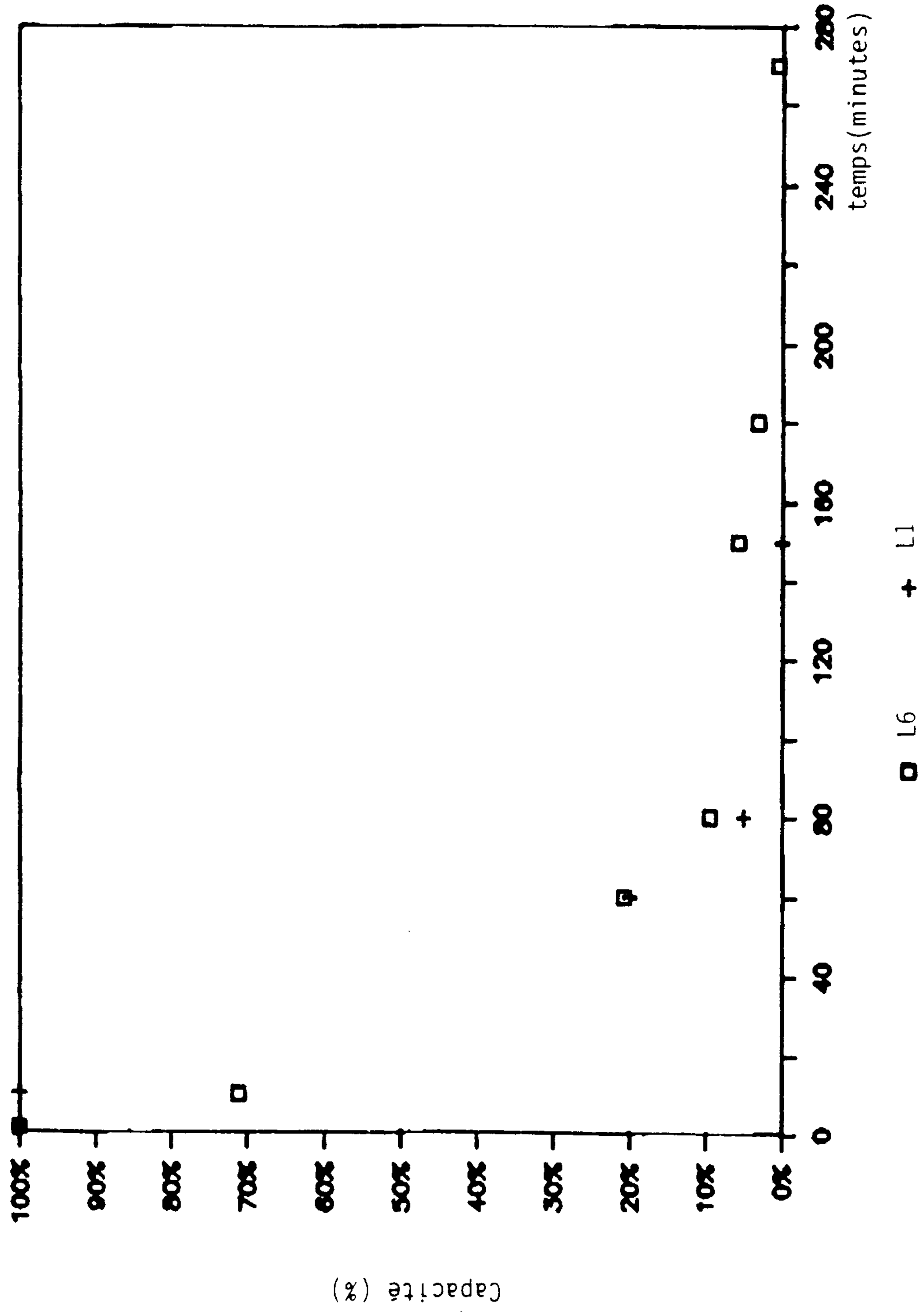

Capacité (%)
100%
90%
80%
70%
60%
50%
40%
30%
20%
10%
0%
0
40
80
120
160
200
240
280
temps(minutes)
L6
L1

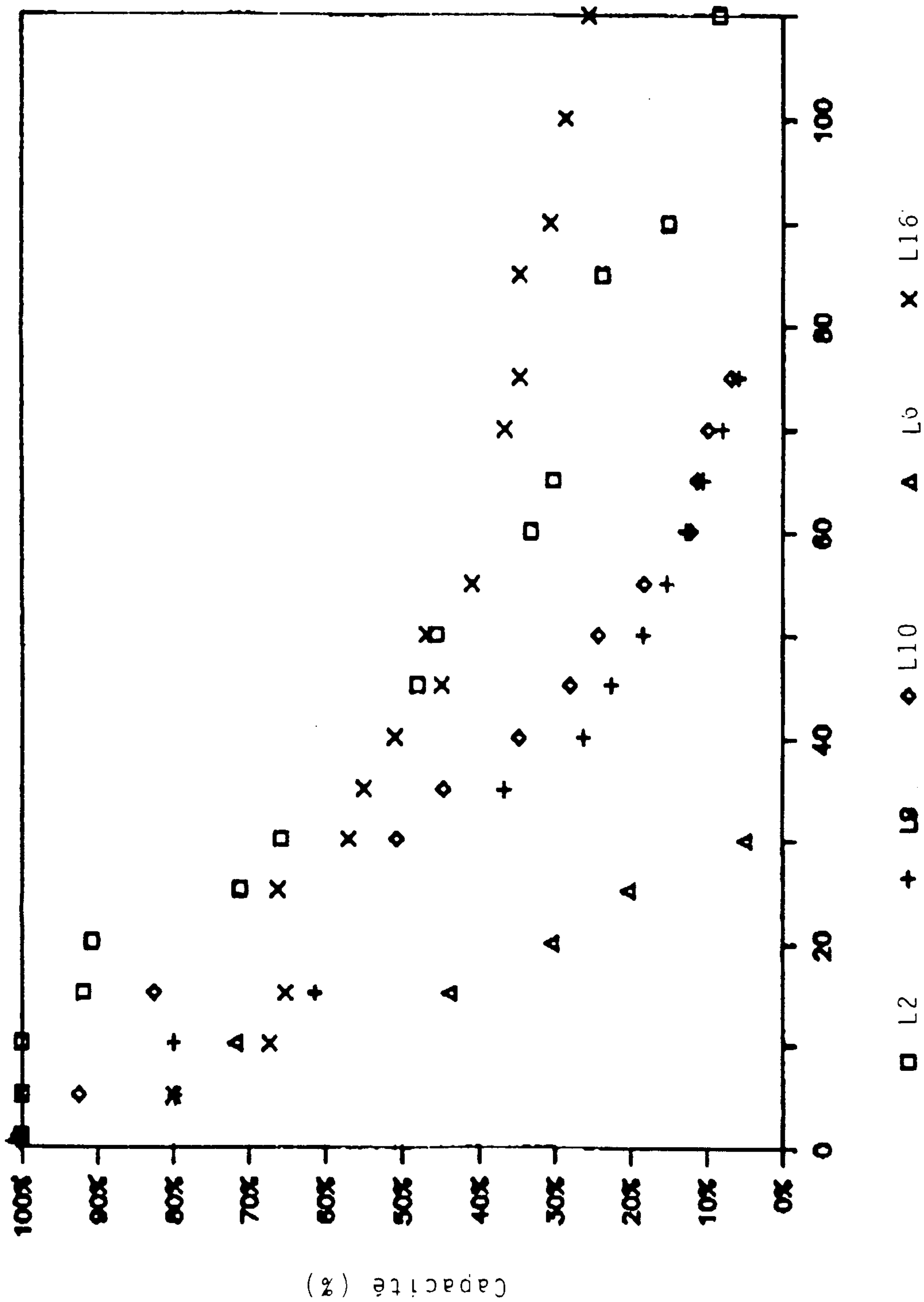
Capacité (%)
100%
90%
80%
70%
60%
50%
40%
30%
20%
10%
0%
0
20
40
60
80
100
L2
L9
L10
L5
L16

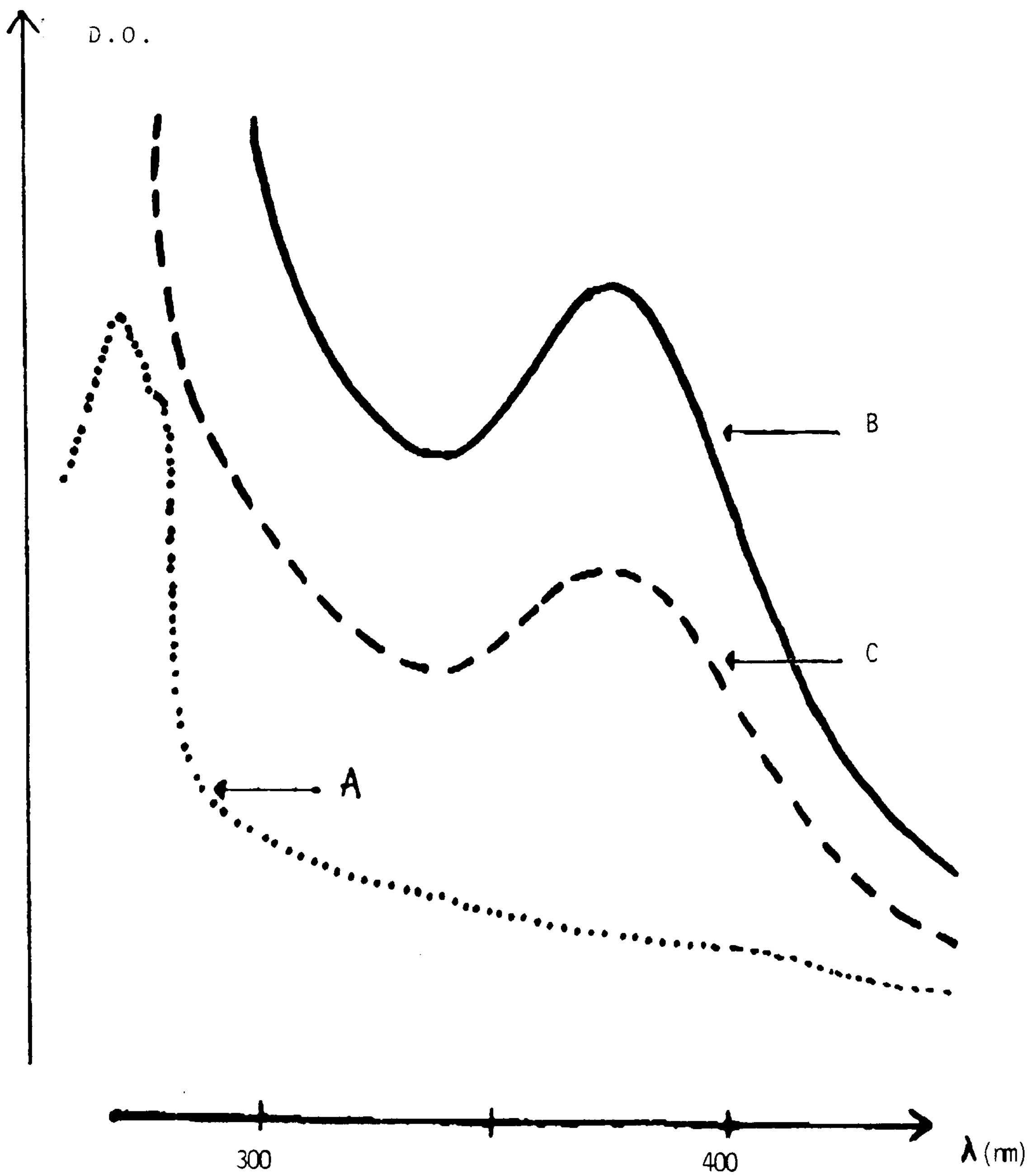
D.O.
B
C
A
300
400
λ (nm)